(19) Europäisches Patentamt
European Patent Office
Office européen des brevets

(11) EP 1 891 999 B1

(12) **EUROPEAN PATENT SPECIFICATION**

(45) Date of publication and mention of the grant of the patent:
**07.12.2016 Bulletin 2016/49**

(21) Application number: **06730981.5**

(22) Date of filing: **03.04.2006**

(51) Int Cl.:
***A61M 1/36*** *(2006.01)* ***B01D 67/00*** *(2006.01)*
***B01D 71/68*** *(2006.01)*

(86) International application number:
**PCT/JP2006/307033**

(87) International publication number:
**WO 2006/107014 (12.10.2006 Gazette 2006/41)**

# (54) METHOD FOR PROVIDING A STERILIZED BLOOD PURIFIER DEVICE

METHODE ZUR BEREITSTELLUNG EINES STERILISIERTEN BLUTREINIGUNGSGERÄTES

METHODE DE PREPARER UN APPAREIL À PURIFICATION DE SANG STÉRILISÉ

(84) Designated Contracting States:
**DE FR GB**

(30) Priority: **04.04.2005 JP 2005107620**
**29.07.2005 JP 2005222247**

(43) Date of publication of application:
**27.02.2008 Bulletin 2008/09**

(73) Proprietor: **TOYOBO CO., LTD.**
**Osaka-shi**
**Osaka 530-8230 (JP)**

(72) Inventors:
- **MABUCHI, Kimihiro,**
**c/o TOYO BOSEKI KABUSHIKI KAISHA**
**Ohtsu-shi Shiga 5200292 (JP)**
- **YOKOTA, Hideyuki,**
**c/o TOYO BOSEKI KABUSHIKI KAISHA**
**Ohtsu-shi Shiga 5200292 (JP)**
- **KUZE, Katsuaki,**
**c/o TOYO BOSEKI KABUSHIKI KAISHA**
**Ohtsu-shi Shiga 5200292 (JP)**
- **MONDEN, Noriko,**
**c/o TOYO BOSEKI KABUSHIKI KAISHA**
**Ohtsu-shi Shiga 5200292 (JP)**
- **KATO, Noriaki,**
**c/o TOYO BOSEKI KABUSHIKI KAISHA,**
**Ohtsu-shi Shiga 5200292 (JP)**
- **OHNO, Makoto,**
**c/o TOYO BOSEKI KABUSHIKI KAISHA,**
**Ohtsu-shi Shiga 5200292 (JP)**
- **SUZUKI, Mitsuru,**
**c/o TOYO BOSEKI KABUSHIKI KAISHA,**
**Osaka-shi Osaka 5308230 (JP)**

(74) Representative: **Vossius & Partner**
**Patentanwälte Rechtsanwälte mbB**
**Siebertstrasse 3**
**81675 München (DE)**

(56) References cited:
**EP-A1- 0 997 182**
**EP-A2- 1 110 563**
**EP-A2- 1 518 564**
**WO-A1-2005/089918**
**JP-A- 2001 170 171**
**JP-A- 2004 305 840**
**JP-B1- 3 594 032**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 891 999 B1

## Description

### TECHNICAL FIELD

The present invention relates to a method of providing a sterilized blood purifier which has excellent compatibility with blood, safety and reliability of performance.

### BACKGROUND ART

In the hemocathartic therapies for renal failures, etc., blood purifiers such as hemodialyzers, blood filters, hemodialytic filters, etc. are widely used to remove urine toxic substances and waste products from blood. Blood purifiers such as hemodialyzers, blood filters, hemodialytic filters, etc. are fabricated using, as separators, dialytic membranes or ultrafiltration membranes which are manufactured using natural materials such as cellulose or derivatives thereof (e.g., cellulose diacetate, cellulose triacetate, etc.) and synthesized polymers such as polysulfone, polymethyl methacrylate, polyacrylonitrile, etc. Particularly, blood purifiers using hollow fiber mebranes as separators are highly important in the field of blood purification because of their advantages such as the reduction of in vitro circulation blood amounts, high efficiency of removing toxic and waste substances from blood, high blood purifier-fabricating productivity, etc.

Among the above membrane materials, polysulfone-based resins having high water permeability have attracted keen interests as the most suitable materials for the advance of dialytic technologies. However, semipermeable membranes formed of polysulfone-based resins alone are poor in affinity with blood and tend to cause air lock phenomena, since the polysulfone-based resins are hydrophobic. Therefore, such semipermeable membranes as they are can not be used to treat blood.

To solve the problem, there are proposed methods of imparting hydrophilicity to such membranes, by adding hydrophilic polymers to the polysulfone-based resins: for example, there are disclosed methods of blending polyhydric alcohols such as polyethylene glycol, etc. to the polysulfone-based resins (cf. Patent Literatures 1 and 2).

Patent Literature 1: JP-A-61-232860 (1986)
Patent Literature 2: JP-A-58-114702 (1983)

Other methods are disclosed in which polyvinyl pyrrolidone is added to the polysulfone-based resin (cf. Patent Literatures 3 and 4).

Patent Literature 3: JP-B-5-54373 (1993)
Patent Literature 4: JP-B-6-75667 (1994)

As a method to solve the above problem, the method using polyvinyl pyrrolidone has attracted keen interests in view of safety and cost. However, the hydrophilicity-imparting technique by adding polyvinyl pyrrolidone has a problem in that polyvinyl pyrrolidone elutes from membranes and contaminates the purified blood during a hemodialysis. When the amount of eluting polyvinyl pyrrolidone becomes larger, the amount of polyvinyl pyrrolidone, as foreign materials to the organisms, accumulated in vivo becomes larger over a long period of hemodialysis, which is likely to induce side effects or complications. To solve such disadvantages, the amount of eluting polyvinyl pyrrolidone is regulated in the Approval Standard for Dialysis-type Artificial Kidney Apparatus, and is determined by UV absorbance according to this standard. In the meantime, a technique for evaluating the eluation amount-controlling effect based on this standard is disclosed (cf. Patent Literatures 5 to 7). Further, Patent Literature 8 discloses a semipermeable membrane for treating blood, wherein the amount of a hydrophilic polymer eluting from such a semipermeable membrane is 10 ppm or less. This literature discloses a method of inhibiting a hydrophilic polymer from eluting from the semipermeable membrane for treating blood, but does not refer to the influence of hydrogen peroxide on the deterioration and decomposition of a hydrophilic polymer with time, and further on the storage of hollow fiber membranes.

Patent Literature 5: Japanese Patent No. 3314861
Patent Literature 6: JP-A-6-165926 (1994)
Patent Literature 7: JP-A-2000-350926 (2000)
Patent Literature 8: JP-A-2001-170171 (2001)

However, since these materials are synthesized materials, they are recognized as foreign matters to human bodies and induces various vital reactions. For example, when such a material is brought into contact with blood, blood platelet adheres to the material, or white blood cells are activated. Thus, such a material sometimes shows poor compatibility with blood.

Techniques for improving the blood compatibility of membranes by controlling the unevenness of the blood-contacting surfaces of the membranes are disclosed (cf. Patent Literatures 9 and 10). In these techniques, the unevenness of the surface of the membrane is specified based on a value measured with a white interference contrast microscope. According to Patent Literature 1, the number of blood platelets adhered to the membrane is preferably $10^{-6}/cm^2$ membrane area or less. A membrane satisfying this feature has a blood platelet-retaining rate of approximately 100% as a result of rough calculation. This blood platelet-retaining rate will be described in detail later. However, a membrane having an extremely high blood platelet-retaining rate is likely to release the blood platelet activated by the contact with the membrane, and this release is considered to induce the activation of a whole of the blood circulated in a human body, which consequently degrades the biocompatibility of the membrane.

Commonly recognized in the above Patent Literatures is that the smooth blood-contacting surfaces of the membranes are considered to have larger blood cell-contacting areas, which is likely to induce the activation of the blood cells. It is considered that the control of the physical properties of the surface of the membrane is one of the effective methods for improving the blood compatibility of the membrane. However, this approach alone has a limit because of the use of the material which is essentially a foreign matter to the human body.

Patent Literature 9: JP-A-2000-126286 (2000)
Patent Literature 10: JP-A-11-309353 (1999)

The present inventors have carefully researched the eluting behaviors of polyvinyl pyrrolidone, and have discovered that hydrogen peroxide impossible to measure by a known UV absorbance method is contained in an extract obtained by a testing method regulated in the Approval Standard for Dialysis-type Artificial Kidney Apparatus. When hydrogen peroxide is present in a blood purifier or permselective separation membrane, the deterioration of polyvinyl pyrrolidone due to the oxidation thereof is accelerated, and the storage stability of hollow fiber membranes becomes poor since the amount of eluting polyvinyl pyrrolidone tends to increase while the hollow fiber membranes are being stored. However, the above Patent Literatures disclose the techniques for suppressing the elution of the hydrophilic polymers from the semi-permeable membranes for treating blood but do not refer to the influences of hydrogen peroxide on the aging deterioration and decomposition of the hydrophilic polymers in the hollow fiber membranes, and further on the storage of the membranes.

In any of the conventional techniques disclosed in Patent Literatures 5 to 8, the evaluation is made on specified sites of the hollow fiber membranes. However, it is found that the evaluation of the membranes at such specified sites alone can hot meet a demand for high safety of hollow fiber membranes, because the amount of elution within the hollow fiber membrane bundle largely changes because of the influence of variation in drying conditions, while the hollow fiber membranes are being dried in the course of the fabrication of a blood purifier using the same. If hydrogen peroxide, elucidated by the present inventors, is present at specified sites of a hollow fiber membrane bundle, the deterioration reaction of the materials of the hollow fiber membrane bundle starts from such sites, and this deterioration reaction transmits over a whole of the hollow fiber membrane bundle. Therefore, it is needed to make it sure to keep, to a predetermined value or less, the amount of hydrogen peroxide in a whole of the hollow fiber membrane bundle for use as a blood purifier in its lengthwise direction.

In the meantime, a blood purifier is subjected to a radioactive ray exposure treatment in order to crosslink polyvinyl pyrrolidone in a permselective hollow fiber membrane packed in the blood purifier or to sterilize the blood purifier. However, the radioactive ray exposure induces not only the crosslinking reaction and the sterilizing action but also the denature of a part of the hydrophilic polymer. In other words, the hydrophilic polymer reacts with water and oxygen in the treating atmosphere to have an instable functional group and partial structure which are being oxidized, or a new functional group which is formed by hydrolysis. Even if the content of the hydrophilic polymer in a whole of the membrane is small, most of the hydrophilic polymer is present in the form of a concentrate on the surfaces of the agglomerated polysulfone particles by the phase separation. Therefore, the influence of the hydrophilic polymer on the blood can not be ignored. As a result, the physiochemical change of the denatured portion of the hydrophilic polymer is likely to lower the anti-thrombogenic property of the membrane. The denature of the hydrophilic polymer further continues during the long-term storage of the membranes after the radiation exposure, and thus, the anti-thrombogenic property of the membrane degrades before the practical use of the membrane.

For example, a technique to solve this problem is disclosed: that is, the carboxyl group content and the peroxide content in a membrane exposed to a radioactive ray are controlled within predetermined ranges. The resultant membrane is excellent in the anti-thrombogenic property and is able to maintain the anti-thrombogenic state over a long period of storage (cf. Patent Literature 11).

Patent Literature 11: JP-A-2000-135421 (2000)

However, the technique disclosed in this Patent Literature is to be applied to a so-called wet type blood purifier

which is filled with water and is then exposed to a radioactive ray. Naturally, this wet type blood purifier is heavy in weight because of the water filling the blood purifier, which leads to various problems: that is, the transport and handling of such a purifier is hard; and the water filling the blood purifier is frozen in a cold region or in a severely cold season to burst or damage the hollow fiber membranes. Further, the preparation of a lot of sterilized water leads to a higher cost. Above all, the hollow fiber membranes in a wet state which facilitates the breeding of bacteria is supposed to permit the breeding of bacteria in a very short time from the packaging of the blood purifier until the sterilization thereof. Consequently, a long time is required to completely sterilize the blood purifier manufactured in this way, and such a blood purifier costs higher and, undesirably, has a problem in its safety. This technique has problems in that the blood purifier is exposed to a radioactive ray in the presence of a radical-trapping agent which is needed to be washed and removed before the use of the blood purifier. Under such a situation, there is an increasing demand for a method for avoiding the above problems by subjecting, to radiation exposure in the absence of a radical-trapping agent, a so-called dry type blood purifier packed with dry permselective hollow fiber membranes.

When a blood purifier is used as a dialyzer for artificial kidney, it is needed to completely sterilize the blood purifier. In this sterilization treatment, sterilization methods using formalin, an ethylene oxide gas, high-pressure steam and a radioactive ray such as γ-ray or an electron beam are employed, and these sterilization methods exhibit peculiar effects, respectively. Among those, the sterilization methods by way of exposure to radioactive rays or electron beams are preferably employed, because subjects in packages can be treated as they are, and because the sterilization effects thereof are excellent.

However, it is known that hollow fiber membranes for use in blood purifiers, adhesives for use in fixing the hollow fiber membranes, etc. tend to deteriorate under the radiation exposure. Therefore, there is proposed a method for sterilization while preventing such deterioration. For example, there is disclosed a method for preventing the deterioration of hollow fiber membranes by way of γ-ray exposure after wetting the hollow fiber membranes above their saturation water contents (cf. Patent Literature 12). However, this method suffers from the same problems as in the above Patent Literature 11.

Patent Literature 12: JP-B-55-23620 (1980)

There is disclosed a method for avoiding the wet state of hollow fiber membranes and inhibiting the deterioration of the hollow fiber membranes due to radiation exposure, wherein the hollow fiber membranes containing a sterilization protective agent such as glyceline, polyethylene glycol or the like, in a dried state, are exposed to γ-ray (cf. Patent Literature 13). However, this method is hard to keep lower the water content of the hollow fiber membranes because of the protective agent contained in the hollow fiber membranes. In addition, this method suffers from problems of the deterioration of the protective agent due to the γ-ray exposure and of labors for washing off the protective agent just before the use of the membranes.

Patent Literature 13: JP-A-8-168524 (1996)

To solve this problem, there is disclosed a process for manufacturing a dialyzer (cf. Patent Literature 14). This process include the steps of packing semi-permeable membranes in a dialyzer, saturating the dialyzer with water in an amount of 100% or more based on the weight of the semi-permeable membranes, displacing the inner atmosphere of the dialyzer with an inert gas, and exposing the dialyzer to γ-ray. However, this Patent Literature does not refer to the required properties of the hollow fiber membranes before the radioactive ray exposure or the influence of such exposure on the priming of the hollow fiber membranes.

Patent Literature 14: JP-A-2001-170167 (2001)

To solve the above problems, there is disclosed a method for sterilizing hollow fiber membranes by way of exposure to a radioactive ray, while the water content of the hollow fiber membranes is being controlled to 5% or lower, and while the relative humidity of an ambient atmosphere around the hollow fiber membranes is being controlled to 40% or lower (cf. Patent Literature 15). By this method, the above problem is solved, and the UV absorbance of an extract from the membranes at a wavelength of 220 to 350 nm, measured according to the elution test of dialysis membranes regulated in the Approval Standard for Dialysis-Type Artificial Kidney Apparatus, satisfies a reference value of 0.1 or less. However, Patent Literature 15 does not refer to any of the influence of the oxygen concentration in the ambient atmosphere around the hollow fiber membranes during the sterilization treatment and the aging change in the amount of an eluted substance after the sterilization treatment.

Patent Literature 15: JP-A-2000-288085 (2000)

Further, there is disclosed a method for decreasing the insolubilized component of a membrane material to 10 wt.% or less by exposing hollow fiber membranes to γ-ray, with the water content of the hollow fiber membranes kept at 10 wt.% or less, in the sterilization by way of exposure to γ-ray (cf. Patent Literature 16). This Patent Literature discloses that the amount of a hydrophilic polymer, extracted with a 40% aqueous ethanol solution, per 1 $m^2$ of the subject liquid-contacting area of the membrane can be decreased to 2.0 $mg/m^2$ or less. However, this Patent Literature also does not refer to any of the influence of the oxygen concentration in the ambient atmosphere around the hollow fiber membranes during the γ-ray exposure, the aging change in the amount of an eluted substance after the sterilization treatment, and the influence of the sterilization on the priming of the membranes.

Patent Literature 16: JP-A-2001-205057 (2001)

In the meantime, there is known a method for preventing the base material of a medical device from deteriorating due to oxygen, wherein the medical device is sealed together with an oxygen scavenger in an oxygen-impermeable packaging material and is then exposed to a radioactive ray. A blood purifier is also disclosed therein (cf. Patent Literatures 17 to 19).

Patent Literature 17: JP-A-62-74364 (1987)
Patent Literature 18: JP-A-62-204754 (1987)
Patent Literature 19: WO98/58842

The deterioration of the base material due to the radiation exposure in the presence of the oxygen scavenger is exemplified as the emission of odors in Patent Literature 15, as decreases in the strength of the base material and the performance for dialysis in Patent Literature 16, and as a decrease in the strength of the base material and occurrence of aldehydes in Patent Literature 17. However, any of the Patent Literatures does not refer to an increase in the amount of the above-mentioned extract, or to the importance of the water content in the hollow fiber membranes, while the oxygen concentration in the packaging bag during the radiation exposure is described.

Further, any of the Patent Literatures refers to the importance of the gas-, particularly, oxygen-impermeability of the material of the packaging bag for use in the sterilization by radioactive ray exposure in the system using the above oxygen scavenger, but not to the humidity permeability thereof.

There is further disclosed a method for sterilizing a liquid-treating device filled with a wet or semi-wet membrane-protecting agent by way of radiation exposure under an inert gas atmosphere (cf. Patent Literature 20). This Patent Literature describes the use of an oxygen scavenger as a means for achieving an inert gas atmosphere, and also describes the use of water as the membrane-protecting agent. On the other hand, while not referring to the lower limit in the water content of the semi-wet membrane-protecting agent, this Patent Literature describes the following problem in the part of Problems to be Solved by the Invention: "glyceline, physiologic saline or water oozes and adheres to the outer wall of the liquid-treating device and the interior of the packaging bag, and also adheres to an operator's hand while the liquid-treating device is being operated." This problem suggests that the membrane-protecting agent has a saturation water content or more. Therefore, it can be recognized that this method suffers from the same problem as in the method disclosed in Patent Literature 12.

Patent Literature 20: JP-A-8-280795 (1996)

There is disclosed a method for sterilizing a vacuum-packed dry type hollow fiber membrane type blood purifier by way of exposure to γ-ray, in order to sustain the sterilization effect over a long period of time (cf. Patent Literature 21). However, this Patent Literature does not refer to the deterioration of the hollow fiber membranes during the γ-ray exposure or the storage thereof, or to the water content of the hollow fiber membranes.

Patent Literature 21: JP-A-2001-149471 (2001)

It is disclosed that the γ-ray exposure of the dried hollow fiber membranes increases the amount of a peroxide in the hollow fiber membranes, in comparison with the γ-ray exposure of hollow fiber membranes in a wet state. However, this Patent literature does not refer to a method for suppressing the formation of the peroxide due to the γ-ray exposure of the dried hollow fiber membranes (cf. Patent Literature 22).

Patent Literature 22: JP-A-2000-135421 (2000)

Further Patent Literatures disclose methods for preventing the elution of polyvinyl pyrrolidone and methods for sterilizing hollow fiber membranes by way of exposure to γ-ray, etc. in the manufacturing of permselective hollow fiber

membranes for use in hemocathartic treatment, as described above. Some of them disclose the water content of the hollow fiber membranes during the exposure or the conditions for the exposure atmospheres, but not the required properties of the hollow fiber membranes before the radiation exposure or the influence of the radiation exposure on the priming of the hollow fiber membranes.

There is further disclosed a package of liquid separation membranes for use in an industrial scale water treatment or the like: the separation membranes are packed in a film of a specific composition which inhibits the air permeability of the film. There is also disclosed a method for storing the same (cf. Patent Literature 23). This Patent Literature relates to the package of wet liquid separation membranes filled with deoxygenerated water having a specific concentration of dissolved oxygen and to the method for storing the same.

Patent Literature 23: JP-A-2004-195380 (2004)

Further Patent Literatures disclose methods for drying hollow fiber membrane bundles by way of exposure to microwaves, but do not refer to the generation of hydrogen peroxide during the drying or the storage stability of the dried hollow fiber membrane bundles (cf. Patent Literatures 24 to 27).

Patent Literature 24: JP-A-2003-175320 (2003)
Patent Literature 25: JP-A-2003-175321 (2003)
Patent Literature 26: JP-A-2003-175322 (2003)
Patent Literature 27: JP-A-2004-305997 (2004)

There are proposed various trials to improve the wetability of separation membranes when a dry type blood purifier is being subjected to a priming treatment. Patent Literature 28 discloses a technique for controlling the ratio of the albumin-screening coefficients of a hemodialysis membrane comprising a cellulosic polymer, to "SCalb (24 hr.)/SCalb (0 hr.) $\geq$ 1.2", wherein SCalb (0 hr.) represents the albumin-screening coefficient found immediately after the priming of the membranes with physiologic saline or a dialysate, and SCalb (24 hr.) represents the albumin-screening coefficient found after the membranes have been left to stand for 24 hours since the priming of the membranes. However, the technique disclosed in this Patent Literature may not be able to provide a blood purifier stable in performance and quality, because of too large difference in performance between the membranes just after the priming and the same membranes left to stand for 24 hours after the priming.

Patent Literature 28: JP-A-2004-313359 (2004)

Further, there is disclosed a porous polymer membrane which comprises a hydrophobic polymer and a hydrophilic polymer and which is excellent in water wetability as a whole, while possessing excellent mechanical strength derived from the hydrophobic polymer (cf. Patent Literature 29). The membrane of this Patent Literature may be good in water wetability, since the skeleton of the hydrophobic polymer is coated with a very thin hydrophilic polymer-rich layer. However, in this Patent Literature, the deterioration and decomposition of the hydrophilic polymer during the long period storage of the membrane.is not taken into consideration, as is apparent from the high nozzle temperature and the use of the air drying.

Patent Literature 29: JP-A-2005-58906 (2005)

Patent Literature 30 discloses a method for sterilizing a dialyzer for purifying blood, comprising the steps of wetting membranes with deaerated water or an aqueous solution of a substance harmless to human bodies, and sterilizing the membranes in the wet state with high-pressure steam. The technique disclosed in this Patent Literature is intended to simplify the priming operation, but no technique relating to the performance-exhibiting rate of the membrane and the stabilization of the performance after the priming is described.

Patent Literature 30: JP-A-7-148251 (1995)

Patent Literature 31 relates to a blood purifier comprising a dry type polyvinyl pyrrolidone-containing polysulfone-based permselective hollow fiber membrane bundle, wherein the amount of polyvinyl pyrrolidone which elutes from the hollow fiber membrane bundle is 10 ppm or less, and wherein the amount of hydrogen peroxide in an extract from every one of the sites of 10 portions into which the hollow fiber membrane bundle is divided in the lengthwise direction is 5 ppm or less, when each of such sites is subjected to a test regulated in the Approval Standard for Dialysis-Type Artificial Kidney Apparatus. However, this Patent Literature dose not refer to the wetability of the hollow fiber membranes after the priming treatment or to the performance-exhibiting rate thereof in relation to the wetability.

Patent literature 31: Patent Registration No. 3636199

Further EPO 997 182 A discloses a hollow fibre membrane having a PVP concentration in the outer sens 5-50%. EP 1518 564 A and EP 111 0563 discloses methods of packing and sterilisation of blood treatment devices.

BRIEF DESCRIPTION OF THE DRAWINGS

Fig. 1 shows a graph which schematically illustrates the amounts of hydrogen peroxide which elute from the respective sites of ten portions into which a hollow fiber membrane bundle is divided.
Fig. 2 shows a graph which schematically illustrates a relationship between the amount of hydrogen peroxide which elutes from a hollow fiber membrane and variation in amount of elution within a hollow fiber membrane bundle.
Fig. 3 shows a graph which schematically illustrates a relationship between a time from the sealing of the inlets and outlets of a blood purifier until $\gamma$-ray exposure and the water permeability-exhibiting rate of the blood purifier after a priming treatment.
Fig. 4 shows a graph which schematically illustrates that the relationship between the concentration of dissolved oxygen in water saturated with an inert gas and the amount of eluting hydrogen peroxide generally tends to vary depending on the intensity of a radioactive ray.
Fig. 5 shows a graph which schematically illustrates that the variation degree in elution amount generally tends to increase when the maximum amount of hydrogen peroxide which elutes from a hollow fiber membrane exceeds a given value.
Fig. 6 shows a graph which schematically illustrates a general relationship between the performance-exhibiting rate of a blood purifier after a priming treatment and a time until a sterilization treatment.

DISCLOSURE OF THE INVENTION

PROBLEMS TO BE SOLVED BY THE INVENTION

An object of the present invention is to provide a method of providing a sterilized blood purifier which is free from the foregoing problems of the conventional techniques, namely, a blood purifier which has high levels of blood compatibility, performance-sustaining property and safety when brought into contact with blood and which shows an excellent water permeability-exhibiting rate after a priming treatment and high reliability in long-term storage stability.

MEANS FOR SOLVING THE PROBLEMS

The present invention provides a method of providing a sterilized blood purifier according to cl.1

In this case the content of polyvinyl pyrrolidone in the uppermost layer of the outer surface of the permselective hollow fiber membrane is preferably from 25 to 50 mass %.

The blood purifier is packed with the bundle of the polysulfone-based permselective hollow fiber membranes containing polyvinyl pyrrolidone, adjusted in water content to 5 to 600 mass % by the use of deaerated water, and which is sealed at its all outlets and inlets for blood and a dialysate, is sealed in a packaging bag capable of shutting out an external air and water vapor and is then exposed to a radioactive ray.

Also the deaerated water in and around the polysulfone-based permselective hollow fiber membrane is preferably water saturated with an inert gas.

The concentration of dissolved oxygen in the deaerated water is preferably 0.5 ppm or less.

The blood purifier is exposed to a radioactive ray after at least 48 hours has passed since the sealing of all the outlets and inlets for blood and the dialysate.

Further, the content of polyvinyl pyrrolidone in the uppermost layer of the inner surface of the permselective hollow fiber membrane is from 5 to 50 mass %.

Further, preferably, the contents of the polysulfone-based resin and polyvinyl pyrrolidone in the permselective hollow fiber membrane are from 99 to 80 mass % and from 1 to 20 mass %, respectively.

EFFECT OF THE INVENTION

The blood purifier used in the present invention is of dry type, and therefore is light in weight, is not frozen and is hard to breed bacteria therein. The blood purifier shows an excellent water permeability-exhibiting rate after a priming treatment, and has an advantage in that the priming treatment is done in a shorter time. Further, no radical-trapping

agent is contained, and therefore, there is an advantage in that no previous operation of washing off a radical-trapping agent is needed. Furthermore, the present invention produces an effect which the conventional techniques have never achieved, namely, an effect that the deterioration of the permselective hollow fiber membrane due to radiation exposure can be inhibited even when the blood purifier in a dried state is exposed to a radioactive ray in the absence of a radical-trapping agent. Therefore, the blood purifier is excellent in long-term storage stability, since the amount of hydrogen peroxide formed by the above deterioration reaction is small. For example, the polysulfone-based permselective hollow fiber membrane loaded in the blood purifier is inhibited from forming hydrogen peroxide even when exposed to a radioactive ray, and thus, the deterioration of polyvinyl pyrrolidone, etc. induced by the hydrogen peroxide is inhibited. Therefore, the maximal UV absorbance of the blood purifier at a wavelength of 220 to 350 nm, regulated in the Approval Standard for Dialysis-Type Artificial Kidney Apparatus, can be kept at 0.10 or less, even after the long-term storage of the blood purifier. Therefore, the safety of the blood purifier can be ensured when the blood purifier is stored over a long period of time.

BEST MODES FOR CARRYING OUT THE INVENTION

Hereinafter, the present invention will be described in more detail.

A hollow fiber membrane bundle for use in the present invention comprises a polysulfone-based resin containing polyvinyl pyrrolidone. The term "polysulfone-based resin" referred to in the present invention is a collective name of resins having sulfone bonds. While there is no particular limit in selection thereof, examples of the polysulfone-based resin are polysulfone resins and polyethersulfone resins, both having repeating units of the following formulae, which are widely used as polysulfone-based resins and are commercially available with ease:

[Chemical formula 1]

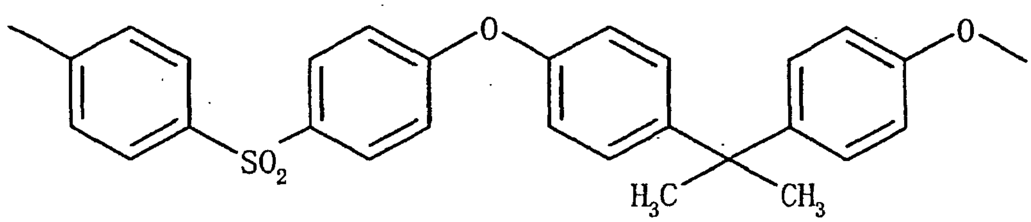

[Chemical formula 2]

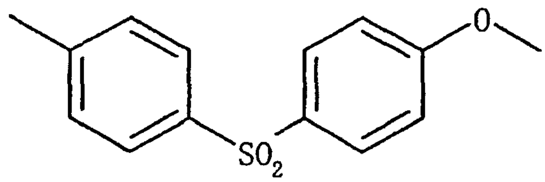

Polyvinyl pyrrolidone for use in the present invention is a water-soluble polymer which is obtained by vinyl-polymerizing N-vinylpyrrolidone and which is commercially available under the trade name of "Colidone" from BASF, "Plasdone" from ISP, or "Pitzcol" from DAI-ICHI KOGYO SEIYAKU CO., LTD., and each of these products has a variety of molecular weights. It is preferable to use polyvinyl pyrrolidone having a low molecular weight, in view of an efficiency of imparting hydrophilicity to membranes, while it is preferable to use polyvinyl pryrrolicone having a high molecular weight so as to decrease the eluting amount thereof. The kind of polyvinyl pyrrolidone may be appropriately selected in accordance with the required properties of a hollow fiber membrane bundle as a final product. That is, a single kind of polyvinyl pyrrolidone having a single molecular weight may be used, or otherwise, two or more kinds of polyvinyl pyrrolidone having different molecular weights may be used as a mixture. Further, a commercially available product may be purified for use as polyvinyl pyrrolidone which has a sharpened molecular weight distribution.

Preferably, the permselective hollow fiber membrane bundle of the present invention is manufactured using polyvinyl pyrrolidone which contains hydrogen peroxide in an amount of 300 ppm or less. When polyvinyl pyrrolidone as a raw material has a hydrogen peroxide content of 300 ppm or less, the amount of hydrogen peroxide which elutes from the resultant hollow fiber membrane bundle can be easily decreased to 5 ppm or less, and this is preferable because

the quality of the hollow fiber membrane bundle can be stabilized. The content of hydrogen peroxide in polyvinyl pyrrolidone for use as a raw material is more preferably 250 ppm or less, still more preferably 200 ppm or less, far still more preferably 150 ppm or less.

Hydrogen peroxide present in polyvinyl pyrrolidone as a raw material triggers the deterioration of polyvinyl pyrrolidone due to oxidation thereof, and hydrogen peroxide rapidly increases in amount, along with the proceeding of the oxidation deterioration of polyvinyl pyrrolidone. This is considered to further accelerate the oxidation deterioration of polyvinyl pyrrolidone. Accordingly, to reduce the content of hydrogen peroxide to 300 ppm or less is the first means to inhibit the oxidation deterioration of polyvinyl pyrrolidone in the course of the manufacturing of the permselective hollow fiber membrane. Another means to inhibit the deterioration of polyvinyl pyrrolidone as a raw material during the transport or storage thereof is also effective. For example, polyvinyl pyrrolidone is enveloped in an aluminum foil laminate bag to thereby be shielded from light. Preferably, an inert gas such as a nitrogen gas or the like is further enveloped in the same bag, together with an oxygen scavenger, when polyvinyl pyrrolidone is stored. When the packaging bag is opened to divide the polyvinyl pyrrolidone in small portions, the weighing and charging of the polyvinyl pyrrolidone are done under an atmosphere displaced with an inert gas, and preferably, such portions of polyvinyl pyrrolidone are stored under the above-described conditions. It is preferably recommended to displace the inner atmosphere of a supply tank with an inert gas in the course of the manufacturing of the hollow fiber membrane bundle. Polyvinyl pyrrolidone which is reduced in hydrogen peroxide content by the recrystallization method or the extraction method also may be used.

The process for manufacturing the permselective hollow fiber membrane of the present invention is not limited. However, preferably, the hollow fiber membrane of the present invention is manufactured by, for example, the process disclosed in JP-A-2000-300663. For example, polyethersulfone disclosed in this Literature (4800P manufactured by Sumitomo Chemical Company, Limited) (16 mass parts), polyvinyl pyrrolidone (K-90 manufactured by BASF) (5 mass parts), dimethylacetoamide (74 mass parts) and water (5 mass parts) are mixed and dissolved in one another, and the resultant solution is deaerated for use as a membrane-forming solution, and a 50% aqueous dimethylacetoamide solution is used as inner coagulation liquid. The membrane-forming solution and the hollow portion-forming solution are concurrently discharged from the outer side and the inner side of a double tube orifice and are introduced into a coagulation water bath of 75°C through a air gap section with a length of 50 cm, to thereby form a hollow fiber membrane in the bath. The hollow fiber membrane is then washed with water, wound up and dried at 60°C.

The content of the polyvinyl pyrrolidone to the polysulfone-based resin in the hollow fiber membrane is selected so that sufficient hydrophilicity can be imparted to the hollow fiber membrane. Preferably, the amount of the polysulfone-based resin is from 99 to 80 mass %, and that of polyvinyl pyrrolidone, from 1 to 20 mass %. When the content of polyvinyl pyrrolidone to the polysulfone-based resin is too small, the hydrophilicity-imparting effect to the hollow fiber membrane becomes poor, and also, the water permeability-exhibiting rate of the membrane after a priming treatment is likely to be insufficient, since the water permeability of the membrane lowers. Accordingly, the content of polyvinyl pyrrolidone is more preferably 1.5 mass % or more, still more preferably 2.0 mass % or more, far still more preferably 2.5 mass % or more. On the other hand, when this content is too large, the hydrophilicity-imparting effect saturates, and the amount(s) of polyvinyl pyrrolidone and/or the oxidation-deteriorated material which elute(s) from the hollow fiber membrane tend(s) to increase, with the result that the amount of polyvinyl pyrrolidone which elutes from the hollow fiber membrane is likely to exceed 10 ppm. Accordingly, the content of polyvinyl pyrrolidone is more preferably 18 mass % or less, still more preferably 15 mass % or less, far still more preferably 13 mass % or less, particularly 10 mass % or less.

In the present invention, preferably, the amount of polyvinyl pyrrolidone which elutes from the above polysulfone-based permselective hollow fiber membrane bundle is 10 ppm or less, all the sites of 10 portions, into which the hollow fiber membrane bundle is divided in the lengthwise direction, show 5 ppm or less in the eluting amount of hydrogen peroxide from the extracts of the 10 portions of the membrane bundle, which are subjected to a test regulated in the Approval Standard for Dialysis-Type Artificial Kidney Apparatus.

When the amount of hydrogen peroxide which elultes from the above-described site exceeds 5 ppm, the storage stability of the hollow fiber membrane bundle becomes poor because of the oxidation deterioration of polyvinyl pyrrolidone due to the hydrogen peroxide as described above, and in some cases, the amount of eluting polyvinyl pyrrolidone becomes larger, when the hollow fiber membrane bundle is stored over a long period of time. The degradation of the storage stability is most markedly indicated by an increase in the amount of eluting polyvinyl pyrrolidone. In addition, the deterioration of the polysulfone-based resin is induced to make the hollow fiber membrane fragile; or the deterioration of a polyurethane adhesive for use in the fabrication of a blood purifier is accelerated to increase the eluting amount of a deteriorated product such as urethane oligomer or the like, which may possibly lead to poor safety. Increases in the amounts of eluting substances which are produced by the oxidizing action of hydrogen peroxide during the long-term storage of the hollow fiber membrane bundle can be evaluated by measuring the absorbance of UV (220 to 350 nm) regulated in the Approval Standard for Dialysis-type Artificial Kidney Apparatus.

The amount of eluting hydrogen peroxide is also determined by using an extract obtained by a method according to the elution test method regulated in the Approval Standard for Dialysis-type Artificial Kidney Apparatus.

As described above, hydrogen peroxide, even if present in a specific site of the hollow fiber membrane bundle,

triggers the deterioration of the materials of the hollow fiber membrane bundle from such a site, and such deterioration transmits in a whole of the hollow fiber membrane bundle. Therefore, it is necessary that the amount of hydrogen peroxide present in the lengthwise direction of the hollow fiber membrane bundle for use in the blood purifier should be kept at a predetermined value or less in the overall sites thereof. That is, the oxidation and deterioration of polyvinyl pyrrolidone by hydrogen peroxide in a specific site sequentially transmits and spreads in a whole of the hollow fiber membrane bundle, and such deterioration further increases hydrogen peroxide in amount, and further, deteriorated polyvinyl pyrrolidone becomes easy to elute from the hollow fiber membrane bundle, since the molecular weight of such polyvinyl pyrrolidone becomes lower. This deterioration reaction sequentially proceeds. Accordingly, the hollow fiber membrane bundle, when stored over a long period of time, increases in eluting amounts of hydrogen peroxide and polyvinyl pyrrolidone, which is likely to lead to poor safety when the bundle is used in a blood purifier. For this reason, all the sites of 10 portions into which the polysulfone-based permselective hollow fiber membrane bundle is divided in the lengthwise direction should show preferably 5 ppm or less, more preferably 4 ppm or less, still more preferably 3 ppm or less, in the amount of eluting hydrogen peroxide, when measured in the respective sites.

To control the amount of eluting hydrogen peroxide within the above specified range, for example, it is effective to reduce, to 300 ppm or less, the content of hydrogen peroxide in polyvinyl pyrrolidone for use as a raw material. However, as mentioned above, hydrogen peroxide is also generated in the course of the manufacturing of hollow fiber membrane bundle, and it is therefore necessary to strictly control the conditions for manufacturing the hollow fiber membrane bundle. Particularly, the generation of hydrogen peroxide during the step of dissolving a membrane-forming solution and the drying step in the manufacturing process gives a significant influence on the eluting amount of hydrogen peroxide. Therefore, the optimization of the drying conditions is important. This optimization of the drying conditions can be a particularly effective means to lessen the variation in the amount of eluting hydrogen peroxide in the lengthwise direction of the hollow fiber membrane bundle.

Regarding the step of dissolving the membrane-forming solution, it is known that, when the membrane-forming solution comprising a polysulfone-based resin, polyvinyl pyrrolidone and a solvent is stirred and dissolved, and if the polyvinyl pyrrolidone contains hydrogen peroxide, hydrogen peroxide is explosively increased in amount because of the influence of oxygen in a dissolution tank and the influence of heating for the dissolution of the membrane-forming solution. Therefore, it is preferable to feed raw materials into a dissolution tank whose atmosphere is previously displaced with an inert gas. As the inert gas, a nitrogen gas, an argon gas or the like is preferably used. As required, a solvent or a non-solvent may be added. In such a case, preferably, the oxygen dissolved in the solvent or the non-solvent is displaced with an inert gas before the use of the solvent or the non-solvent.

As another method to inhibit the generation of hydrogen peroxide, it is also important to dissolve a membrane-forming solution in a shorter time. To form the solution in a shorter time, it is effective to raise the dissolving temperature and/or to increase the stirring rate. However, the deterioration and decomposition of polyvinyl pyrrolidone tend to proceed because of the influences of the temperature, the stirring linear velocity and the shearing force, when such measures are taken. In fact, as a result of the present inventors' researches, it is confirmed that the peak top of the molecular weight of polyvinyl pyrrolidone in a membrane-forming solution shifts in the decomposing direction (i.e. on the side of lower molecular weights) along with a rise in the dissolving temperature, and that a shoulder which seems to indicate a decomposed product appears on the side of the lower molecular weights. Thus, raising the dissolving temperature in order to improve the dissolving rate of raw materials accelerates the deterioration/decomposition of polyvinyl pyrrolidone, which leads to the blending of a decomposed product of polyvinyl pyrrolidone into the resultant permselective hollow fiber membrane. When such a hollow fiber membrane is used in, for example, a blood purifier, such a decomposed product elutes into the blood. Therefore, the quality and safety of such a blood purifier are inferior. To overcome this problem, the present inventors have tried to blend raw materials at a lower temperature in order to inhibit the decomposition of polyvinyl pyrrolidone. The dissolution of the raw materials at a temperature of as extremely low as a freezing point requires a high running cost. Therefore, the temperature is usually from 5 to 70°C, preferably 60°C or lower. However, a simple measure to lower the dissolving temperature requires a longer dissolving time which may induce the deterioration/decomposition of polyvinyl pyrrolidone and a lower efficiency of the operation and may require a large-scale apparatus. Therefore, this method has a problem in view of industrial manufacturing. Particularly when polyvinyl pyrrolidone is dissolved at a low temperature, polyvinyl pyrrolidone is not homogeneously dissolved, and it becomes difficult to further dissolve polyvinyl pyrrolidone, and a longer time is required to homogenously dissolve polyvinyl pyrrolidone.

As a result of the present inventors' extensive trials to dissolve polyvinyl pyrrolidone at a lower temperature and in a shorter time, they have found out that it is preferable to previously knead the components of a membrane-forming solution prior to the dissolution thereof, followed by dissolving them. The present invention is accomplished based on this finding. The components such as a polysulfone-based resin, polyvinyl pyrrolidone and a solvent may be kneaded at once, or polyvinyl pyrrolidone and the polysulfone-based resin may be separately kneaded. It is to be noted that, when polyvinyl pyrrolidone contacts oxygen, the deterioration of polyvinyl pyrrolidone is accelerated to generate hydrogen peroxide as described above. Accordingly, it is important to pay careful attentions so as to prevent the contact between polyvinyl pyrrolidone and oxygen: that is, the above kneading should be carried out under an atmosphere

displaced with an inert gas, and preferably, the kneading should be carried out in a separate line. In a further method, only polyvinyl pyrrolidone and a solvent may be previously kneaded, and a polysulfone-based resin may be directly supplied to a dissolution tank without previous kneading thereof. This method is also included in the scope of the present invention.

Separately from the dissolution tank, a kneading line may be provided for kneading the components, and then, the knead mixture may be supplied to the dissolution tank; or otherwise, a dissolution tank equipped with a kneading function may be used to carry out both of kneading and dissolution in the dissolution tank. In the former case where the kneading and the dissolution are carried out in the separate apparatuses, the type and mode of the kneading apparatus are not limited: that is, the kneading apparatus may be either of batch type or of continuous type. A static method using a static mixer or the like may be employed, or a dynamic method using a kneader or an agitation type kneader may be employed, although the latter type is preferable in view of the kneading efficiency. The latter dynamic kneading method is not limited, and it may be any of pin type, screw type, agitator type and the like, among which the screw type is preferable. The shapes and the number of revolutions of the screws may be optionally selected in consideration of a balance between the kneading efficiency and heat generation. On the other hand, the type of the dissolution tank equipped with the kneading function is not limited. However, it is recommended to employ, for example, a knead-dissolving apparatus of the type which exhibits a kneading effect by its so-called planetary motions: that is, two frame type blades self-rotate and revolve round each other in the apparatus, and examples thereof are a planetarium mixer and a trimix of INOUE MFG. INC.

The ratio of the resin components, i.e. polyvinyl pyrrolidone and the polysulfone-based resin to the solvent in the kneading step is not limited. However, the mass ratio of the resin components to the solvent is preferably from 0.1 to 3, more preferably from 0.5 to 2.

As described above, the technical point of the present invention is to inhibit the deterioration of polyvinyl pyrrolidone and simultaneously to efficiently dissolve the same. Therefore, preferably, in a system in relation to at least polyvinyl pyrrolidone, the kneading and dissolving are carried out at a temperature not higher than 70°C under a nitrogen atmosphere. In case where polyvinyl pyrrolidone and the polysulfone-based resin are kneaded in separate lines, respectively, the above method may be applied to the kneading line for the polysulfone-based resin. There is a relationship of antinomy between the efficiencies of kneading and dissolving and heat generation. The use of an apparatus capable of avoiding this antinomy relationship and the selection of conditions are important factors of the present invention. In this sense, a cooling method for the kneading mechanism is important, and careful attentions should be paid thereto.

Subsequently, the knead mixture kneaded by the above method is dissolved. While the dissolving method is not limited, for example, a dissolving method using a stirrer type dissolving apparatus may be employed. To dissolve the knead mixture at a lower temperature and in shorter time (within 10 hours), the Froude number ($Fr = n^2d/g$) is preferably from 0.7 to 1.3, and the Reynolds number ($Re = nd^2\rho/\mu$) is preferably from 50 to 250, wherein n represents the number of revolutions per second (rps) of the blades; $\rho$, the density (Kg/m$^3$); $\mu$, the viscosity (Pa.s); g, the gravitational acceleration (= 9.8 m/s$^2$); and d, the diameter of the stirring blade (m). When the Froude number is too large, the inertia force becomes stronger so that the raw materials fly in the tank and adhere to the wall and ceiling of the tank. As a result, a predetermined composition of a membrane-forming solution can not be obtained. Therefore, the Froude number is more preferably not larger than 1.25, still more preferably not larger than 1.2, far still more preferably not larger than 1.15. On the other hand, when the Froude number is too small, the force of inertia weakens so that the dispersibility of the raw materials tends to lower, and particularly, polyvinyl pyrrolidone can not be homogeneously dissolved, and is hard to be further dissolved, or it takes longer in homogenous dissolution thereof. Therefore, the Froude number is more preferably not smaller than 0.75, still more preferably not smaller than 0.8.

When the Reynolds number is too large, a longer time is required to deaerate the membrane-forming solution or the deaeration of the solution becomes insufficient, because of bubbles involved into the solution while the solution being stirred, since the membrane-forming solution of the present invention is a low viscous fluid. Therefore, the Reynolds number is more preferably not larger than 240, still more preferably not larger than 230, far still more preferably not larger than 220. On the other hand, when the Reynolds number is too small, the stirring force tends to be smaller so that non-homogenous dissolution is likely to occur. Therefore, the Reynolds number is more preferably not smaller than 35, still more preferably not smaller than 40, far still more preferably not smaller than 55, and particularly not smaller than 60. When a hollow fiber membrane is formed of such a membrane-forming solution, the filament-drawing property tends to lower due to the presence of bubbles, which leads to a lower operability. In addition, in view of the quality of the resultant membrane, the bubbles involved into the membrane induce a defect of the membrane at such a site, which leads to a poor air-tightness and a lower burst pressure of the membrane. The deaeration of the membrane-forming solution is effective, but such deaeration is likely to induce a change in the composition of the membrane-forming solution because of the control of the viscosity of the membrane-forming solution or the evaporation of the solvent. Therefore, deliberate attentions are needed to deaerate the membrane-forming solution.

Since polyvinyl pyrrolidone tends to decompose due to the oxidation thereof under the influence of oxygen in an air, the dissolution of the membrane-forming solution is preferably carried out under an inert gas atmosphere. As the

inert gas, nitrogen or argon is used, of which nitrogen is preferably used. In the dissolution of the membrane-forming solution, the concentration of the residual oxygen in the tank is preferably 3% or less. When the pressure for charging a nitrogen gas is increased, the dissolution time is expected to be shorter, while the cost for an apparatus capable of generating such a high pressure becomes higher, and the safety for operation becomes lower. In view of theses points, the pressure for charging an inert gas is preferably from the atmospheric pressure to 2 kgf/$cm^2$.

The stirring blades to be used in the present invention are of the shapes which are used for dissolving a low viscous membrane-forming solution, and examples of such blades include, but not limited to, radial stream types such as disc turbine type, paddle type, curved blade fan turbine type and feather turbine type; and axial flow types such as propeller type, inclined paddle type and Faudler type.

The above-described low temperature dissolving method makes it possible to inhibit the deterioration/decomposition of polyvinyl pyrrolidone and to provide a highly safe hollow fiber membrane. More specifically, it is preferable to use a membrane-forming solution which has been left to stand within a residence time of 24 hours after the dissolution of raw materials. This is because it is recognized that thermal energy, accumulated while the membrane-forming solution is thermally insulated, tends to deteriorate the raw materials.

To control the amount of eluting hydrogen peroxide within the specified range, it is important to avoid the contact with oxygen in the drying step. For example, the hollow fiber membrane is dried under an atmosphere displaced with an inert gas. However, this method is disadvantageous in view of cost. As an inexpensive drying method, it is effective and recommended to dry the hollow fiber membrane by exposure to microwaves under reduced pressure. In general drying of a material by removing a liquid therefrom, it is known that either application of a reduced pressure or microwave exposures is singly employed. The use of a reduced pressure in combination with microwave at the same time so as to dry a material is generally hard to employ, in consideration of the properties of microwave. However, the present inventors have employed this combination which involves difficulties, in order to prevent the oxidation deterioration of polyvinyl pyrrolidone and to improve the safety of the hollow fiber membrane by reducing the amount of materials eluting from the hollow fiber membrane and to improve the productivity thereof. They further have optimized the drying conditions to thereby develop an economically advantageous method which is able to solve the above discussed problems.

The preferable conditions for this drying method are as follows: that is, a hollow fiber membrane bundle is dried by exposure to microwave having an output of 0.1 to 100 KW under a reduced pressure of 20 KPa or lower. Preferably, the frequency of the microwave is 1,000 to 5,000 MHz, and the highest temperature which the hollow fiber membrane bundle has while being dried is preferably 90°C or lower. The employment of the decompression method in combination is effective to accelerate the evaporation of the water content and thus has advantages in that the output of microwave can be lowered, in that the microwave exposure time can be reduced, and additionally in that the elevation of the temperature of the hollow fiber membrane bundle is suppressed at a relatively low temperature. Because of these advantages, the performance of the hollow fiber membrane bundle as a whole is hardly affected. A further excellent point of the drying under reduced pressure is that the drying can be carried out at relatively low temperature, which is particularly effective to markedly inhibit the deterioration and decomposition of polyvinyl pyrrolidone. Accordingly, a suitable drying temperature is sufficient within a range of from 20 to 80°C, more preferably from 20 to 60°C, still more preferably from 20 to 50°C, far still more preferably from 30 to 45°C.

The drying under reduced pressure means that the center portion and the outer peripheral portion of the hollow fiber membrane bundle are evenly decompressed, and thus, the evaporation of the water content is uniformly accelerated to thereby evenly dry the hollow fiber membranes. Consequently, it becomes possible to avoid damage of the hollow fiber membrane bundle due to uneven drying thereof. Further, the exposure to a microwave is effective to substantially evenly heat the center portion and the overall peripheral portion of the hollow fiber membrane bundle. As a result of the synergism of the even heating and the even decompression of the hollow fiber membrane bundle, an unique effect can be produced in the drying of the hollow fiber membrane bundle. The decompression degree may be arbitrarily selected according to the output of microwave, the total water content in the hollow fiber membrane bundle and the number of hollow fiber membrane bundles to be dried.. To prevent a rise in the temperature of the hollow fiber membrane bundle being dried, the decompression degree is preferably 20 kPa or lower, more preferably 15 kPa or lower, still more preferably 10 kPa or lower. Too high a decompression degree exceeding 20 kPa not only lowers the water content-evaporating efficiency but also raises the temperature of the polymer which composes the hollow fiber membrane bundle, to thereby induce the deterioration of the polymer. The higher the decompression degree, the better it is to inhibit a rise in the temperature of the polymer and to improve the drying efficiency. However, it costs higher to maintain the sealing degree of the apparatus. Accordingly, the decompression degree is preferably 0.1 kPa or higher, more preferably 0.25 kPa or higher, still more preferably 0.4 kPa or higher.

While microwave having a higher output is preferable to reduce the drying time, too high an output is undesirable because excessive drying or excessive heating is likely to deteriorate and decompose polyvinyl pyrrolidone, if a hollow fiber membrane bundle contains polyvinyl pyrrolidone, and because the excessive drying or excessive heating is likely to cause a problem in that such a hollow fiber membrane bundle is hard to wet in use. Even an output of lower than 0.1 kW is possible to dry a hollow fiber membrane bundle, however, such a low output requires a longer drying time, which

may leads to a poor treating efficiency. An optimal combination of a decompression degree with an output of microwave may be experimentally and appropriately selected according to the water content retained in a hollow fiber membrane bundle and the number of hollow fiber membrane bundles to be treated.

A rough standard for satisfying the drying conditions according to the present invention is described: for example, when 20 hollow fiber membrane bundles, each one having 50 g of water content, are dried, it is appropriate to set the output of microwave at 1.5 kW, and the decompression degree, at 5 kPa, relative to the total water content of 1,000 g (= 50 g X 20).

The output of microwave is more preferably from 0.1 to 80 kW, still more preferably from 0.1 to 60 KW. The output of microwave is determined from, for example, the total number of hollow fiber membrane bundles and the total water content. Sudden exposure to a microwave having a high output makes it possible to dry a hollow fiber membrane bundle in a shorter time, but is likely to partially denature the hollow fiber membrane and to shrink or deform the same. For example, the vigorous drying of a hollow fiber membrane bundle containing a water retainer or the like by exposure to a microwave having a high output is likely to fly and dissipate the water retainer. The method of drying by exposure to a microwave under a reduced pressure hitherto has never been invented, in consideration of the influence of the decompression on a hollow fiber membrane. According to the present invention, drying a hollow fiber membrane bundle by exposure to a microwave under a reduced pressure accelerates the evaporation of an aqueous liquid even at a relatively low temperature. This advantage provides double effects that the deterioration of polyvinyl pyrrolidone and the damage of the hollow fiber membrane such as deformation thereof attributed to the exposure to a microwave of high output and a high temperature can be prevented.

In the present invention, the method of drying by exposure to a microwave under reduced pressure can be carried out in one step with the output of the microwave kept constant. Otherwise, so-called multi-step drying in which the output of a microwave is sequentially and stepwise lowered along with the proceeding of the drying is also included as a preferable mode in the scope of the present invention. In this regard, the significance of the multi-step drying is described as follows. When a hollow fiber membrane bundle is dried by way of exposure to a microwave under a reduced pressure and at a relatively low temperature of from about 30 to about 90°C, the multi-step drying method is preferably employed in which the output of the microwave is sequentially decreased in accordance with the proceeding of the drying of the hollow fiber membrane bundle. In this method, the decompression degree, temperature, output of microwave and exposure time are determined in consideration of the total amount of the hollow fiber membranes to be dried, an industrially acceptable and appropriate drying time, and so on. The multi-step drying may be carried out in an optional number of steps, for example, 2 to 6 steps. However, in view of productivity, drying in 2 to 4 steps is industrially acceptable. In case where the total water content of a hollow fiber membrane bundle is relatively large, the multi-step drying is carried out, for example, at a temperature of not higher than 90°C under a reduced pressure of about 5 to about 20 kPa and by exposure to a microwave having an output which is stepwise changed: that is, the output of microwave is from 30 to 100 kW in the first step, followed by from 10 to 30 kW in the second step, 0.1 to 10 kW in the third step and so on, which is determined by taking the microwave exposure time into account. When a difference between each of the outputs of microwaves is large, for example, when the higher output of microwave in one step is 90 kW, and the lower output thereof in another step, 0.1 kW, the number of steps to sequentially decrease the output may be increased to, for example, 4 to 8. In the present invention, the technical idea of decompressing is applied to the microwave exposure, and therefore, there is produced an advantage that a hollow fiber membrane bundle can be dried at a relatively lower output of microwave. For example, the drying is carried out in the first step where the exposure to a microwave of an output of 10 to 20 kW is continued for about 10 to about 100 minutes, followed by the second step where the exposure to a microwave of an output of 3 to 10 kW is continued for about 5 to about 80 minutes, the third step where the exposure to a microwave of an output of 0.1 to 3 kW is continued for about 1 to about 60 minutes, and so on. Preferably, the output of a microwave and the exposure time in each of the steps are decreased in association with the decreasing degree of the water content in the hollow fiber membrane bundle. This drying method is very mild to the hollow fiber membrane bundle, which is not at all expected from the conventional methods, and this mild drying distinguishes the actions and effects of the present invention.

In another mode where the water content in a hollow fiber membrane bundle is 400 mass % or less, it is effective to dry the bundle by exposure to a microwave of a low output of 12 kW or lower. For example, when the total water content of a hollow fiber membrane bundle is so relatively small as about 1 to about 7 kg, the bundle is dried at 80°C or lower, preferably 60°C or lower, under a reduced pressure of about 3 to about 10 kPa, while the output of a microwave and the exposure time are being adjusted according to the drying degree of the bundle as follows: that is, exposure to a microwave having an output of 12 kW or lower, for example, from about 1 to about 5 kW, is continued for about 10 to about 240.minutes, followed by exposure to a microwave having an output of 0.5 to lower than 1 kW for about 1 to about 240 minutes, preferably about 3 to about 240 minutes, and exposure to a microwave having an output of 0.1 to lower than 0.5 kW for about 1 to about 240 minutes. By doing so, the drying is evenly carried out. The decompression degree may be roughly set at 0.1 to 20 kPa in each of the steps. However, the decompression degree in each of the steps may be appropriately set according to a situation, in consideration of changes in the decreases of the total water amount and

the water content of the hollow fiber membrane bundle. For example, in the first step where the water content of the hollow fiber membrane bundle is relatively large, the decompression degree is set at, for example, 0.1 to 5 kPa, and the output of microwave is set at 10 to 30 kW; and in the second and third steps, the drying is carried out under a reduced pressure of 5 to 20 kPa which is slightly higher than the pressure of the first step and by exposure to a microwave of an output of 0.1 to 5 kW. This operation of changing the decompression degree in each of the steps further distinguishes the significance of the present invention which rest in the drying by exposure to a microwave under a reduced pressure. Also, it is of course necessary to always pay careful attentions to even microwave exposure and an exhaust gas within the microwave exposure apparatus.

In the present invention, it is effective to employ the method of drying a hollow fiber membrane bundle by exposure to a microwave under a reduced pressure, in combination with a drying method by alternately inversing the air-flowing direction, although complicated steps are required. The drying method by exposure to a microwave and the drying method by alternately inversing the air flow direction have advantages and disadvantages in themselves, respectively. These methods in combination are employed in order to obtain a high quality product. It is also possible to dry a hollow fiber membrane bundle by employing the drying method by alternately inversing the air flow direction in the first stage, and employing the drying method by exposure to a microwave under a reduced pressure in the next stage, at a point of time when the hollow fiber membrane bundle has been dried to an average water content of about 20 to about 60 mass %. In this case, there may be employed a combined drying method which comprises the step of exposing a hollow fiber membrane bundle to a microwave to dry the same, followed by the step of drying the same by alternately inversing the air flow direction. How to select the methods in combination is determined in consideration of the quality of a hollow fiber membrane bundle obtained after the drying, particularly the quality of a polysulfone-based permselective hollow fiber membrane bundle which is to have no partial sticking of hollow fiber membranes in the lengthwise direction. While these drying methods can be concurrently carried out, this is not practically advantageous, since a complicated apparatus and complicated steps are required, which leads to a higher cost. Otherwise, an effective heating means such as far infrared rays or the like may be used in combination, and this is also included in the scope of the drying method according to the present invention.

The highest temperature which a hollow fiber membrane bundle being dried shows can be measured as follows: an irreversible thermo label is stuck to a side edge of a protective film for a hollow fiber membrane bundle, and the hollow fiber membrane bundle is dried, and the indication of the label is confirmed after the removal of the dried bundle. In this regard, the highest temperature of the hollow fiber membrane being dried is preferably not higher than 90°C, more preferably not higher than 80°C, still more preferably not higher than 70°C. When the highest temperature of the bundle exceeds 90°C, the structure of the membrane is likely to change, which may lead to poor performance of the resultant hollow fiber membrane bundle or to the oxidation deterioration thereof. Especially, it is necessary to prevent an increase in the temperature of a hollow fiber membrane bundle containing polyvinyl pyrrolidone as much as possible, because polyvinyl pyrrolidone is easily decomposed by heat. To prevent an increase in the temperature of the hollow fiber membrane bundle, it is effective to optimize the decompression degree and the output of a microwave and to intermittently expose the membrane bundle to a microwave. The lower the drying temperature, the better. However, the drying temperature is preferably 30°C or higher in view of cost for maintaining the decompression degree and the saving of the drying time.

The frequency of microwave is preferably from 1,000 to 5,000 MHz, more preferably from 1,500 to 4,000 MHz, still more preferably from 2,000 to 3,000 MHz, in view of an effect to inhibit exposure spots on a hollow fiber membrane bundle and an effect to push out water from the pores of the hollow fiber membrane bundle.

In the drying by exposure to a microwave, it is important to evenly heat and dry a hollow fiber membrane bundle. In the above-described drying by exposure to a microwave, uneven heating is caused due to reflected waves incidental to the generation of a microwave. Therefore, it is important to device a method for reducing the uneven heating due to the reflected waves. As such a method, the method disclosed in, for example, JP-A-2000-340356 can be preferably employed, in which a reflecting plate is provided in an oven to reflect such reflected waves to thereby evenly heat a hollow fiber membrane bundle, although the method is not limited to this one.

It is preferable to dry the hollow fiber membrane bundle by way of exposure to a far infrared ray after the water content in the hollow fiber membrane bundle has been reduced to 10 to 20 mass %. Exposure to a microwave or drying by heat (or an air) is preferred in view of quick drying of a subject to be dried. However, in case of a separation membrane containing polyvinyl pyrrolidone, there is a problem in that polyvinyl pyrrolidone is likely to deteriorate and decompose due to heat, in association with the proceeding of the drying, i.e. in association with decrease in the water content in the hollow fiber membrane. Accordingly, it is preferable to mildly dry the hollow fiber membrane bundle with a lower energy in the final stage of the drying (i.e. when the membrane bundle has had a low water content). The far infrared ray is a kind of electromagnetic waves, and penetrates the inside of a subject to be dried, as well as a microwave. Therefore, the use of the far infrared ray is preferable, because the subject can be uniformly dried without any spot, with a low energy.

Preferably, the wavelength of the far infrared ray is from 1 to 30 μm. Water and organic substances show higher far infrared absorption rates at a wavelength of from 3 to 12μm. Therefore, a far infrared ray with too short or too long

a wavelength makes it hard to raise the temperature of the subject to be dried, which leads to a longer drying time and a higher cost for drying. Therefore, the wavelength of the far infrared ray is preferably from 1.5 to 26 $\mu$m, more preferably from 2 to 22 $\mu$m, still more preferably from 2.5 to 18 $\mu$m.

As a radiation medium for exposure to the far infrared ray, it is preferable to use a stainless steel medium having a coating layer of a metal oxide on its surface. For example, the use of a far infrared radiator comprising austenite type stainless steel particles each coated with a layer of a metal oxide such as $Al_2O_3$, $Fe_2O_3$, $TiO_2$, CaO, MgO, $K_2O$, $Na_2O$ or the like is preferable, since a far infrared ray can be efficiently obtained at a lower cost.

In the meantime, in case where the hollow fiber membrane bundle is dried by way of exposure to a far infrared ray after the completion of drying by way of a microwave, no discharge phenomenon occurs even when the far infrared ray is irradiated under reduced pressure, differently from the drying by way of microwave. Accordingly, the drying can be conducted with a higher decompression degree, as compared with the drying by way of microwave. The decompression degree is preferably 5 kPa or less, more preferably 4 kPa or less, still more preferably 3 kPa or less, far still more preferably 2 kPa or less, in view of drying efficiency. Preferably, the energy of the far infrared ray exposure is controlled to 80°C or lower, more preferably 70°C or lower, as a temperature detected with a thermocouple provided at the center portion of an oven. The rate of converting a radiation caused by this far infrared exposure into an energy absorbed by water is high, and thus, the far infrared exposure is excellent in heat efficiency and is safe since the temperature control in accordance with the proceeding of the drying can be suitably made. This drying method by way of exposure to a far infrared ray is significant as a dry finishing for maintaining the surface effect of the hollow fiber membrane bundle, including the color, surface roughness, bending, cracking and smooth and flexible feeling thereof.

Specifically, a preferred drying method according to the present invention comprises a plurality of drying steps: that is, a drying step (1) of concurrently exposing a hollow fiber membrane bundle to a microwave and a far infrared ray, a drying step (2) of exposing the membrane bundle to a microwave, and a drying step (3) of exposing the membrane bundle to a far infrared ray. A suitable drying method (A) according to the present invention generally comprises the drying steep (1) of concurrently exposing a hollow fiber membrane bundle to a microwave and a far infrared ray, and the drying step (3) of exposing the membrane bundle to a far infrared ray after the water content in the membrane bundle has been decreased to a given value. Another drying method (B) according to the present invention comprises the drying step (2) of exposing a hollow fiber membrane bundle to a microwave, and the drying step (3) of exposing the membrane bundle to a far infrared ray after the water content in the membrane bundle has been decreased to a given value. Needless to say, it is essential that each of the drying steps is carried out under the suitable control of the temperature and the control of the pressure if the drying step is carried out under reduced pressure, and if needed, under ventilation and exhaustion of an air.

Theoretically, the combined use of the drying steps (1) and (2), the combined use of the drying steps (3) and (1), the combined use of the drying steps (2) and (3) and so on are possible, taken into consideration the site operation of a drying apparatus for carrying out the drying method of the present invention. However, the practical effects of these drying methods have not yet been sufficiently examined in comparison with the drying methods (A) and (B).

As described above, the exposure to a far infrared ray may be started after the completion of the exposure to a microwave, or may be done during the exposure to a microwave so that the drying by way of exposure to the microwave may be done simultaneously with the drying by way of exposure to a far infrared ray. By concurrently carrying out the exposure to a microwave and the exposure to a far infrared ray, the evaporation of water which is excited by the exposure to a microwave and is transferred to the surface of the hollow fiber membrane is accelerated by the exposure to a far infrared ray, which results in an improved drying efficiency. This efficient evaporation of the water content in the surface of the hollow fiber membrane is effective to inhibit the variation of the concentration of polyvinyl pyrrolidone in the surface of the hollow fiber membrane which is induced by the water content in the surface of the hollow fiber membrane, so that, advantageously, the partial sticking of the membranes is inhibited. As described above, the drying by way of exposure to a microwave is preferably carried out under reduced pressure. Therefore, preferably, the drying method is conducted by concurrently carrying out the drying by way of exposure to a microwave and the drying by way of exposure to a far infrared ray under reduced pressure, stopping the exposure to the microwave at a moment of time when the above water content has been achieved, and continuing the exposure to the far infrared ray to further dry the membrane bundle. In this stage, the decompression degree of the system is decreased after the completion of the exposure to the microwave so as to make conditioning, and then, the decompression degree is again increased to start the exposure to the far infrared ray. Accordingly, in the present invention, it is preferable to use a microwave drying apparatus which comprises a heating oven having a far infrared heater attached thereto and an exhaustion system for reducing the pressure in the heating oven, so as to dry the membrane bundle.

In case where the hollow fiber membrane bundle is dried by way of exposure to a microwave and exposure to a far infrared ray under reduced pressure while an additional condition of temperature being taken into consideration, in general, the water content of the membrane bundle is acceleratedly decreased, for example, by applying a microwave of high output to the membrane bundle at a high temperature under reduced pressure for a short time. However, the water content and polyvinyl pyrrolidone are unevenly present in the hollow fiber membrane bundle, which gives an

influence on the microwave heating to induce a bumping phenomenon, and this bumping damages the materials or the porous structure of the hollow fiber membrane bundle. Accordingly, such a hollow fiber membrane bundle can not have a structure surely withstanding a burst pressure. In the present invention, the output of the microwave and the far infrared ray are suitably controlled, and the temperature and the pressure are also controlled. By doing so, the drying of a whole of the hollow fiber membrane bundle including the inside and outside thereof, by way of exposure to a microwave is facilitated, while the drying of a whole of the hollow fiber membrane bundle including the surfaces thereof by way of exposure to a far infrared ray is facilitated. Thus, the drying by way of exposure to the microwave and the drying by way of exposure to the far infrared ray produce a synergistic drying effect.

In the present invention, preferably, an inert gas such as a nitrogen gas is used to return the inner pressure of the drying cabinet to a normal pressure after the completion of the drying. Since the temperature of the hollow fiber membrane bundle is high just after the completion of the drying, the feeding of an oxygen-containing gas such as an air for returning the inner pressure of the drying cabinet to a normal pressure is likely to oxidize and deteriorate polyvinyl pyrrolidone due to the oxygen and heat, if the hollow fiber membranes contain polyvinyl pyrrolidone. Therefore, the feeding of an inert gas to return the inner pressure to a normal pressure after the completion of the drying is effective to inhibit the oxidization and deterioration of polyvinyl pyrrolidone in the hollow fiber membrane bundle.

The drying of the hollow fiber membrane bundle by way of exposure to a microwave and a far infrared ray for an unlimited time does not necessarily give a good influence on the quality of the hollow fiber membrane bundle. It can be also supposed that the polysulfone resin or the polyvinyl pyrrolidone material, constituting the hollow fiber membrane bundle may be deteriorated by heat or an ambient atmosphere such as oxygen, water, steam or the like. Accordingly, in view of an industrial production, it is needed to consider an appropriate and acceptable drying time. The present inventors have found that the drying time from the start of drying to the completion thereof is preferably 3 hours or shorter, more preferably 2.5 hours or shorter, still more preferably 2 hours or shorter, in view of the protection of the quality of the hollow fiber membrane which is to be exposed to relatively severe drying conditions such as microwave and far infrared ray, and further in view of the industrial productivity.

Furthermore, preferably, the hollow fiber membrane should not be bone-dried. If bone-dried, the deterioration of polyvinyl pyrrolidone is likely to be accelerated, and the formation of hydrogen peroxide is likely to be markedly accelerated, although the particular reason therefor is not known. Further, the wetability of the hollow fiber membrane found when the membrane is rewetted before use tends to lower, or it makes hard for polyvinyl pyrrolidone to absorb water, which facilitates the elution of polyvinyl pyrrolidone from the hollow fiber membrane. The water content of the hollow fiber membrane found after the drying is preferably from 1 mass % to less than a saturation water content, more preferably 1.5 mass % or more. When the water content of the hollow fiber membrane is too high, breeding of bacteria may be facilitated during the storage of the hollow fiber membrane, fiber crushing may occur due to the weight of the hollow fiber membrane itself, or a failure in adhesion for assembling the blood purifier may occur. Therefore, the water content of the hollow fiber membrane is preferably 10 mass % or less, more preferably 7 mass % or less. In this regard, the water content referred to in the present invention is determined as follows: the mass (g) of a hollow fiber membrane bundle is measured; then, the membrane bundle is vacuum-dried under reduced pressure (-750 mmHg or lower) for 12 hours; then, the mass (g) of the dried membrane bundle is measured; and then, the water content of the bundle is determined from a decrease in amount (g) as a difference between the masses found before and after the drying of the membrane bundle, and from the mass (g) of the dried membrane bundle, by the following equation:

$$(\text{Decrease in amount}/\text{the mass of the dried membrane bundle}) \times 100 = \text{a water content (mass \%)}.$$

Further, washing off hydrogen peroxide which is introduced from polyvinyl pyrrolidone as the raw material or which is formed in the course of the production of the hollow fiber membrane bundle is effective as a method for controlling the above characteristic value within the specified range.

In the present invention, the amount of polyvinyl pyrrolidone which elutes from the hollow fiber membrane bundle is preferably 10 ppm or less, as described above.

When the eluting amount of polyvinyl pyrrolidone from the hollow fiber membrane bundle exceeds 10 ppm, the eluting polyvinyl pyrrolidone is likely to cause side effects or complications in a patient over a long term of hemodialyses. The method for achieving this feature may be optionally selected without any limit. For example, the content of polyvinyl pyrrolidone to the polysulfone-based resin is adjusted within the above-mentioned range; or otherwise, the hollow fiber membrane-forming conditions are optimized. The amount of polyvinyl pyrrolidone which elutes from the hollow fiber membrane bundle is more preferably 8 ppm or less, still more preferably 6 ppm or less, far still more preferably 4 ppm or less. The amount of polyvinyl pyrrolidone which elutes from the hollow fiber membrane bundle is determined by using an extract which is obtained according to the method regulated in the Approval Standard for Dialysis-type Artificial Kidney Apparatus. In detail, a hollow fiber membrane (1.0 g) is optionally removed from a dried hollow fiber membrane bundle,

and 100 ml of RO water is added to the hollow fiber membrane, followed by extraction therefrom at 70°C for one hour to obtain an extract.

There is no limit in selection of the method for decreasing the elution amount of polyvinyl pyrrolidone. Preferably, the content of polyvinyl pyrrolidone to the polysulfone-based resign, the membrane-forming conditions for the hollow fiber membrane and the washing method are optimized so that the above specified elution amount of hydrogen peroxide and the concentration of polyvinyl pyrrolidone in the surfaces of the membrane can be concurrently satisfied. Further, crosslinking by exposure to a radioactive ray is also effective.

In the present invention, preferably, the content of polyvinyl pyrrolidone in the uppermost layer of the outer surface of the hollow fiber membrane bundle is limited within a specified range, in order to obtain a balance among the characteristics such as the elution amount of polyvinyl pyrrolidone, inhibition of the infiltration of endotoxin into the blood side, and inhibition of the sticking of the hollow fiber membrane bundles in drying the same. As a method for meeting this requirement, for example, the content of polyvinyl pyrrolidone to the polysulfone-based resin is limited within the above specified range, or the hollow fiber membrane-forming conditions are optimized. It is also effective to wash the hollow fiber membrane bundle. As the membrane-forming conditions, the adjustment of the humidity in the air gap section at the outlet of a nozzle, the optimization of the drawing conditions, the temperature of the coagulation liquid, the composition ratio of the solvent to the non-solvent in the coagulation liquid, and the introduction of the washing step are effective.

In the present invention, it is important to carry out a washing step prior to the above-described drying step in the course of the manufacturing of the hollow fiber membrane bundle. The washing step is introduced as a means for decreasing the amount of eluting hydrogen peroxide or a means for controlling the content of polyvinyl pyrrolidone in the outer surface of the hollow fiber membrane within the specified range, as described above. For example, a wet hollow fiber membrane having passed through a water washing bath is directly wound onto a hank to make a bundle of 3,000 to 20,000 hollow fiber membranes. Next, the resultant hollow fiber membrane bundle is washed to remove excess of the solvent and polyvinyl pyrrolidone. In the present invention, preferably, the hollow fiber membrane bundle is immersed in hot water of 70 to 130°C or an aqueous solution of 10 to 40 vol % ethanol or isopropanol of room temperature to 50°C for washing.

(1) In case of washing with hot water, the bundle of hollow fiber membranes is immersed in an excess of RO water at a temperature of 70 to 90°C for 15 to 60 minutes, and then is removed therefrom, followed by centrifugal hydroextraction. This operation is repeated several times while the RO water being replaced with fresh one. Thus, the bundle of hollow fiber membranes is washed.

(2) Another method of washing the bundle of hollow fiber membranes may be employed, in which the bundle of the membranes is immersed in an excess of RO water in a compressed container at 121°C for about 2 hours for the treatment of the same.

(3) In case of washing with an aqueous ethanol or isopropanol solution, preferably, the same operation as in the method (1) is repeated.

(4) Also preferable is a washing method comprising the steps of radially arranging the bundle of hollow fiber membranes in a centrifugal washing container, and carrying out centrifugal washing for 30 minutes to 5 hours while shower-like spraying a washing water of 40 to 90°C to the bundle of membranes from the center of rotation.

In this regard, two or more of the above washing methods may be employed in combination. When the treating temperature is too low in any of the methods, it is needed to increase the washing operations in number, which leads to higher cost. When the treating temperature is too high, the,decomposition of polyvinyl pyrrolidone tends to accelerate, and the washing efficiency, on the contrary, tends to lower. The above washing makes it possible to optimize the content of polyvinyl pyrrolidone in the uppermost layer of the outer surface of the membrane, and to inhibit the sticking of the membrane or to decrease the amounts of the eluting substances, which leads to a decrease in the eluting amount of hydrogen peroxide.

The polysulfone-based permselective hollow fiber membrane bundle obtained by the above method is stored in a dried state for 3 months or longer, and then is subjected to a test regulated in the Approval Standard for Dialysis-type Artificial Kidney Apparatus. Preferably, the hollow fiber membrane bundle shows maximal UV absorbances of 0.10 or less in extracts therefrom, at all the sites thereof. This evaluation is made as follows: a dried sample is stored at a room temperature for 3 months in a dry box under an atmosphere (an air) of which the humidity is controlled to 50%RH, and then, the UV absrobance of the sample at a wavelength of 220 to 350 nm is measured. This feature is preferred when the manufacturing process and the transport of the hollow fiber membrane bundle, and the storage thereof in a dried state are taken into consideration.

In the present invention, preferably, the polysulfone-based permselective hollow fiber membrane bundle packed in a blood purifier shows a platelet-retaining rate of from 70 to 98% after 60 minutes has passed since the perfusion of human blood admixed with heparin at a flow rate of 150 ml/min. into the blood-contacting side of the blood purifier having

a membrane area of 1.5 $m^2$ (based on the inner diameter of the hollow fiber membrane).

To know an index which indicates blood compatibility, the adhesion of the platelets to the hollow fiber membrane bundle when the bundle contacts the blood is evaluated. Conventionally, studies to reduce the amount of adhered platelets (or to improve the platelet-retaining rate) have been made in order to improve the blood compatibility of the hollow fiber membrane bundle. It is considered that the activation of blood components due to contact with a material as a foreign matter to the organism is unavoidable in a certain sense independently of the activation degree. A membrane showing a very high platelet-retaining rate is evaluated to be apparently good ) in blood compatibility. However, when this is evaluated from another view point, the platelet activated upon contacting a material as a foreign matter are also likely to be released into the blood. As a result of further intensive studies from this view point, it is found that a preferable platelet-retaining rate is from 70 to 98%. When the platelet-retaining rate is lower than this lower limit, the amount of adhered platelets becomes larger, with the result that thrombus is more likely to occur or that the hemocathartic function tends to lower. Therefore, the platelet-retaining rate is more preferably 75% or higher, still more preferably 80% or higher. On the other hand, when the platelet-retaining rate is higher than the upper limit, the activated platelets are also released into the blood, so that the blood components such as blood cells and blood plasma, which circulate in the organism, are stimulated to activate a whole of the blood in the organism. As a result, there is a possible danger of inducing inopexia, or embolus, as the case may be. Therefore, the platelet-retaining rate is more preferably 97% or lower, still more preferably 96% or lower, far more preferably 95% or lower.

The platelet-retaining rate referred to in the present invention indicates a value which is calculated from the numbers of platelets in the blood found before and after the perfusion of the blood, by the following method:

(1) Heparin calcium is previously added to a blood-collecting bag so that the concentration of the heparin calcium can be 5 U/mL, and blood is collected from the intravenous of the inner elbow of a healthy adult into this blood-collecting bag. Prior to the perfusion of the blood, the blood is sampled for analysis of the blood components.

(2) The blood side and the dialysate side of a blood purifier comprising a hollow fiber membrane bundle having a membrane area of 1.5 $m^2$ are primed with physiologic saline, and the whole of the human blood admixed with heparin is perfused on the blood side of this blood purifier at a flow rate of 150 mL/min. In this connection, a circuit is formed so as to allow the blood flowing out of the blood-collecting bag to pass through the blood side of the blood purifier and to return to the blood-collecting bag.

(3) After the perfusion of the blood under an atmosphere of 37°C for 60 minutes, the blood is sampled to analyze the blood components.

(4) The platelet-retaining rate is calculated from the numbers of the platelets in the blood found before and after the perfusion of the blood, by the following equation.

(Platelet-retaining rate) [%] =
100 X [{the number of platelets in the blood after the perfusion) X (hematocrit in the blood before the perfusion)}/(hematocrit in the blood after the perfusion)] ÷ (the number of platelets in the blood before the perfusion)

As the index of blood compatibility, there is used a rate of increase in the platelet factor IV (hereinafter referred to as PF4) found when the blood is perfused in contact with the membrane of the blood purifier. When the blood contacts a foreign matter, the adhesion and activation of the blood cells are induced, and simultaneously, the coagulation system is also activated to finally form thrombus. The PF4 concentration indicates the degree of the activation of the platelets at this step. From the fact that the ratio of the concentrations of PF4 found before and after the perfusion of the blood (i.e. the rate of increase in PF4) is low, it is known that the platelets are hard to be activated, which means excellent blood compatibility. The rate of increase in PF4 in the blood-purifying membrane of the present invention is preferably 5 or less in multiplying factor, more preferably 3 or less in multiplying factor, still more preferably 2 or less in multiplying factor. The lower limit is 1.0.

As the index for the blood compatible performance-maintaining rate, a C characteristic value is known. The C characteristic value is a percentage of a value of the water permeability measured using the blood, found after 120 minutes has passed since the start of perfusion of the blood, to a value thereof found after 15 minutes has passed since the start of perfusion of the blood. A small C characteristic value means that the performance of the blood purifier lowers with time due to the adsorption of the blood components and so on. In view of the maintaining of the performance, the

C characteristic value of the hollow fiber type blood purifying membrane of the present invention is preferably 70% or more, more preferably 75% or more, still more preferably 80% or more. Generally, a treating time of 3 to 5 hours is needed in an ordinary hemodialysis. When the C characteristic value is less than 70%, the performance-maintaining property of the blood purifier is low, and thus, a sufficient treating effect sometimes can not be obtained. The water permeability of the blood purifier tends to lower with time due to the adsorption of the blood components during the blood perfusion. Accordingly, a high C characteristic value can be recognized to indicate that the adsorption of the blood components is small, and thus indicates the blood compatibility.

The present inventors have found that the above platelet-retaining rate has a correlation with a cationic dye-adsorbing rate of the hollow fiber membrane.

An important index of the blood compatibility of the surface of a material is, for example, a charged state, balance between hydrophilicity and hydrophobicity, non-specific adsorption capacity or the like of the membrane. The cationic dye-adsorbing rate of the hollow fiber membrane of the present invention is preferably from 40 to 70%. The cationic dye-adsorbing rate herein referred to can be considered to be an index for indicating the charged state, balance between hydrophilicity and hydrophobicity, non-specific adsorption capacity and the like. When the cationic dye-adsorbing rate is from 40 to 70%, the condition of the membrane surface is optimized, and thus, it is considered that such a membrane is excellent in biocompatibility. When the cationic dye-adsorbing rate is lower than the lower limit, the negative charging is too small, the static interaction with the platelets charged negative at their surfaces becomes larger to facilitate the adhesion of the platelets. Therefore, the cationic dye-adsorbing rate is.more preferably 43% or more, still more preferably 46% or more. When the cationic dye-adsorbing rate is larger than the upper limit, the hydrophobic interaction and the non-specific adsorption become larger to facilitate the adsorption of various blood components. Thus, the blood-purifying function tends to lower with time. Therefore, the cationic dye-adsorbing rate is more preferably 68% or less, still more preferably 65% or less, far still more preferably 63% or less.

The cationic dye-adsorbing rate referred to in the present invention indicates a value which is calculated from the concentrations of a cationic dye in a solution found before and after the perfusion of the solution by the following procedure:

(1) A cationic dye is dissolved in water to prepare a cationic dye solution with a concentration of 0.5 ppm.
(2) The cationic dye solution is sampled before contacting a membrane.
(3) One thousand milliliters of the cationic dye solution is measured and taken to fill the blood side and the dialysate side of a blood purifier having a membrane area of 1.5 $m^2$.
(4) After filling the blood purifier, the rest of the cationic dye solution is pooled and is then perfused on the blood side of the blood purifier at a flow rate of 200 mL/min. At this stage, a circuit is formed to allow the solution flowing out of the pool to pass through the blood side of the blood purifier and to return to the pool.
(5) After the perfusion for 5 minutes, the cationic dye solution filling the blood purifier is combined with the pooled cationic dye solution, and this mixture is sampled.
(6) An analytical curve is obtained from the absorbance (Abs$\lambda$max) at the maximum absorption wavelength ($\lambda$max) of the UV absorption spectrum of the cationic dye solution, and the concentrations of the cationic dye in the cationic dye solution found before and after contacting the membrane are measured.
(7) The cationic dye-absorbing rate is calculated by the following equation:

(The cationic dye-absorbing rate) [%] = 100 X (the concentration of the cationic dye in the solution after the perfusion)/(the concentration of the cationic dye in the solution before the perfusion).

There is no limit in selection of the cationic dye in the present invention. Examples of the cationic dye include methylene blule, crystal violet, toluidine blue, azur, etc., among which methylene blue is preferable since it is relatively inexpensive, easily available and low in harmfulness.

Polyvinyl pyrrolidone in the uppermost layer of the blood-contacting surface (i.e. the inner surface) of the membrane is considered to give a main influence on the blood compatibility, performance stability and performance-exhibiting rate of the membrane after a priming treatment. In the permselective hollow fiber membrane of the present invention, the content of polyvinyl pyrrolidone in the uppermost layer of the blood-contacting surface (or the inner surface) of the membrane is preferably from 5 to 50 mass %, more preferably from 10 to 40 mass %, still more preferably from 15 to 40 mass %. Too high or too low content of polyvinyl pyrrolidone in comparison with the above range is likely to

induce excessive adsorption of the blood components. When the content of polyvinyl pyrrolidone is higher than the upper limit, a lot of polyvinyl pyrrolidone is likely to elute upon contacting the blood, which may arise a problem in view of safety.

The blood compatibility of the membrane has significant connection with the physical properties thereof such as the very fine surface texture of the membrane. Preferably, the permselective hollow fiber membrane of the present invention has a mesh structure at its blood-contacting surface (or the inner surface). The mesh structure herein referred to means that the membrane is composed of very fine fibril-like structures, but not very fine particle-like structures. When the membrane has an inner surface composed of the aggregate of very fine particles, such a surface point-contacts with the blood cells and considerably stimulates the blood cells, and thus is likely to induce the activation of the blood cells. When the membrane has a smooth blood-contacting surface, the contact area between such a surface and the blood cells becomes larger, which may be likely to induce the activation of the blood cells as mentioned above. In comparison with these surface structures, the mesh structure of the membrane surface linearly contacts with the blood cells, and thus, the stimulation of the blood cells and the contact area between the surface and the blood cells are appropriate, and thus, the blood compatibility of such a surface of the membrane is considered to be good.

As concrete means for obtaining a permselective hollow fiber membrane with excellent blood compatibility which the present invention aims at, the following means are exemplified. The use of some of these means in suitable combination makes it possible to provide a permselective hollow fiber membrane with excellent blood compatibility.

1. Optimization of Reduced Viscosity of Polysulfone-Based Resin

The reduced viscosity of a polysulfone-based resin to be used is preferably from 0.15 to 0.6. Although the detailed mechanism is not known, the use of a polysulfone-based resin having such a reduce viscosity is considered to be preferable and effective to appropriately control the solidification of a hollow fiber membrane in a coagulation bath and to adjust the content of polyvinyl pyrrolidone in the blood-contacting surface of the hollow fiber membrane within the above preferable range. The reduced viscosity of the polysulfone-based resin is more preferably from 0.2 to 0.6, still more preferably from 0.3 to 0.6, far still more preferably from 0.35 to 0.58. Examples of the polysulfone-based resin having such a reduced viscosity include polyether sulfone manufactured by Sumitomo Chemical Company, Limited, such as Sumica-Exel® 3600P (reduced viscosity: 0.36), 4800P (reduced viscosity: 0.48), 5200P (reduced viscosity: 0.52), etc.

2. Optimization of Linear Velocity of Hollow Portion-Forming Agent and Dope Immediately After the Discharge Thereof from Nozzle

To manufacture a hollow fiber membrane, generally, a dope is discharged together with inner coagulation liquid from a tube-in-orifice nozzle, and is introduced into a coagulation bath through a air gap area, as described above. In this stage, preferably, the linear velocity of the inner coagulation liquid and the dope found immediately after the discharge thereof from a nozzle have a relationship of (the linear velocity of the inner coagulation liquid > the linear velocity of the dope), since a shear stress acts at the interface between the inner surface of the hollow fiber membrane and the inner coagulation liquid to cause friction therebetween so that suitable electric charges are imparted to them. Preferable conditions therefor will be described later.

3. Friction with Non-Conductive Material in Manufacturing of Membrane

In the course of manufacturing the hollow fiber membrane, the hollow fiber membrane being fed is allowed to contact a non-conductive material to thereby statically charge the hollow fiber membrane. This method is useful to impart a preferable property to the hollow fiber membrane, which the present invention is intended to impart. The non-conductive material to contact the hollow fiber membrane being fed is preferably used on a hollow fiber membrane-contacting members of a membrane-forming apparatus. The hollow fiber membrane-contacting members herein referred to are, for example, a guide, a roller and the like. The non-conductive material to be used is, for example, ebonite, Teflon®, ceramics or a metal material coated with any of these materials.

4. Spraying of Mist-like Water

Since mist-like water is very weakly charged, the permselective hollow fiber membrane is statically charged by spraying the mist-like water to the hollow fiber membrane. Thus, the preferable property is imparted to the hollow fiber membrane, as intended in the present invention. The above operation makes it possible to realize the preferable property by imparting static electricity, and simultaneously serves as a washing operation. Specifically, for example, in the hollow fiber membrane-forming step, water is sprayed to the hollow fiber membrane being fed to wash the hollow fiber membrane, and then, the hollow fiber membrane is dried and wound up; or otherwise, the hollow fiber membrane obtained through

the membrane-forming step is made into a bundle of membranes, and water is sprayed to this bundle to wash the same.

5. Use of Alkaline Earth Metal-Containing Water

Preferably, the amount of an alkaline earth metal contained in water for use in the inner coagulation liquid, the coagulation tank, the washing tank or the like, in the hollow fiber membrane-manufacturing process, is within a predetermined range. Although the detailed mechanism is not known, it is supposed that the alkaline earth metal present as a bivalent ion functions to mildly crosslink the carbonyl group and hydroxyl group of polyvinyl pyrrolidone and the oxygen atom of the ether bond, which are very weakly charged negative, and thereby optimizes the static and/or dynamic conditions of polyvinyl pyrrolidone in the hollow fiber membrane. Thus, the function of polyvinyl pyrrolidone as a hydrophilicity-imparting agent is optimized, and simultaneously, elution of polyvinyl pyrrolidone is inhibited, and further, the charged condition of the surface of the hollow fiber membrane is optimized. The total amount of the alkaline earth metal contained in water is preferably from 0.02 to 1 ppm, more preferably from 0.03 to 0.5 ppm. There is no limit in selection of the method for obtaining such water.. Preferably, water to be used in the manufacturing of the hollow fiber membrane is purified with a RO membrane so as to remove impurities. For example, a salt of metal is added to water after the purification thereof. To perfectly avoid the inclusion of impurities in the process of purification, ion exchanged water is used as raw water to be supplied to the RO membrane. For example, ordinary clean water is used as the raw water to remain traces of an alkaline earth metal in the resultant water. In the method of adding a metal salt to purified water, preferably, the water adjusted with metal ions is subjected to ultrafiltration to remove impurities before use.

6. Prevention of Excessive Drying of Hollow Fiber Membrane

The deterioration reaction of polyvinyl pyrrolidone in the uppermost layer of the inner surface of the hollow fiber membrane is one of important factors for the cationic dye-adsorbing rate. The deterioration reaction of polyvinyl pyrrolidone in the above uppermost layer acceleratedly proceeds when the hollow fiber membrane is excessively dried in the drying step for the hollow fiber membrane. For example, when the hollow fiber membrane with the high content of polyvinyl pyrrolidone in the uppermost layer is excessively dried, the deterioration reaction of polyvinyl pyrrolidone proceeds. When the content of polyvinyl pyrrolidone in the uppermost layer is high, the hydrophilicity of the hollow fiber membrane is essentially high so that the adsorption of methylene blue is inhibited and so that the blood compatibility of the hollow fiber membrane is supposed to be sufficient. However, it is experimentally found that the deterioration of polyvinyl pyrrolidone in the uppermost layer tens to increase the cationic dye-adsorbing rate and that the blood compatibility of the membrane lowers. Although the reason therefor is not definitely known, it is supposed that the pyrrolidone ring in polyvinyl pyrrolidone is opened to form a carboxyl group, so that the balance of negative charges in the inner surface of the hollow fiber membrane changes to thereby increase the cationic dye-adsorbing rate.

The water content in the hollow fiber membrane found after the completion of the drying gives a significant influence on the deterioration reaction of polyvinyl pyrrolidone due to the above excessive drying. When the water content is less than 1 mass %, the deterioration reaction acceleratedly proceeds. Therefore, preferably, the drying is stopped when the water content is 1 mass % or more.

The effect of the above inhibition of the excessive drying of the hollow fiber membrane is significant, because this effect significantly affects the control of the UV absorbance of an extract from the hollow fiber membrane within a preferable range specified in the present invention and the inhibition of the partial sticking of the membrane. Thus, this effect produces double effects.

The properties of the outer surface of the permselective hollow fiber membrane for use in the blood purifier of the present invention is also important, as well as the properties of the inner surface of the same.

In the present invention, the content of polyvinyl pyrrolidone in the uppermost layer of the outer surface of the permselective hollow fiber membrane is preferably from 25 to 50 mass %. When the content of polyvinyl pyrrolidone in the uppermost layer of the outer surface is less than 25 mass %, the content of polyvinyl pyrrolidone in a whole of the membrane, particularly the inner surface of the membrane, becomes too small, which is likely to lower the blood compatibility and water permeability of the membrane. In case of the membrane dried, the performance-exhibiting rate of the membrane after a priming treatment may be low. When a hemodialyzer is used for hemocatharsis, it is needed to allow physiologic saline to flow into the inside and the outside of the hollow fiber membrane of the hemodialyzer to thereby wet and defoam the membrane. In this priming operation, it is considered that the circularity of the hollow fiber membrane, the crushing and deformation of the end portion thereof and the hdyrophilicity of the material thereof.give some influences on the performance-exhibiting rate of the membrane after the priming treatment. When the hollow fiber membrane comprises a polyslufone-based resin and polyvinyl pyrrolidone and when the membrane is used in a dry membrane type blood purifier, the balance between the hydrophilicity and the hydorphobicity of the hollow fiber membrane gives a significant influence on the performance-exhibiting rate of the membrane after the priming treatment. Therefore, the content of polyvinyl pyrrolidone in the uppermost layer of the outer surface of the membrane is more preferably 27

mass % or more, still more preferably 29 mass % or more, far still more preferably 31 mass % or more. When the content of polyvinyl pyrrolidone in the uppermost layer of the outer surface exceeds 50 mass %, the possibility of infiltration of endotoxin in the dialysate into the blood side of the membrane is increased. This leads to side reactions such as fever or to poor workability for assembling the blood purifier because of the sticking of the hollow fiber membranes due to polyvinyl pyrrolidone present on the outer surfaces of the dried membranes. Therefore, the content of polyvinyl pyrrolidone in the uppermost layer of the outer surface is more preferably 47 mass % or less, still more preferably 43 mass % or less, far still more preferably 41 mass % or less.

The content of the above hydrophilic polymer in the uppermost layer of the hollow fiber membrane is measured and calculated according to the ESCA method described later, and this content is determined as an absolute value of the content in the uppermost layer (to a depth of several angstrom to several tens angstrom from the surface layer) of the hollow fiber membrane. According to the ESCA method, it is generally possible to measure the content of the hydrophilic polymer (PVP) in the uppermost layer until a depth of 10 nm (100 angstrom) from the surface of the permselective hollow fiber membrane.

In the present invention, the thickness of the permselective hollow fiber membrane is preferably from 10 to 60 $\mu$m. When this thickness exceeds 60 $\mu$m, the permeability of a substance having a medium or high molecular weight which is low in transfer velocity tends to lower, while the water permeability of the hollow fiber membrane is high. As the thickness of the hollow fiber membrane becomes thinner and thinner, the substance permeability of the hollow fiber membrane becomes higher. Therefore, the thickness of the hollow fiber membrane is more preferably 55 $\mu$m or less, still more preferably 50 $\mu$m or less, far still more preferably 47 $\mu$m or less. On the other hand, when this thickness is less than 10 $\mu$m, the strength of the hollow fiber membrane becomes lower, and further, it becomes difficult to maintain the water content of the hollow fiber membrane within a given range. Therefore, the thickness of the membrane is more preferably 25 $\mu$m or more, still more preferably 30 $\mu$m or more.

To adjust the contents of polyvinyl pyrrolidone in the uppermost layers of the inner surface and the outer surface of the hollow fiber membrane and the pore diameter of the hollow fiber membrane within the above specified ranges, respectively, the content of polyvinyl pyrrolidone to the polysulfone-based resin in the membrane-forming solution is adjusted to 65 : 35 to 90 : 10, or the membrane-manufacturing conditions are optimized. Otherwise, washing the hollow fiber membrane is also effective. As the effective membrane-manufacturing conditions, the humidity in the air gap is controlled; and the drawing condition, the temperature of a coagulation bath, the composition ratio of a solvent to a non-solvent in the coagulation bath, etc. are optimized. As the washing method, washing with hot water or alcohol and centrifugal washing are effective. Among those methods, the control of the humidity in the air gap and the optimization of the composition ratio of the solvent to the non-solvent in the outer coagulation bath are particularly effective as the membrane-manufacturing conditions; and the washing with alcohol is particularly effective as the washing method.

There is no particular limit in selection of the method for manufacturing the hollow fiber membrane bundle having the above-described characteristics. However, the manufacturing under the following conditions is preferable.

Preferably, the air gap is enclosed by a material capable of shutting out an external air. Preferably, the inner humidity of the air gap section is controlled by taking into consideration the composition of the membrane-forming solution, the nozzle temperature, the air gap length, and the temperature and the composition of the outer coagulation bath. For example, a membrane-forming solution of the composition: polyether slufone/polyvinyl pyrrolidone/dimethylacetoamide/RO water = 10 to 25/0.5 to 12.5/52.5 to 89.5/0 to 10.0 is discharged from a nozzle of 30 to 60°C and is allowed to pass through an air gap with a length of 50 to 1,000 mum and is then introduced into an outer coagulation bath with a concentration of 0 to 70 mass % and of 50 to 80°C. In this case, the absolute humidity of the air gap section is from 0.01 to 0.3 kg/1 kg of a dry air. By controlling the humidity of the air gap section within this range, surface pore ratio the average pore area and the content of polyvinyl pyrrolidone of the outer surface of the hollow fiber membrane can be controlled within suitable ranges, respectively.

The air gap length is more preferably from 100 to 900 mm, still more preferably from 200 to 800 mm. Too long an air gap length induces fusion of fibers due to fiber cutting and fiber swinging, and thus, the membrane-forming stability becomes lower. On the other hand, too short an air gap length induces insufficient proceeding of phase separation, and thus, uniform pore diameter can not be obtained.

To manufacture the hollow fiber membrane, a dope is discharged together with inner coagulation liquid from a tube-in-orifice nozzle and is introduced into a coagulation bath through a air gap section. When the linear velocity of the inner coagulation liquid and the dope found immediately after the discharge thereof meet a relationship of (the linear velocity of the discharged inner coagulation liquid > the linear velocity of the discharged dope), a shear stress acts at the interface between the inner surface of the hollow fiber membrane and the inner coagulation liquid to cause friction there between so that the inner surface of the membrane is appropriately charged. More preferably, the linear velocity of the discharged inner coagulation liquid is 3 to 10 times faster than that of the discharged dope. When this multiplying factor is smaller than 3, a stress at the interface between the inner surface of the hallow fiber membrane and the inner coagulation liquid is small, which is likely to make it impossible to suitably control the electrical charging of the inner surface of the membrane. When this multiplying factor is larger than 10, the pressure loss of the spinneret becomes

larger so that the discharged amount varies. As a result, the shape of the hollow fiber membrane becomes non-uniform. Further, the dope-discharging velocity is preferably 10,000 cm/min. or less. When the dope-discharging velocity is more than 10,000 cm/min., the pressure loss of the spinneret becomes larger, and the discharged amount tends to vary. As a result, the membrane-forming operation becomes instable, and the structure of the resultant hollow fiber membrane becomes non-uniform.

The discharge linear velocity and the ratio of the linear velocity can be calculated by the following equation:

(Discharge linear velocity just after discharging) (cm/min.) = a discharged amount (ml/min.)/(the area of a discharge hole) ($cm^2$)

(Ratio of linear velocity) = (the discharge linear velocity of the inner coagulation liquid)/(the discharge linear velocity of the dope)

The inner coagulation liquid is an aqueous solution of preferably 0 to 80 mass %, more preferably 20 to 70 mass %, still more preferably 25 to 60 mass %, far still more preferably 35 to 50 mass %, of dimethylacetoamide (DMAc). By controlling the concentration of the inner coagulation liquid within this range, a pore diameter capable of satisfying both of solute permeability and polyvinyl pyrrolidone- and endotoxin-cutting property can be obtained. Although particular reason therefor is not known, it can be supposed that the use of a mixed solution of water and dimethylacetoamide is effective to change the mobility of the polysulfone-based resin and polyvinyl pyrrolidone to take suitable balance in the ratio of hydrophilicity to hydrophobicity in the blood-contacting surface of the hollow fiber membrane. When the concentration of the inner coagulation liquid is too low, a dense layer on the blood-contacting surface becomes thick, which is likely to lead to poor solute permeability. On the other hand, when this concentration is too high, the formation of a dense layer is likely to be incomplete, and polyvinyl pyrrolidone and endotoxin (fragment) in the hollow fiber membrane is likely to elute into the blood. Thus, the biocompatibility (or blood compatibility) of the hollow fiber membrane becomes poor.

The outer coagulation liquid is preferably an aqueous solution of 0 to 40 mass % of N-methyl-2-pyrrolidone (NMP) or DMAc and of 10 to 80°C. When the temperature and concentration of the outer coagulation liquid are too high, the surface pore ratio and the average pore area of the dialysate-side surface of the hollow fiber membrane become too large, and thus, the backflow of endotoxin (fragment) into the blood side of the hollow fiber membrane tends to increase in amount. When the temperature and concentration of the outer coagulation liquid are too low, a large amount of water is needed to dilute the solvent brought from the membrane-forming solution, and disposal of the waste liquid requires higher cost. Therefore, the temperature and concentration of the outer coagulation liquid are more preferably from 20 to 80°C and from 0 to 35 mass %, still more preferably from 30 to 80°C and from 0 to 30 mass %, far still more preferably, from 40 to 80°C and from 5 to 30 mass %, particularly from 50 to 80°C and from 10 to 30 mass %, respectively.

In the manufacturing of the hollow fiber membrane of the present invention, it is preferable not to substantially draw the hollow fiber membrane after the structure thereof has been perfectly fixed. The wording of "not substantially draw" means that the hollow fiber membrane removed from the outer coagulation liquid is fed in the next step, under such a tension that does not relax the hollow fiber membrane being fed, and is finally wound up onto a hank. When the hollow fiber membrane perfectly fixed in its membrane structure is drawn, the pores of the membrane deform, crush, tear and orientate, which permits more elution of polyvinyl pyrrolidone and more infiltration of endotoxin into the blood side. In the meantime, it is difficult to form the hollow fiber membrane while the velocity of all the rollers are being equally controlled, since the hollow fiber membrane being fed is drawn longer by friction due to the contact with the members or the resistance of the liquid in the manufacturing process. The tension that does not relax the hollow fiber membrane specifically means that the velocity of the rollers for use in the manufacturing process are controlled so as not to relax or excessively tense the semi-solid fiber of the membrane-forming solution, discharged from the nozzle. The draw ratio herein referred to means a ratio of the velocity of the rollers. The draw ratio of the rollers is preferably from 0.01 to 1.5%, more preferably from 0.05 to 1%, still more preferably from 0.1 to 0.7%.

The ratio of the discharge linear velocity to the velocity of the first roller in the coagulation bath (i.e. draft ratio) is preferably from 0.7 to 1.8. When the draft ratio is smaller than 0.7, the hollow fiber membrane being fed is relaxed, which may leads to a lower productivity. Therefore, the draft ratio is more preferably not lower than 0.8, still more preferably not lower than 0.9, far still more preferably not lower than 0.95. When the draft ratio exceeds 1.8, the structure

of the hollow fiber membrane is likely to be broken because of the tearing of the dense layer of the hollow fiber membrane and so on. Therefore, the draft ratio is more preferably not larger than 1.7, still more preferably not larger than 1.6, far still more preferably not larger than 1.5, particularly not larger than 1.4. By controlling the draft ratio within this range, the deformation or destruction of the pores can be prevented, and the pores are prevented from clogging due to the protein in the blood, with the result that the hollow fiber membrane can exhibit reliable performance with time and sharp fractionation characteristics.

To suitably charge the surface of the hollow fiber membrane negative, it is preferable to decrease static electrical charges on the surface of the hollow fiber membrane. The static electrical charges on the surface of the hollow fiber membrane mainly occur due to drying or friction. To prevent the drying of the hollow fiber membrane, the hollow fiber membrane is not bone-dried in the drying step or is treated with glycerin. The concentration of an aqueous glycerin solution to be used for the treatment is preferably from 10 to 70 mass %, more preferably from 15 to 65 mass %. Otherwise, it is effective to destaticize an air for use in the drying step. The destaticizing treatment is conducted as follows: a destaticizer capable of generating positive and negative ions is used to impart, to the hollow fiber membrane, ions having an opposite pole to that of the charges on the hollow fiber membrane, corresponding to the charged amount of the membrane, to thereby neutralize the static electric charges on the membrane. As the method for imparting the ions having the opposite pole corresponding to the charged amount, a method using an ion current control system destaticizer is employed to directly destaticize the hollow fiber membrane. The ion current control system operates as follows: an ion current generated by an electric potential difference between a charged material and the earth electrode of the destaticizer is sensed to thereby grasp the charged condition of the charged material, and then, a time (or a pulse width) for applying a high voltage to the positive and negative electrode needles is controlled so as to impart ions having the opposite pole in correspondence with the charged amount. As a method for preventing friction which causes static electric charges, it is effective to optimize the materials for the rollers and the guides of the membrane-forming apparatus. As the materials for the roller and the guides, there are given Teflon®, bakelite, stainless steel, plastics, etc., among which stainless steel is suitable, since the use thereof can minimize the friction with the hollow fiber membrane. Preferably, the rollers and the guides have gentle curves in their shapes, at their sites to contact the hollow fiber membrane, so as to minimize the friction with the hollow fiber membrane. It is also preferable to provide an earth. By controlling the steps so as not to cause static electric charges and friction in this way, the negative electric charges intrinsic to the polysulfone-based resin can be suitably controlled.

The type of the blood purifier of the present invention is not limited. However, a blood purifier of the type in which a permselective hollow fiber membrane bundle is inserted in a container and in which both ends of the membrane bundle are fixed with a resin is preferable. An example of this blood purifier is shown in Fig. 1.

The blood purifier 1 is assembled as follows: the permselective hollow fiber membrane bundle 3 is inserted in the tubular housing 2: both end portions of the hollow fiber membrane bundle 3 are fixed to both end portions of the housing 2 with an adhesive 4 or the like; and both end portions of the housing 2 are covered with the caps 5a and 5b, respectively. The projected inlet 6a for introducing a dialysate into the housing 2 is formed in the proximity of one end portion of the housing 2, and the projected outlet 6b for discharging the dialysate is formed in the proximity of the other end portion of the housing 2. The projected inlet 7a for introducing the blood into the housing 2 is formed on the cap 5a, and the projected outlet 7b for discharging the blood is formed on the cap 5b.

As indicated by the arrowhead A, the blood enters from the blood-introducing inlet 7a into a space defined by the cap 5a and one end portion of the permselective hollow fiber membrane bundle 3, passes through the hollow fiber membrane of the bundle 3; and then, the blood enters into a space defined by the other end portion of the membrane bundle 3 and the cap 5b and flows out from the blood-discharging outlet 7b as indicated by the arrowhead B. On the other hand, as indicated by the arrowhead C, the dialysate enters from the dialysate-introducing inlet 6a into the housing 2, and flows alongside the outside of the hollow fiber membrane of the bundle 3, and flows out from the dialysate-discharging outlet 6b as indicated by the arrowhead D. In this regard, the blood to be dialyzed and the dialysate are allowed to flow in directions opposite to each other, which is so-called counterflow. During this operation, waste produce in the blood flowing in the permselective hollow fiber membrane is dialyzed into the outside.dialysate through the hollow fiber membrane.

As the materials for the housing and the caps, polycarbonate, polyester, polypropylene, etc. are exemplified. As the material for the adhesive for use in fixing both end portions of the bundle, polyurethane resins, epoxy resins, silicone resins, etc. are given.

The method for inserting the adhesive for fixing the end portions of the bundle is not limited. In this regard, a centrifugation bonding method is recommended, in which the adhesive is inserted by making use of a centrifugal force caused by rotating the blood purifier. Also, this centrifugation bonding method is not limited. For example, filling jigs are attached to both ends of the housing loaded with the dried permselective hollow fiber membrane bundle, and the housing is set on a centrifugation-bonding machine. Predetermined amounts of unhardened adhesives are inserted from the dialysate inlet 6a and the dialysate outlet 6b at and around a room temperature, while the centrifugation bonding machine is being rotated at a predetermined rotating velocity. The, the temperature of the centrifugation bonding machine is

increased to a hardening temperature of the inserted adhesive, to thereby complete the hardening of the adhesive, or to thereby pre-harden the adhesive until at least the flowability of the adhesive is eliminated. Then, the centrifugation bonding machine is stopped. In the latter case, the housing in a still state is heated to post-harden the adhesive so that the adhesive is completely hardened. This centrifugation bonding method may be a double layer centrifugation bonding method, in which the inside of the permselective hollow fiber membrane bundle is plugged with a flexible resin layer to reinforce the permselective hollow fiber membrane at the adhesive interface.

In the above centrifugation bonding method, it is important to uniformly insert the adhesive in a whole of the space inside the hollow fiber membrane bundle. Failure in adhesion occurs when the adhesive is not uniformly inserted so that the amount of the inserted adhesive becomes insufficient at some sites in the space. Particularly when the sticking of the hollow fiber membranes is present, penetration of the adhesive is hindered. To disentangle this sticking portion of the membranes, for example, a nozzle is used to blow an air onto the end portion of the hollow fiber membrane bundle; that is, so-called membrane-spreading treatment is made on the hollow fiber membrane bundle. However, this treatment is undesirable in spite of its disentangling effect on the stuck hollow fiber membranes, because the end portion of the hollow fiber membrane bundle is deformed to slant the hollow fiber membranes.

The permselective hollow fiber membrane bundle of the present invention is inhibited from the partial sticking thereof when dried, and thus, the uniform insertion of the adhesive is ensured without any membrane-spreading treatment. However, it is preferable to take the following measure, since it is important to ensure the uniform insertion of the adhesive. For example, the use of an adhesive having a low viscosity is preferred. In case of a two-part adhesive, the viscosity of the adhesive found after 2 minutes since the mixing of two components is preferably 2,000 mPa.s or lower, more preferably 1,600 mPa.s or lower. In addition, it is preferable to decrease the packing density of the permselective hollow fiber membrane bundle which is wrapped in a packaging material hollow inside when the hollow fiber membrane bundle is inserted into the housing for assembling the blood purifier.

The number and length of the hollow fiber membranes of the bundle to be inserted may be appropriately selected according to a demand of the market and the characteristics of the hollow fiber membrane bundle. The length and diameter of the housing are selected according to the size of the hollow fiber membrane bundle to be inserted.

It is essential to sterilize the blood purifier. As the sterilization treatment, sterilization by way of exposure to a radioactive ray such as $\gamma$-ray or an electron beam is preferable because of its reliability and simplicity. However, by the exposure to a radioactive ray, polyvinyl pyrrolidone is deteriorated, and hydrogen peroxide is generated, and hydrogen peroxide which is present during the radiation exposure concurrently accelerates the generation of hydrogen peroxide. Therefore, it is preferable to maintain the above-described characteristics even after the radiation exposure treatment. To impart the above-described characteristics even after the radiation exposure treatment, the use of a polysulfone-based permselective hollow fiber membrane which has the above-described characteristics before the radiation exposure treatment is important, however, this matter is merely one of the essential requirements. This requirement is satisfied, and then, a further treatment for inhibiting the deterioration reaction of the membrane due.to the radiation exposure is needed.

In the present invention, the water content of the polysulfone-based permselective hollow fiber membrane bundle is preferably 600 mass % or less. It is also preferable to contain no radical-trapping agent in the blood purifier during the radiation exposure. When the water content of the hollow fiber membrane bundle exceeds 600 mass %, the handling of the blood purifier becomes harder because of the increased weight, or the cost for the transport thereof increases, or bacteria easily proliferate, or such a blood purifier is frozen in a cold region. Further, polyvinyl pyrrolidone is excessively crosslinked, which may be likely to activate the coagulation reaction of the blood when such a membrane bundle is used in a blood purifier. On the other hand, when the water content of the hollow fiber membrane bundle is less than 0.8 mass %, the deterioration of polyvinyl pyrrolidone due to the radiation exposure is accelerated to increase hydrogen peroxide, carboxyl group and peroxides in amounts, to increase the UV absorbance (at 220 to 350 nm) of an extract from the hollow fiber membrane in a test regulated in the Approval Standard for Dialysis-Type Artificial Kidney Apparatus, and to lower the long term storage stability, the blood compatibility and the stability of the blood compatibility. Therefore, the water content is more preferably from 1.0 to 300 mass %, still more preferably from 1.5 to 200 mass %.

The object of the present invention, that is, to inhibit the deterioration reaction of polyvinyl pyrrolidone due to radiation exposure of a membrane bundle in a dried state and in the absence of a radical-trapping agent, is difficult to achieve. Conventionally, it is unavoidable to expose a membrane bundle in a wet state to a radioactive ray in the presence of a radical-trapping agent. As a result of the present inventors' intensive studies to solve this problem, it is supposed that the above deterioration reaction of polyvinyl pyrrolidone is accelerated by an oxygen gas adsorbed on a portion of a polysulfone-based permselective hollow fiber membrane at which polyvinyl pyrrolidone is locally present, and that such deterioration reaction of polyvinyl pyrrolidone is inhibited by water adsorbed on the portion of the same membrane at which polyvinyl pyrrolidone is locally present. Based on this supposed mechanism, the present inventors have found a method for inhibiting the deterioration reaction of polyvinyl pyrrolidone, and accomplished the present invention. While it is widely known that the above deterioration reaction of polyvinyl pyrrolidone is affected by oxygen, the phenomenon that the deterioration reaction of polyvinyl pyrrolidone is inhibited by traces of water content which is adsorbed on a

portion of the hollow fiber membrane at which polyvinyl pyrrolidone is locally present has been firstly discovered by the present inventors. Hereinafter, preferred modes of the present invention will be described.

The present inventors have found that a blood purifier comprising a permselective hollow fiber membrane which has the above-described characteristics and which is adjusted in water content to 5 mass % or more by using deaerated water when exposed in a dried state to a radioactive ray can inhibit the deterioration reaction of polyvinyl pyrrolidone even in the absence of a radial-trapping agent.

That is, preferably, a blood purifier, which is loaded with a polyvinyl pyrrolidone-containing polysulfone-based permselective hollow fiber membrane bundle adjusted in water content to 5 to 600 mass % by the use of deaerated water and which is tightly sealed at all of its inlets and outlets for blood and a dialysate, is sealed in a packaging bag capable of shutting out an external air and water vapor, and is then exposed to a radioactive ray.

In the present invention, the deaerated water present in and around the polysulfone-based permselective hollow fiber membrane is preferably deoxygenerated water, more preferably water saturated with an inert gas.

The deoxygenerated water is water of which the amount of dissolved oxygen is 0.5 ppm or less. The amount of dissolved oxygen is more preferably 0.2 ppm or less, still more preferably 0.1 ppm or less.

Generally, about 20 1 of an air is dissolved in 1 $m^3$ of water, and an oxygen gas is dissolved in ordinary tap water at a rate of 8 mg/1 liter of water. The method for preparing the deoxygenerated water is not limited, in so far as the above amount of dissolved oxygen is satisfied. The deoxygenerated water prepared by the publicly known deaerating method can be used. As the deaerating method, there are given the heat deaerating method, the vacuum deaerating method, the nitrogen gas-bubbling method, the membrane deaerating method, the reducer addition method, the reduction method, etc. The membrane deaerating method is particularly preferable, since it is possible to reduce the amount of dissolved oxygen to the level of ppb. The membrane deaerating method may be carried out by either the non-porous membrane method or the porous membrane method.

The present inventors have researched from many points of view in order to provide a highest quality blood purifier which is highly sterilized and which shows no decrease or variation in its quality even after a long period of storage thereof. As a result, they have known that the amount of water in a blood purifier and the technical attentions to the dissolved oxygen in water give very subtle influences. Based on the present inventors' knowledge, a highest quality blood purifier can be provided in a clinic site by paying careful technical attentions to an active oxygen generated due to radiation exposure, particularly in the step of sterilizing the blood purifier before the transport thereof. The amount of dissolved oxygen in water, which causes the generation of active oxygen, can be reduced by a simple treatment of water. However, when this treatment is applied to the blood purifier, it is needed to pay lots of careful attentions to the routes of oxygen through which oxygen in an air diffuses or infiltrats. Therefore, the use of water previously saturated with an inert gas rather than the above treatment of water is more effective to inhibit the infiltration of oxygen and is effective to facilitate the treatment of water.

The maximum amount, i.e. 5 ppm of eluting hydrogen peroxide can be set as a rough target value at which the variation in the eluting amount as shown in Fig. 3 can be suppressed relatively low to thereby provide a high quality blood purifier. The relationship between the maximum amount of hydrogen peroxide eluted from the hollow fiber membrane in the blood purifier and the variation in the eluting amount clearly indicates a technically significant boundary, as shown in the distribution of Examples and Comparative Examples, and it is recognized that the maximum eluting amount of hydrogen peroxide up to 5 ppm is an acceptable limit value. To provide a high quality blood purifier by suppressing, to a very low level, the variation in the amount of hydrogen peroxide eluted from the hollow fiber membrane bundle, it is preferable to set a target value at 5 ppm or less in the maximum eluting amount of hydrogen peroxide. As the maximum amount of eluting hydrogen peroxide increases more and more, the variation of the eluting amount tends to monotonically increase. This tendency proves that the use of water saturated with an inert gas in order to reduce the amount of dissolved oxygen in water, contained in the hollow fiber membrane bundle before the sterilization thereof and to inhibit the infiltration of oxygen into the bundle is effective to achieve the object, i.e. to provide the high quality blood purifier, and also proves that there is an advantage that very stable manufacturing steps can be established also in the manufacturing site.

Preferably, dissolved oxygen in water should be present in an amount so small as, for example, 0.001 ppm or less. However, technical difficulties for such a reducing operation give not a little influence on the cost for the blood purifier. Inevitably, there is a limit in reduction of such an amount. It is a rough reference to reduce the amount of dissolved oxygen to about 0.001 to about 0.5 ppm. By paying careful attentions to this reduction, the maximum eluting amount of hydrogen peroxide can be decreased to 5 ppm or less. In this regard, the amount of dissolved oxygen in water can be measured with a dissolved oxygen meter OM-51-L1 manufactured by HORIBA.

It is preferable to use deoxygenerated water which has been subjected to a reverse osmosis treatment (RO treatment).

The use of the above deoxygenerated water alone is insufficient and difficult to perfectly inhibit the above-described undesirable deterioration reactions, because oxygen in an ambient air is again dissolved in water, and because the redissolved oxygen gas is adsorbed onto a portion of the membrane at which polyvinyl pyrrolidone is locally present. This problem can be solved by the use of water saturated with an inert gas such as nitrogen. Since the water contains

an inert gas in a saturated state, an oxygen gas is inhibited from dissolving in water even under radiation exposure in an atmosphere containing oxygen. Thus, the concentration of oxygen in water is maintained low.

There is no limit in selection of the method for preparing the water saturated with an inert gas, and it is possible to employ a method of bubbling an inert gas such as nitrogen. As the method for removing dissolved oxygen in water, the inert gas-bubbling method is known. The dissolved oxygen in water is consequently removed by introduction of the inert gas. Otherwise, it is also preferable to dissolve an inert gas in water after the removal of oxygen. Specifically, an inert gas is bubbled in water from which oxygen is previously removed by the heat deaerating method, the vacuum deaerating method, the membrane deaerating method or the reducer-addition method. By doing so, the removal of the oxygen and the dissolution of the inert gas are efficiently carried out. The amount of dissolved oxygen in the water saturated with an inert gas is preferably 0.5 ppm or less, more preferably 0.2 ppm or less, still more preferably 0.1 ppm or less. In this connection, the water to be herein used is preferably subjected to a RO treatment.

The use of the above-described deaerated water makes it possible to more efficiently inhibit the deterioration of the hollow fiber membrane, particularly the deterioration reaction of polyvinyl pyrrolidone due to radiation exposure, than the use of non-deaerated water. Thus, there can be enhanced the effect of inhibiting the undesirable deterioration reactions which induce an increase in the amount of generated hydrogen peroxide, an increase in UV absorbance (at 220 to 350 nm) of an extract from the hollow fiber membrane in the test regulated in the Approval Standard of Dialysis-Type Artificial Kidney Apparatus, a decrease in anti-thrombogenic effect, and decreases in long-term storage stability and performance-exhibiting rate after a priming treatment.

In the present invention, in order to more effectively exhibit the effect of the use of the deaerated water for inhibiting the deterioration reactions due to radiation exposure, the oxygen concentration in the atmosphere inside the blood purifier before the sterilization is preferably 4.0 vol.% or less, more preferably 3.0 vol.% or less, still more preferably 2.0 vol.% or less. When this oxygen concentration exceeds 4.0 vol.%, the deterioration of the hollow fiber membrane, particularly the deterioration of polyvinyl pyrrolidone, is likely to be induced during the exposure to a radioactive ray or an electron beam, even if the above-described requirements are satisfied. On the other hand, when the oxygen concentration in the atmosphere inside the blood purifier before the sterilization is too low, the effect of sterilization by way of the radiation exposure may not be sufficiently exhibited, while the deterioration of the materials of the hollow fiber membrane and a potting material can be inhibited. Accordingly, the oxygen concentration in the atmosphere inside the blood purifier is preferably 0.1 vol.% or more, more preferably 0.2 vol.% or more, still more preferably 0.3 vol.% or more.

Preferably, the oxygen concentration in the atmosphere inside the blood purifier after the sterilization is 2.0 vol.% or less. When this oxygen concentration is too high, the materials of the hollow fiber membrane are oxidized and deteriorated, and the resultant deteriorated and decomposed products are likely to elulte into the blood during the use of the blood purifier for hemodialysis. Accordingly, this oxygen concentration is more preferably 1.8 vol.% or less, still more preferably 1.5 vol.% or less. On the contrary, this oxygen concentration in the atmosphere inside the blood purifier after the sterilization is preferably 0.01 vol.% or more. When this oxygen concentration is too low, the oxygen in the system may already have been consumed during the sterilization treatment, and thus, it becomes impossible to confirm whether or not sufficient sterilization effect can be achieved. Accordingly, the oxygen concentration in the atmosphere inside the blood purifier after the sterilization is more preferably 0.1 vol.% or more, still more preferably 0.5 vol.% or more.

In the above-described method, the mechanism for inhibiting the deterioration reaction of polyvinyl ) pyrrolidone during the radiation exposure is supposed as follows. It is supposed that polyvinyl pyrrolidone in the hollow fiber membrane is locally present but not evenly dispersed, and that water present in the inside and the surface of the hollow fiber membrane is selectively adsorbed onto the periphery of highly hydrophilic polyvinyl pyrrolidone, and thus is locally present in the hollow fiber membrane. Then, it is supposed that the water present around polyvinyl pyrrolidone blocks the attack of oxygen activated by the radiation exposure, onto polyvinyl ) pyrrolidone, to thereby inhibit the deterioration reaction of polyvinyl pyrrolidone. It is therefore supposed that the use of deaerated water makes it possible to more effectively exhibit the inhibition effect. It is further supposed that the use of the hollow fiber membrane of the present invention, which is reduced in the amount of hydrogen peroxide which is activated as well as oxygen by the radiation exposure to cause the deterioration reactions, is effective to further inhibit the above deterioration reaction. These double inhibition effects are supposed to establish the effect of the invention.

There is no limit in selection of the method for adjusting the oxygen concentration in the blood purifier. However, it is preferable to fill the blood purifier with an inert gas for such adjustment. As described above, the blood purifier is assembled using the hollow fiber membrane bundle dried by the above-described method, and the blood purifier is charged and filled with deoxygenerated water or water saturated with an inert gas to thereby purge the blood purifier of the air present in the blood purifier, and simultaneously to fill the inside and the periphery of the hollow fiber membranes with the deoxygenerated water or the water saturated with the inert gas. After that, the blood purifier is charged and filled with an inert gas, so as to concurrently deoxygenerate the water and lower the oxygen concentration. The use of a nitrogen gas as the inert gas is preferable in view of cost-effectiveness. When a trace of oxygen is allowed to coexist so as to inhibit the blood compatibility and the sterilization effect from lowering, it is preferable to use an inert gas which is adjusted in oxygen concentration, for the displacement of the interior of the blood purifier.

In the above-described method, preferably, all the inlets and the outlets of the blood side and the dialysate side of the blood purifier are tightly sealed with caps, after the adjustment of the water content and the oxygen concentration of the inside of the blood purifier. By doing so, the evaporation of the water content from the hollow fiber membranes in the blood purifier is inhibited, and simultaneously, the infiltration of an oxygen gas in an external air, into the blood purifier is inhibited, so that the effect of the present invention can be effectively exhibited. Further, the infiltration of bacteria into the blood purifier can be inhibited. Furthermore, the evaporation of the water content from the hollow fiber membrane can be inhibited over a long period of time, and therefore, the shrinkage of the hollow fiber membrane due to the drying thereof with time and the degradation of the membrane properties can be inhibited. For this reason, there is produced the effect of inhibiting the occurrence of defects in the blood purifier and the degradation of the membrane properties, when the blood purifier is stored over a long period of time. For example, the shrinkage of the hollow fiber membranes causes peeling at the interface between the adhesive and the portion of the hollow fiber membranes fixed to the blood purifier with the adhesive, and accordingly, such a portion of the hollow fiber membranes is wetted with a liquid. In case where the hollow fiber membrane is crimped to inhibit the drift current of the dialysate, the crimps of the hollow fiber membrane are relaxed by drying the membrane, with the result that the drift current of the dialysate may be increased in amount.

In the present invention, preferably, the blood purifier tightly sealed by the above-described method is sealed in the above-described packaging bag and is then exposed to a radioactive ray. By sealing the blood purifier in the packaging bag, the adhesion of dirt and bacteria on the outer surface of the blood purifier is prevented. In this method, an inner gas of the packaging bag is not limited. While an air may be used as the inner gas, an inert gas such as a nitrogen gas is preferable, since the growth of bacteria (aerobic bacteria) included after the sterilization is inhibited, and since the above tight sealing effect is compensated. Further, in the present invention, preferably, the blood purifier tightly sealed as described above is left to stand for a certain time and is then exposed to a radioactive ray or an electron beam. Therefore, advantageously, the infiltration of an oxygen gas from an external air into the blood purifier during this standing time can be prevented.

The above-described method can be employed as a method for sterilizing the blood purifier more simply and at a lower cost, when the water content in the permselective hollow fiber membrane is 5 mass % or more. On the other hand, in case where there is used a hollow fiber membrane which is manufactured in a clean room equivalent to the standard of at least class 100,000 and which is not needed for so careful attentions to the sterilization thereof, the water content of the hollow fiber membrane is adjusted to less than 5 mass %, and the oxygen concentration and the humidity of an atmosphere around the hollow fiber membrane may be optimized, when the hollow fiber membrane is subjected to a radiation exposure treatment. Of course, no problem arises when the above-described method is applied to a hollow fiber membrane of which the water content is 5 mass % or more. The first requirement for this method relates to the oxygen concentration in the atmosphere around the hollow fiber membrane when the hollow fiber membrane is subjected to the sterilization treatment. Preferably, the radiation exposure is made on the hollow fiber membrane with the oxygen concentration in the atmosphere set at 3.6 vol. % or less, more preferably 1 vol. % or less, still more preferably 0.1 vol. % or less. When the oxygen concentration in the atmosphere exceeds 3.6 vol. %, the amount of hydrogen peroxide which is generated due to the deterioration of polyvinyl pyrrolidone increases, with the result that the above-described characteristics can not be satisfied.

The second requirement for the method relates to the amount of water adsorbed onto a portion of the hollow fiber membrane at which polyvinyl pyrrolidone is locally present. In the above-described method, it is preferable to optimize the water content in the hollow fiber membrane and the humidity in the packaging bag. The water content in the hollow fiber membrane is preferably 0.8 mass % or more. The humidity in the packaging bag is preferably above 40%RH as the relative humidity at 25°C. The relative humidity in the packaging bag is more preferably from 50 to 90%RH (at 25°C), still more preferably from 60 to 80%RH (at 25°C).

When the blood purifier sealed in the packaging bag having a relative humidity of 40%RH or lower (at 25°C) is exposed to a radioactive ray such as γ-ray, the components of the hollow fiber membrane, particularly polyvinyl pyrrolidone, are oxidized and deteriorated due to a trace of an oxygen gas even under a deoxygenerated condition, which leads to the generation of hydrogen peroxide, which further leads to the above undesirable deterioration reactions. On the other hand, when the relative humidity in the packaging bag is above 90%RH (at 25°C), dew drops occur within the packaging bag, which is likely to degrade the quality of the blood purifier.

The relative humidity referred to in the present invention can be calculated from the partial vapor pressure (p) at 25°C and the saturated vapor pressure (P) by the following equation:

$$\text{Relative humidity (\%RH)} = p/P \times 100.$$

The measurement is made by inserting the sensor of a temperature- and humidity-meter (Ondotori RH Type manufactured

by T&D) into the packaging bag and sealing the bag.

The mechanism for inhibiting the deterioration of polyvinyl pyrrolidone by setting the relative humidity in the packaging bag at above 40%RH (at 25°C) is supposed as follows. The deterioration of polyvinyl pyrrolidone is accelerated by oxygen. In the present invention, the inner atmosphere of the packaging bag is conditioned to inhibit oxidization, i.e. is kept in a substantially anoxia state. However, achieving a perfect anoxia state is difficult, and thus, a trace of an oxygen gas is present in the packaging bag. Accordingly, the deterioration reaction of polyvinyl pyrrolidone present on the surface of the hollow fiber membrane is accelerated when this polyvinyl pyrrolidone contacts the trace of oxygen gas in the packaging bag. Thus, the deterioration reaction of polyvinyl pyrrolidone starts from polyvinyl pyrrolidone present on the surface of the hollow fiber membrane. While the reason therefor is not known, it is experimentally recognized that the above-described deterioration reaction is inhibited by increasing the water content in the hollow fiber membrane. Polyvinyl pyrrolidone is locally present in the hollow fiber membrane. Therefore, the vapor in the packaging bag is selectively adsorbed onto a portion of the surface of the hollow fiber membrane at which polyvinyl pyrrolidone is locally present, when the relative humidity is increased. This adsorbed water is supposed to inhibit the deterioration reaction of polyvinyl pyrrolidone. It is accordingly supposed that a significant inhibition effect is produced by increasing the humidity. On the other hand, it is known that a hollow fiber membrane containing polyvinyl pyrrolidone has a humidity-controlling function, i.e. a humidity-adsorbing or -releasing function (cf. JP-A-2004-97918). When the relative humidity in the packaging bag is low, the water content adsorbed by polyvinyl pyrrolidone present on the surface of the hollow fiber membrane is released into the inner space of the packaging bag. Thus, the amount of the water adsorbed by polyvinyl pyrrolidone on the local portion of the surface of the membrane to be deteriorated becomes smaller. This state of the decreased amount of adsorbed water is supposed to accelerate the deterioration of polyvinyl pyrrolidone. It is supposed that, because of the synergetic effect of these phenomena, the relative humidity in the packaging bag gives a significant influence on the inhibition of the deterioration reaction of polyvinyl pyrrolidone.

As a method for satisfying the second requirement, for example, a blood purifier loaded with a polyvinyl pyrrolidone-containing polysulfone-based permselective hollow fiber membrane bundle which is adjusted in water content to from 0.8 to less than 5 mass % is sealed together with an oxygen scavenger in a packaging bag having an oxygen permeability of 10 $cm^3/m^2$.24hr.MPa (at 20°C and 90%RH) or less and an aqueous vapor permeability of 50 $g/m^2$.24hr.MPa (at 40°C and 90%RH) or less; and the blood purifier in the packaging bag is then exposed to a radioactive ray while the relative humidity in the inner atmosphere of the packaging bag is being kept at 40%RH at 25°C.

The oxygen scavenger for use in the above-described method is to absorb the oxygen in the packaging bag and thereby to achieve a substantial deoxygenerated state. Accordingly, there is no limit in selection of the oxygen scavenger, so long as it has a deoxygenerating function. For example, the following are preferably used.

There is no limit in selection of the oxygen scavenger, so long as it has a deoxygenerating function. For example, there are given oxygen scavengers which comprise, as main oxygen-absorbing agents, sulfite, hydrogensulfite, dithionite, hydroquinone, catechol, resorcinol, pyrogallol, gallic acid, rongalite, ascorbic acid and/or a salt thereof, sorbose, glucose, lignin, dibutylhydroxytoluene, dibutylhydroxyanisole, metal powder (e.g. ferrous salt and iron powder, etc.) and the like. The oxygen scavenger may be appropriately selected from these materials for use. An oxygen scavenger mainly comprising metal powder, if needed, may contain, as an oxidation catalyst, one or more compounds selected from halogenated metal compounds such as sodium chloride, potassium chloride, magnesium chloride, calcium chloride, aluminum chloride, ferrous chloride, ferric chloride, sodium bromide, potassium bromide, magnesium bromide, calcium bromide, iron bromide, nickel bromide, sodium iodide, potassium iodide, magnesium iodide, calcium iodide, iron iodide, etc. Further, other functional fillers such as a deodorant may be included in the packaging bag. The form of the oxygen scavenger is not limited, and it may be in the form of powder, particles, mass or sheet; or it may be a sheet- or film-shaped oxygen scavenger obtained by dispersing an oxygen absorber composition in a thermoplastic resin.

The packaging bag to be used in the present invention is to form a space deoxygenerated with the above oxygen scavenger, and is also required to have a function to maintain the deoxygenerated state over a long period of time. Therefore, the packaging bag is needed to be made of a material having a low oxygen permeability. The oxygen permeability of the material is preferably 10 $cm^3/m^2$.24hr.MPa or less (20°C, 90%RH), more preferably 8 $cm^3/m^2$.24hr.MPa or less (20°C, 90%RH), still more preferably 6 $cm^3/m^2$.24hr.MPa or less (20°C, 90%RH), far still more preferably 4 $cm^3/m^2$.24hr.MPa or less (20°C, 90%RH).

When the oxygen permeability exceeds 10 $cm^3/m^2$.24hr.MPa (20°C, 90%RH), an oxygen gas passes through the packaging bag from an external, even when the packaging bag is tightly sealed. As a result, the oxygen concentration in the packaging bag increases, which, undesirably, makes it impossible to maintain the substantial deoxygenerated condition.

As described above, in the present invention, it is necessary that the hollow fiber membrane packed in the blood purifier maintains a specified water content. Accordingly, the packaging bag to be used in the present invention is made of a material having a low water vapor permeability. The water vapor permeability of the packaging bag is preferably 50 $g/m^2$.24hr.MPa or less (40°C, 90%RH), more preferably 40 $g/m^2$.24hr.MPa or less (40°C, 90%RH), still more preferably 30 $g/m^2$.24hr.MPa or less (40°C, 90%RH), far still more preferably 20 $g/m^2$.24hr.MPa or less (40°C,

90%RH). When the water vapor permeability of the packaging bag exceeds 50 $g/m^2$.24hr.MPa (40°C, 90%RH), water vapor passes through the packaging bag, even when the packaging bag is tightly sealed. Therefore, the drying of the hollow fiber membrane proceeds, which may make it impossible to maintain a desirable water content as described above.

The materials and structure of the packaging bag to be used in the present invention may be optionally selected, so long as the above properties are satisfied. Preferable examples of the material for the packaging bag are oxygen- and water vapor-impermeable materials such as an aluminum foil, aluminum-deposited film, inorganic oxide-deposited film of silica and/or alumina, vinylidene chloride polymer composite film and the like. The sealing method for the packaging bag also may be optionally selected. For example, the packaging bag may be sealed by any of the heat sealing method, impulse sealing method, fusion sealing method, frame sealing method, ultrasonic sealing method, high frequency sealing method and the like. Thus, the material for the packaging bag is preferably a composite material of a film having a sealing property and any of the above impermeable materials. Particularly preferable is a laminate sheet comprising a structural layer of an aluminum foil capable of substantially shutting out an oxygen gas and a water vapor, an outer layer of a polyester film, an intermediate layer of an aluminum foil, and an inner layer of a polyethylene film, since this laminate sheet has both of impermeability and a heat sealing property.

There is no limit in selection of the method for adjusting the humidity in the packaging bag within the above-specified range. For example, the following methods can be employed:

(1) The packaging bag is charged with a gas controlled in humidity, before the blood purifier is sealed in the packaging bag; or the blood purifier is sealed in the packaging bag under a humidity-controlled atmosphere.
(2) The humidity in the packaging bag is controlled by the water content of the permselective hollow fiber membrane.
(3) An oxygen scavenger capable of releasing the water content is used.
(4) The blood purifier is sealed together with an oxygen scavenger and a humidity-conditioning agent in the packaging bag.

There is no limit in selection of the humidity-conditioning agent, so long as it has a humidity-absorbing and -releasing function to control the relative humidity in the packaging bag within the above-specified range. Although not limited to, B type silica gel is widely used as the humidity-conditioning agent. As a humidity-conditioning agent analogous to the B type silica gel, there are given porous inorganic particles such as improved B type silica gel which is improved in humidity-absorbing and -releasing function by sharpening the pore distribution of silica gel, or by compounding a humidity-conditioning aid which comprises an alkaline metal compound or an alkaline earth metal compound, mesoporous silica alumina gel, mesoporous hollow fiber-like aluminum silicate, zeolite, etc. Further, the humidity-conditioning agent may be particles of a water absorbable polymer which is obtained by copolymerizing, blending or alloying sodium acrylate crosslinked polymer, a polyethylene glycol chain and a polyvinyl pyrrolidone chain. The shape of the humidity-conditioning agent is not limited: for example, it may be in the form of powder, particles, mass, sheet or the like. Preferably, the powdery or particular humidity-conditioning agent is wrapped in a humidity permeable packaging material for use. Otherwise, the humidity-conditioning agent may be used as a composite with a film, sheet, paper, non-woven cloth, woven cloth or the like. In this case, the base material of the composite is preferably a hydrophilic material. Otherwise, humidity-conditioning particles may be compounded with a hydrophilic binder, and is then compounded with a base material made of a general-purpose material such as polyester, polyolefin or the like. In case of a humidity-conditioning agent comprising a water absorbable polymer, the polymer may be directly formed into a film or a sheet, or made into a fiber, which is then used in the shape of paper, non-woven cloth, woven cloth or the like. Otherwise, a foaming agent is used with the polymer to form a foamed sheet or a foam. For example, a humidity-conditioning sheet obtained by impregnating a water absorbing sheet (paper, non-woven cloth or woven cloth) with an inorganic salt humidity-conditioning agent such as ammonium chloride, a sheet-shaped water-containing gel obtained by fixing water and a surfactant with a mesh-structured water absorbable polymer which is obtained by crosslinking sodium polyacrylate with an inorganic crosslinking agent such as magnesium aluminate metasilicate.

Preferably, the above-described humidity-conditioning agent is previously seasoned under an atmosphere of a temperature of 25°C and a relative humidity of 80 to 90%RH before use.

It is needed to maintain, under a substantially deoxygenerated condition, the ambient atmosphere around the hollow fiber membranes packed in the blood purifier, in order to carry out the above-described method. Accordingly, the opening portions of the blood purifier are needed to be kept open.

The above-described method using deaerated water is called the deaerated water method, and the method using the oxygen scavenger is called the oxygen scavenger method.

In the present invention, radiation exposure is carried out after preferably at least 48 hours, more preferably at least 72 hours, has passed since the sealing. When this period from the sealing to the radiation exposure is too long, bacteria is likely to proliferate. Therefore, the radiation exposure should be done within preferably 10 days, more preferably 7 days, still more preferably 5 days, after the sealing. There is no limit in selection of the temperature during this period from the sealing to the radiation exposure. For example, it may be a room temperature. When the radiation exposure is

done within shorter than 48 hours after the sealing, the water permeability-exhibiting rate of the membrane bundle after the priming treatment may become poor. It is difficult to specify the factors for the performance-exhibiting rate of the membrane bundle after the priming treatment, since lots of technical factors are involved because of the state of the hollow fiber membrane bundle which is obtained by cutting a continuous hollow filament into pieces of predetermined lengths. However, the significant influence of the contents of polyvinyl pyrrolidone in the uppermost layers of the inner surface and the outer surface of the permselective hollow fiber membrane, the condition of 10 ppm or less in the amount of polyvinyl pyrrolidone eluted from the hollow fiber membrane bundle, and the influences of the water content adjusted with deaerated water and the concentration of dissolved oxygen in the deaerated water are not ignorable.

However, the following are predicted when the performance-exhibiting rates of the membrane bundles after the priming treatments are examined in relation to the periods of time until the sterilization treatments, in view of the rule of thumb and from the technical view points in the manufacturing sites. As is understood from the graph shown in Fig. 6, the performance-exhibiting rates of the membrane bundles after the priming treatments are variable, when the periods of time until the sterilization treatments are as relatively short as 10 hours, 20 hours, 30 hours and so on. Therefore, there is high possibility that uniform and stable products can not be obtained. On the other hand, when exposure treatments are conducted on the membrane bundles after about 48 hours, for example, 60 hours, 120 hours or so, has passed since the sealing, the performance-exhibiting rates of the membrane bundles after the priming treatments are converged in a region where a difference range in variation of the performance-exhibiting rates is small. Under these conditions, it is no doubt that such membrane bundles, when used in a clinical site, will show good rise in exhibition of their performance and will be very advantageous in handling thereof to shorten the dialyzing time.

The relationship between the behaviors of the hollow fiber membrane bundles and the eluting amounts of hydrogen peroxide from the membrane bundles is examined on condition that the performance-exhibiting rates thereof after the priming treatments are set at 90%. When the membrane bundles which show, for example, 5 ppm or less in the hydrogen peroxide eluting amounts, are sterilized within about 48 hours or shorter after the sealing, the performance-exhibiting rates of such membrane bundles show less variations around 90%. On the other hand, when the hydrogen peroxide-eluting amounts of hollow fiber membrane bundles are, for example, 10 ppm or more, such membrane bundles tend to show more variations in a region of 48 hours shorter, as shown in Fig. 6. On the other hand, when the periods of time until the sterilization treatments are set at 48 hours or longer, the performance-exhibiting rates of the hollow fiber membrane bundles after the priming treatments are converged in an ideal region of 90% or more, although the hydrogen peroxide-eluting amounts of the hollow fiber membrane bundles give some influence. It is supposed that such behaviors will be likewise observed in the analysis of a permselective hollow fiber membrane in which the content of polyvinyl pyrrolidone in the uppermost layer of the outer surface thereof is from 25 to 50 mass %.

While there is some difference in behavior, it is predicted that a similar tendency to the tendency shown in Fig. 6 will be observed in the analyses of the critical behavior of a blood purifier comprising a polysulfone-based permselective hollow fiber membrane bundle which has a water content of 600 mass % or less, adjusted by the use of deaerated water or water saturated with an inert gas, of which the concentration of dissolved oxygen is from 0.001 to 0.5 ppm, on condition that the performance-exhibiting rate after a priming treatment is set at 90%, and the period of time until a sterilization treatment, at 48 hours. When the behavior of a hollow fiber membrane bundle is analyzed from these technical view points, the performance-exhibiting rate of the membrane bundle after a priming treatment and the period of time until a sterilization treatment are found to be technical factors for significantly improving the availability of the hollow fiber membrane bundle, the management of the quality thereof and the advantages thereof in a clinical site. This period of time is not necessarily fixed to 48 hours, and should be optionally selected in consideration of the factors such as the quality, productivity, materials, structure of an intended blood purifier.

While it can not be determined by the univocal reason when the complicated materials and structure of the hollow fiber membrane bundle are taken into consideration, the following can be supposed. When the inside of a blood purifier is so conditioned that the oxygen concentration therein is adjusted low by controlling the amount of dissolved oxygen in and around a polysulfone-based permselective hollow fiber membrane to from 0.001 to 5 ppm, the concentration of dissolved oxygen and the water content in the hollow fiber membrane bundle become uniform with time because of the diffusion, infiltration and transference of the dissolved oxygen and the water, so that the quality of the material relative to water, dissolved oxygen and a hydrophilic polymer becomes equilibrium. After the hollow fiber membrane bundle has been put in such an eqiulibrium state, the blood purifier comprising such a hollow fiber membrane bundle is sterilized. By doing so, the blood purifier comprising the uniform and high quality hollow fiber membrane bundle is provided. This can be regarded as a kind of material fixing of the hollow fiber membrane bundle made of complicated materials and having a complicated structure. Such a tendency already has been fully described in this specification.

Although the reason why the water permeability-exhibiting rate of the hollow fiber membrane bundle after the priming treatment varies depending on the period of time until the exposure treatment is not known, the following can be supposed: that is, a trace of oxygen adsorbed on the surface of the hollow fiber membrane is transferred to deaerated.water which is locally present around the oxygen, to thereby inhibit the deterioration reaction which is induced by the radiation exposure and which impairs the affinity between the surface of the membrane and water, with the result

that the change of the water permeability-exhibiting rate after the priming treatment is induced by such inhibition.

As the radioactive ray to be used in the present invention, α-ray, β-ray, γ-ray, neutron ray, X-ray, electron beam, UV, or ion beam is used. Among those, γ-ray or an electron beam is preferably used because of its high sterilization efficiency and handling ease. The exposure dose of a radioactive ray is not limited, in so far as sterilization and crosslinking are possible. Generally, 10 to 30 kGy is preferable.

The above-described oxygen scavenger method and the above-described deaerated water method have the following features, respectively.

The oxygen scavenger method can be applied to a blood purifier packed with a permselective hollow fiber membrane having a water content of as low as less than 5 mass %, and this method is suitable to provide a light weight blood purifier. However, an oxygen scavenger is needed, and therefore, the use of a high oxygen- and water vapor-barrier material for a packaging bag is needed, which is disadvantageous in view of cost. On the other hand, the deaerated water method does not need the use of an oxygen scavenger, and the use of a general-purpose material for a packaging bag is possible, which is advantageous in view of cost. However, it is necessary that the water content in the hollow fiber membrane should be 5 mass % or more, which is disadvantageous in view of a decrease in the weight of the blood purifier. Both the methods have opposite features to each other, and thus, a suitable method is appropriately selected from these methods in accordance with a demand from the market. For example, the oxygen scavenger method is preferred for a blood purifier for use in an extremely cold area.

In the present invention, the water permeability of the blood purifier found after 10 minutes has passed after the priming treatment is preferably 90% or more, more preferably 92% or more, still more preferably 94% or more, of the water permeability thereof found after 24 hours has passed after the priming treatment. When the blood purifier shows such a water permeability-exhibiting rate, it is found that the hydrophilicity necessary for the exhibition of the membrane performance is sufficiently obtained by the priming treatment, and also, the reliability of the blood purifier is improved. When the ratio of both the water permeabilities is less than 90%, the hydrophilicity of the membrane imparted by the priming treatment is insufficient, and thus, the intrinsic membrane performance can not be exhibited, and also, the membrane has a hydrophobic portion poor in water compatibility. Thus, there is a danger of clogging the membrane due to the adsorption of protein onto such a hydrophobic portion of the membrane during the perfusion of the blood. When the ratio of both the water permeabilities is 90% or more, it indicates that the condition for exhibiting a substantially necessary membrane performance is proceeding, and that this condition is suitable and proves that the membrane quickly becomes compatible with water.

Prior to use, the blood purifier is filled and washed with physiologic saline and purged of bubbles, namely, the blood purifier is subjected to a so-called priming treatment. In some cases, the polysulfone-based permselective hollow fiber membrane is not sufficiently compatible with water, and thus, a long time is required for the priming treatment, and a long time is required for the membrane to exhibit sufficient water compatibility. Especially in case of a dry type blood purifier as in the present invention, a time which is required for the performance such as water permeability of the hollow fiber membrane to reach a predetermined level in the priming treatment sometimes varies. Therefore, the development of a hollow fiber membrane bundle capable of exhibiting a given level of membrane performance in a shorter time is demanded, and the present invention is intended to meet such a demand.

The blood purifier of the present invention preferably has the following feature. The hollow fiber membrane is removed from the blood purifier which has been stored at a room temperature for one year or longer after exposed to a radioactive ray, and is then equally divided into 10 portions, and each of 10 portions is subjected to the test regulated in the Approval Standard for Dialysis-Type Artificial Kidney Apparatus. The maximum UV absorbances (at 220 to 350 nm) of extracts from all the 10 portions of the membrane should be preferably 0.10 or less. More preferably, this feature can be maintained for 2 years or longer. Particularly, this feature can be maintained for at least 3 years, since 3 years is set as the guarantee periods of blood purifiers. It is experimentally confirmed that a blood purifier which can be maintained at 0.06 or less in the maximum UV absorbances (at 220 to 350 nm) at all the sites thereof after the passage of 1 year can maintain this feature for 3 years.

When the hollow fiber membrane bundle of the present invention is used in a blood purifier, the burst pressure of the hollow fiber membrane bundle is preferably not lower than 0.5 MPa, and the water permeability of the blood purifier is preferably not lower than 150 ml/m$^2$/hr./mmHg. When the burst pressure is lower than 0.5 MPa, latent defects which may induce blood leakage can not be detected, as will be described later. When the water permeability is lower than 150 ml/m$^2$/hr./mmHg, the dialyzing efficiency of the blood purifier tends to lower. It is effective to increase the diameters of the pores of the membrane and the number of the pores in order to improve the dialyzing efficiency. However, there arise disadvantages such as a decrease in the strength of the membrane and occurrence of defects. Therefore, in the hollow fiber membrane of the present invention, the diameters of the pores of the outer surface of the membrane are optimized to thereby optimize the porosity of the support layer so that the resistance of the permeated solute can balance with the strength of the membrane. The water permeability is more preferably not lower than 200 ml/m$^2$/hr./mmHg, still more preferably not lower than 250 ml/m$^2$/hr./mmHg, far still more preferably not lower than 300 ml/m$^2$/hr./mmHg. When the water permeability is too high, the water-removing control during hemodialysis becomes hard. Therefore, the water

permeability is preferably not higher than 2,000 ml/m$^2$/hr./mmHg, more preferably not higher than 1,800 ml/m$^2$/hr./mmHg, still more preferably not higher than 1,500 ml/m$^2$/hr./mmHg, far still more preferably not higher than 1,300 ml/m$^2$/hr./nmHg.

Generally, blood purifiers for use in blood purification are subjected to leak tests by compressing the interiors or exteriors of the hollow fiber membranes with an air, so as to check the defects of the hollow fiber membranes and the blood purifiers in the final stage for providing products. When some leakage is detected in a blood purifier by means of a compressed air, such a blood purifier is scrapped as a defective, or is repaired. The air pressure for use in the leak tests, in many cases, is several times higher than the proof pressure (usually 500 mmHg (0.067 MPa)) for hemodialyzers. However, in case of hollow fiber type blood-purifying membranes having particularly high water permeability, minute flaws, crushes and tears of the hollow fiber membranes, which can not be detected by the ordinary leak tests, often lead to the cutting and pin holes of the hollow fiber membranes which would occur in the course of the manufacturing steps subsequent to the leak tests (mainly, the steps of sterilization and packing) and at the step of the transportation thereof or the handling thereof at clinical sites (unpacking or priming). Such defects of the hollow fiber membranes induce further troubles such as the leakage of blood during treatments, etc. Therefore, such defects should be eliminated. These troubles can be avoided by adjusting the burst pressure to the above-specified range.

It is also effective to adjust the eccentricity of the hollow fiber membrane bundle, in order to inhibit the occurrence of the above-mentioned latent defects.

The burst pressure referred to in the present invention is an index of the pressure resistant performance of a hollow fiber membrane bundle inserted into a blood purifier. The burst pressure is measured by compressing the interior of the hollow fiber membrane with a gas, and gradually increasing the pressure so as to find a pressure which bursts the hollow fiber membrane when the hollow fiber membrane can not withstand the internal pressure. A higher and higher burst pressure leads to a lower possibility of occurrence of cutting or pin holes of hollow fiber membranes in use. Therefore, the burst pressure is preferably not lower than 0.5 MPa, more preferably not lower than 0.55 MPa, still more preferably not lower than 0.6 MPa. When the burst pressure is lower than 0.5 MPa, the hollow fiber membrane is likely to have latent defects. A higher and higher burst pressure is preferable. In order to mainly increase the burst pressure, the thickness of a hollow fiber membrane is increased, or the porosity of a membrane is excessively decreased. However, in this case, desired membrane performance can not be obtained from the resultant membrane. Therefore, the burst pressure is preferably lower than 2.0 MPa, more preferably lower than 1.7 MPa, still more preferably lower than 1.5 MPa, far still more preferably lower than 1.3 MPa, and particularly lower than 1.0, when a hollow fiber membrane is used as a hemodialysis membrane.

The eccentricity, i.e., non-uniformity in the thickness of 100 hollow fiber membranes in a blood purifier, found when the sections of the membranes are observed, is represented by a ratio of a maximum value and a minimum value of the eccentricity. Preferably, the minimum eccentricity out of those of 100 hollow fiber membranes is not smaller than 0.6. When even one hollow fiber membrane, out of the 100 hollow fiber membranes, has a eccentricity of smaller than 0.6, such a hollow fiber membrane has possibility to cause a leakage when used in a clinical site. Therefore, the eccentricity is not represented by an average value, but is represented by a minimum value among the eccentricity of 100 hollow fiber membranes. The higher the eccentricity, the better it is, because the uniformity of the membranes can be improved, because the emergence of latent defects can be prevented, and because the burst pressure can be increased. Therefore, the eccentricity of membranes is more preferably not smaller than 0.7, still more preferably not smaller than 0.8, far still more preferably not smaller than 0.85. When the eccentricity is too small, the latent defects of the membrane tend to emerge, and the burst pressure of the membrane tends to lower, which may lead to blood leakage.

To achieve a eccentricity of not smaller than 0.6, it is preferable to strictly uniform the width of the slit of a spinning nozzle, namely, the discharge outlet for a membrane-forming solution. Generally used as a spinning nozzle for a hollow fiber membrane is a tube-in-orifice type nozzle which comprises an annular slit for discharging a membrane-forming solution, and a hole, inside the annular slit, for discharging a core solution as inner coagulation liquid. The slit width indicates the width of the outer annular slit for discharging the membrane-forming solution. By decreasing the variation in the slit width, the eccentricity of a spun hollow fiber membrane can be reduced. Specifically, the ratio of the maximum value to the minimum value of the slit width is adjusted within a range of from 1.00 to 1.11. The difference between the maximum value and the minimum value is preferably not larger than 10 μm, more preferably not larger than 7 μm, still more preferably not larger than 5 μm, particularly not larger than 3 μm. It is also effective to optimize the temperature of the nozzle, and the nozzle temperature is preferably from 20 to 100°C. When the nozzle temperature is lower than 20°C, the nozzle temperature can not be stabilized because of the influence of a room temperature, and discharge spots of the membrane-forming solution is likely to form. Therefore, the nozzle temperature is more preferably not lower than 30°C, still more preferably not lower than 35°C, far still more preferably not lower than 40°C. When the nozzle temperature exceeds 100°, the viscosity of the membrane-forming solution excessively lowers, and the discharge of the membrane-forming solution becomes instable, or the thermal deterioration or decomposition of polyvinyl pyrrolidone is likely to proceed. Therefore, the nozzle temperature is more preferably not higher than 90°C, still more preferably not higher than 80°C, far still more preferably not higher than 70C.

As a method of raising the burst pressure, it is also effective to decrease the flaws of the surfaces of the hollow

fiber membrane and to decrease the amounts of foreign matters and bubbles therein, to thereby decrease the latent defects thereof. As a method of decreasing the flaws of the surface of the hollow fiber membrane, it is effective to optimize materials for rollers and guides for use in the steps of manufacturing a hollow fiber membrane, and to optimize the surface roughness of such rollers and guides. It is also effective that, when a blood purifier is fabricated using a bundle of hollow fiber membranes or when.a bundle of hollow fiber membranes is inserted into a casing for the blood purifier, a device to allow the hollow fiber membrane bundle not to contact the casing or a device to make it hard to rub the hollow fiber membranes with one another is provided. In the present invention, preferably, the rollers to be used are planished at their surfaces, so as to prevent the hollow fiber membranes from having flaws thereon upon slipping. Preferably, the guides to be used are subjected to mat finishing or knurling process at their surfaces, so as avoid the contact resistance with the hollow fiber membranes as much as possible. Preferably, the bundle of hollow fiber membranes is not directly inserted into the blood purifier casing. For example, the bundle of hollow fiber membranes is wrapped in an embossed film and then is inserted into the casing, followed by removal of the film alone from the casing.

As a method of preventing foreign matters from entering the hollow fiber membranes, it is effective to use raw materials containing less foreign matters, or to filter a membrane-forming solution to thereby reduce the amount of foreign matters. In the present invention, it is preferable to use a filter having pores with diameters smaller than the thickness of the hollow fiber membrane bundle and to filter the membrane-forming solution therethrough before discharging the membrane-forming solution. This is described in more detail. A membrane-forming solution homogeneously dissolved is allowed to pass through a sintered filter having pores with diameters of 10 to 50 μm, provided between a dissolution tank and a nozzle. It is sufficient to carry out the filtration at least once. The filtration treatment is carried out in several stages, to improve the filtration efficiency and to prolong the lifetime of the filter. The pore diameter of the filter is more preferably 10 to 45 μm, still more preferably 10 to 40 μm. When the pore diameter is too small, the back pressure tends to increase, and the quantitative determination tends to lower. As a method of preventing bubbles from entering the hollow fiber membranes, it is effective to defoam the polymer solution for the membranes. Stationary defoaming or decompression defoaming may be carried out, depending on the viscosity of the membrane-forming solution. That is, after the inner atmosphere of the dissolution tank is decompressed to -100 to -750 mmHg, the same tank is sealed and is left to stand for 5 to 30 minutes. This operation is repeated several times to defoam the membrane-forming solution. When the decompression degree is too low, a long time is needed for the deaerating treatment, since the number of the defoaming steps must be increased. When the decompression degree is too high, the cost for increasing the closeness of the system becomes higher. The total treating time is preferably 5 minutes to 5 hours. When the treating time is too long, polyvinyl pyrrolidone is likely to be decomposed and deteriorated due to the influence of the decompression. When the treating time is too short, the effect of defoaming is insufficient.

EXAMPLES

Hereinafter, the effectiveness of the present invention will be described by way of Examples thereof, which should not be construed as limiting the scope of the present invention in any way. In this regard, the methods of evaluating the physical properties in the following Examples are described below.

1. Water Permeability

A circuit on the side of the blood outlet of a dialyzer (nearer to the outlet than a pressure-measuring point) is pinched with forceps for whole filtration. Pure water maintained at 37°C is poured into a compression tank, and is fed to the dialyzer maintained at a constant temperature in a thermostat of 37°C, while the pressure being controlled with a regulator. The amount of a filtrate flowing from the side of a dialysate is measured with a graduated cylinder. The transmembrane pressure (TMP) is defined by the equation:

$$TMP = (Pi + Po)/2$$

In this equation, Pi represents a pressure on the side of the inlet of the dialyzer, and Po represents a pressure on the side of the outlet of the same. TMP is changed at 4 points to measure the filtered flow amounts. The water permeability (mL/hr./mmHg) of the hollow fiber membrane is calculated from the slope of the relationship thereof. It is to be noted that the correlation function between TMP and the filtered flow amount should be not smaller than 0.999. The TMP is measured under a pressure of not higher than 100 mmHg so as to lessen an error in pressure loss due to the circuit. The water permeability of the hollow fiber membrane bundle is calculated from the membrane area and the water permeability of the dialyzer:

$$\mathrm{UFR(H)} = \mathrm{UFR(D)}/\mathrm{A}$$

In this equation, UFR(H) represents the water permeability (mL/$m^2$/hr./mmHg) of the hollow fiber membrane bundle; UFR(D) represents the water permeability (mL/hr./mmHg) of the dialyzer; and A represents the membrane area ($m^2$) of the dialyzer.

2. Calculation of Membrane Area

The membrane area of the dialyzer is calculated based on the inner diameter of the hollow fiber membrane:

$$A = n \times \pi \times d \times L$$

In this equation, n represents the number of the hollow fiber membranes in the dialyzer; π represents the ratio of the circumference of a circle to its diameter; d represents the inner diameter (m) of the hollow fiber membrane; and L represents the effective length (m) of the hollow fiber membranes in the dialyzer.

3. Burst Pressure

The dialysate side of a blood purifier comprising a bundle of about 10,000 hollow fiber membranes is filled with water and is capped. A dry air or a nitrogen gas is fed from the blood side of the blood purifier at a room temperature so as to compress the hollow fiber membranes at a rate of 0.5 MPa/minute. The internal pressure in the hollow fiber membrane is increased to find an air pressure which bursts the hollow fiber membrane and causes bubbles in the liquid filling the dialysate side of the blood purifier. This air pressure is defined as a burst pressure.

4. Deviation in Thickness

The sections of 100 hollow fiber membranes are observed with a projector of a magnification of 200. One hollow fiber membrane which has the largest difference in the thickness at its section is selected out of the hollow fiber membranes in one visual field, and the thickest portion and the thinnest portion of the section of this hollow fiber membrane are measured:

$$\text{Deviation in thickness} = \text{the thinnest portion}/\text{the thickest portion.}$$

The membrane thickness is perfectly uniform when the deviation in thickness is one (eccentricity = 1)..

5. Eluting Amount of Polyvinyl pyrrolidone

An eluate is obtained according to the method regulated in the Approval Standard for Dialysis-type Artificial Kidney Apparatus, and polyvinyl pyrrolidone in the eluate is determined by the colorimetric method.

In case of a dry hollow fiber membrane blood purifier, pure water (100 ml) is added to the hollow fiber membrane bundle (1 g), and elution is made from the wet hollow fiber membrane bundle at 70°C for one hour. The obtained eluate (2.5 ml) is sufficiently mixed with a 0.2 mol aqueous citric acid solution (1.25 ml) and a 0.006N aqueous iodine solution (0.5 ml), and the mixture is left to stand at a room temperature for 10 minutes. After that, the absorbance of the mixture at 470 nm is measured. Determination is made based on an analytical curve found by the measurement according to the above method, using polyvinyl pyrrolidone as a sample.

In case of a wet hollow fiber membrane blood purifier, physiological saline is allowed to pass through the passages on the dialysate side of the blood purifier at a rate of 500 mL/minute for 5 minutes, and then is allowed to pass through the passages on the blood side of the blood purifier at a rate of 200 mL/minute. After that, the physiological saline is allowed to pass from the blood side to the dialysate side at a rate of 200 mL/minute for 3 minutes, while being filtered. After that, the membranes are freeze-dried. The dried membranes are used for the above-described determination.

6. UV Absorbance (220 to 350 nm)

The absorbance of an eluate obtained by the method described in the Approval Standard for Dialysis-Type Artificial Kidney Apparatus is measured at a wavelength of from 200 to 350 nm, using a spectrophotometer (U-3000, manufactured by Hitachi, Ltd.) to find the maximal absorbance within this wavelength range.
The hollow fiber membrane bundle is equally divided into 10 portions in the lengthwise direction. One gram of the hollow fiber membrane bundle in a dried state is weighed from each of these portions for use as a sample. Then, the absorbances of all the samples are measured.
In case of a wet hollow fiber membrane blood purifier, it is.treated in the same manner as described in the part of "Eluting Amount of Polyvinyl pyrrolidone" to obtain a dried hollow fiber membrane, which is then used for measurement.

7. Determination of Hydrogen Peroxide

To an eluate (2.6 ml) obtained by the method described in the Approval Standard for Dialysis-Type Artificial Kidney Apparatus are added an ammonium chloride buffer (pH 8.6) (0.2 ml) and a solution mixture of a hydrogen chloride solution of $TiCl_4$ and an aqueous solution of a 4-(2-pyridilazo)resorcinol monosodium salt equivalent in molar ratio. Further, a 0.4 mM coloring reagent (0.2 ml) is added, and the resulting mixture is heated at 50°C for 5 minutes, and then is cooled to room temperature. The absorbance of the resultant solution is measured at a wavelength of 508 nm. The same measurement is made on a sample to find an analytical curve. The amount of hydrogen peroxide is determined based on this analytical curve.
In measurement, a hollow fiber membrane bundle is equally divided into 10 portions in the lengthwise direction, and 1 g of the hollow fiber membrane is weighed from each of the 10 portions as a sample. Then, all the samples are measured.
In case of a wet hollow fiber membrane blood purifier, it is treated in the same manner as described in the part of "Eluting Amount of Polyvinyl pyrrolidone" to obtain a dried hollow fiber membrane, which is then used for measurement. When the amount of hydrogen peroxide is determined from a wet hollow fiber membrane bundle, a hollow fiber membrane dried by the freeze-dry method is used.

8. Blood Leak Test

Bovine blood which is admixed with citric acid to be inhibited from coagulation and which is maintained at 37°C is fed to a blood purifier at a rate of 200 ml/minute, and is filtered at a rate of 10 ml/minute. The resulting filtrate is returned to the blood to thereby form a circulation system. After 60 minutes has passed, the filtrate of the blood purifier is collected, and the red portion of the filtrate, due to the leakage of red blood cells, is visually observed. This blood leak test is conducted for each of 30 blood purifiers in either of Examples and Comparative Examples, and the number of blood purifiers which permit the blood to leak therefrom is counted.

9. Water Content in Hollow Fiber Membrane

The water content in a hollow fiber membrane is determined by measuring the mass (g) of the hollow fiber membrane before drying the same, vacuum-drying the hollow fiber membrane under reduced pressure (-750 mmHg or lower) for 12 hours, measuring the mass (g) of the dried hollow fiber membrane, and finding the percentage (%) of a difference in mass as a decrease (g) between the masses found before and after the drying, based on the mass (g) of the dried hollow fiber membrane. The water content is determined by the following equation:

$$(\text{Decrease}/\text{the mass of the dried hollow fiber membrane}) \times 100 = \text{water content (mass \%)}.$$

In this regard, by setting the mass of the hollow fiber membrane within a range of 1 to 2 g, the hollow fiber membrane can be bone-dried in 2 hours (showing no further change in mass).

10. Polyvinyl Pyrrolidone Contents in Uppermost Layers of Inner and Outer Surfaces of Hollow Fiber Membrane

The content of polyvinyl pyrrolidone is determined by the X-ray electron spectroscopy for chemical analysis (the ESCA method). One hollow fiber membrane is obliquely cut so as to expose a part of the inner surface thereof, and is stuck on a sample table to measure the inner and outer surfaces thereof by the ESCA method. The measuring conditions are described below:

Apparatus: ULVAC-PHI ESCA 5800
Exciting X-ray: MgKα-ray
Output of X-ray: 14 kV, 25 mA
Escape angle of photoelectron: 45°
Analytical diameter: 400 umϕ
Path energy: 29.35 eV
Resolution: 0.125 eV/step
Degree of vacuum: about $10^{-7}$ Pa or lower

The content of polyvinyl pyrrolidone in the surface of the membrane is calculated from the found value of nitrogen (N) and the found value of sulfur (S) by the following equation:

<Membrane of Polyvinyl Pyrrolildone-admixed PES (Polyether Sulfone)>

Content of polyvinyl pyrrolidone (H polyvinyl pyrrolidone) [mass %] =

$$100 \times (N \times 111)/(N \times 111 + S \times 232)$$

<Membrane of Polyvinyl Pyrrolildone-admixed PSf (Polysulfone)>

Content of polyvinyl pyrrolidone (H polyvinyl pyrrolidone) [mass %] =

$$100 \times (N \times 111)/(N \times 111 + S \times 442)$$

11- Polyvinyl Pyrrolidone Content in Whole of Hollow Fiber Membrane

A hollow fiber membrane is dried at 80°C for 48 hours with a vacuum drier, and 10 mg of the dried membrane is analyzed with a CHN coder (MT-6 Model manufactured by Yanaco), and the content of polyvinyl pyrrolidone is calculated from the content of nitrogen by the following equation:

Polyvinyl pyrrolidone content (mass %) = nitrogen content (mass %) X 111/14

12. Inner Diameter and Thickness of Hollow Fiber Membrane

A hollow fiber membrane is cut in a direction perpendicular to the lengthwise direction with a sharp-edged cutter, and the section of the membrane is observed with a microscope with a magnification of 200. The values of the inner diameters and the outer diameters of five hollow fiber membranes (n = 5) are measured and averaged.

Thickness of membrane [μm] = {(outer diameter) - (inner diameter)}/2.

13. Specific Resistance

The specific resistance of water is calculated from an electric conductivity measured with an electric conductivity meter (CM-40V manufactured by TOA).

14. Storage Stability of Hollow Fiber Membrane Bundle

Each of hollow fiber membrane bundles in dried states, obtained in each of Examples and Comparative Examples, is stored in a dry box (the atmosphere: an air) controlled in humidity to 50%RH for 3 months, and then, the UV absorbance (at 220 to 350 nm) thereof is measured according to the method regulated in the Approval Standard for Dialysis-Type Artificial Kidney Apparatus. The stability of the hollow fiber membrane bundle is evaluated based on the degree of increase in the UV absorbance (at 220 to 350 nm) because of the storage. This degree of increase is measured as follows: the hollow fiber membrane bundle is equally divided into 10 portions as samples in the lengthwise direction, and the samples are measured in the degree of increase in the UV absorbance, and evaluation is made based on the maximum value among these UV absorbances. A hollow fiber membrane of which the maximum value does not exceed 0.10 is passed.

15. Measurement of Oxygen Concentrations in Packaging Bag and Water

The oxygen concentration in a packaging bag is measured by gas chromatography, using a column filled with a molecular sieve (Molecular Sieve 13X-S Mesh 60/80 manufactured by GL Science), an argon gas as a carrier gas, and a thermal conduction type detector, with the column temperature set at 60°C. A gas in the packaging bag is collected by directly pricking the unopened packaging bag with the needle of a syringe.
The oxygen concentration in water is measured with a dissolved oxygen meter, OM-51-L1, manufactured by HORIBA.

16. Platelet-Retaining Rate

The platelet-retaining rate is calculated from the numbers of platelets in the blood found before and after the perfusion of the blood by the following method.

(1) Heparin calcium is added in a blood-collecting bag so that its concentration can be 5 U/mL, and the blood is collected into this bag from the vein of the inner elbow of a healthy adult. Before the perfusion of the blood, the blood is sampled for analysis of the blood components.
(2) The blood side and the dialysate side of a blood purifier having a membrane area of 1.5 $m^2$ are primed with physiologic saline, and the above heparin admixed blood in an entire amount is perfused at a flow rate of 150 mL/min. into the blood side of the blood purifier. At this step, a circuit is formed so that the blood from the blood-collecting bag can pass through the blood side of the blood purifier and return to the blood-collecting bag.
(3) After the blood perfusion for 60 minutes under an atmosphere of 37°C, the blood is sampled to analyze the blood components.
(4) The platelet-retaining rate is calculated from the numbers of platelets in the blood found before and after the blood perfusion, according to the following equation:

(Platelet-retaining rate) [%] = 100 X [{the number of platelets in the blood after the blood perfusion) X (hematocrit in the blood before the blood perfusion)}/(hematocrit in the blood after the blood perfusion)] ÷ (the number of platelets in the blood before the blood perfusion).

17. Cationic Dye-Adsorbing Rate

The cationic dye-adsorbing rate is a value which is calculated from the concentrations of a cationic dye in a solution found before and after the perfusion of the cationic dye solution, according to the following method. Methylene blue is used as the cationic dye.

(1) Methylene blue is dissolved in water so that the concentration thereof can be 0.5 ppm, to prepare a methylene blue solution.
(2) The methylene blue solution is sampled before it is allowed to contact a membrane.

(3) One thousand milliliters of the methylene blue solution is measured and is allowed to fill the blood side and the dialysate side of a blood purifier having a membrane area of 1.5 $m^2$.

(4) After the blood purifier is filled with the methylene blue solution, the rest of the methylene blue solution is pooled, and is perfused into the blood side of the blood purifier at a flow rate of 200 mL/min. At this step, a circuit is formed so that the solution flowing out of the solution pool can pass through the blood side of the blood purifier and return to the pool.

(5) After the perfusion for 5 minutes, the methylene blue solution filling the blood purifier is combined with the pooled methylene blue solution, and this solution mixture is sampled.

(6) An analytical curve is obtained from the absorbance of the methylene blue solution at the maximum absorption wavelength of 490 nm of the UV absorption spectrum, and the concentrations of the methylene blue solution before and after the contact with the membrane are measured.

(7) The methylene blue-adsorbing rate is calculated according to the following equation:

(Methylene blue-adsorbing rate) [%] = 100 X (the concentration of methylene blue in the solution after the perfusion of the solution)/(the concentration of methylene blue in the solution before the perfusion of the solution).

18. Rate of Increase in PF4

The rate of increase in platelet factor IV (PF4) is measured using a PF4-measuring reagent with EIA (sandwich method) manufactured by Roche Diagnostics, according to the measuring method described in the Japan Standard Product Classification No. 877422 (Approval No. 16200EZY0045300, revised edition, 2002) issued by the same company.

19. C Characteristic Value

Bovine blood containing hematocrit (35 mass %) is perfused into the inside of a hollow fiber membrane in a blood purifier, at a flow rate of 200 mL/min., and is simultaneously filtered from the inside of the hollow fiber membrane toward the outside thereof at a flow rate of 20 mL/min. The water permeability in the bovine blood system (hereinafter simply referred to as MFR) is calculated from the transmembrane pressure and the amount of filtrate found after 15 minutes has passed since the start of the perfusion and the filtration. This MFR value is represented by (A). An MFR value (B) is calculated by the same operation after 120 minutes has passed since the start of the perfusion and the filtration. The C characteristic value is calculated from the MFR values (A) and (B) by the equation: 100 (%) X (B)/(A).

20. Storage Stability of Blood Purifier

A blood purifier exposed to a radioactive ray is stored at a room temperature for one year, and then, the UV absorbance (at 220 to 350 nm) is measured by the above-described method. The storage stability is evaluated based on the degree of increase in UV absorbance (at 220 to 350 nm) because of the storage. The degree of increase in UV absorbance is determined by equally dividing the hollow fiber membrane bundle into 10 portions in the lengthwise direction, measuring the UV absorbance of each of samples from the 10 portions, and finding the maximum value out of the resultant values of UV absorbance for evaluating the degree of increase. A hollow fiber membrane bundle which shows a maximum value not exceeding 0.10 is passed as acceptable.

21. Performance-Exhibiting Rate of Blood Purifier after Priming Treatment

Physiologic saline is allowed to inflow from the inlet port of the blood side of a blood purifier (i.e. a priming treatment), and then, the water permeability of the blood purifier is evaluated by the above-described method, at points of time when 10 minutes and 24 hours have passed after the priming treatment, respectively. Then, the rate of the water permeability found after 10 minutes to the water permeability found after 24 hours is calculated. In this regard, the blood purifier filled with water is maintained at a room temperature until 24 hours has passed since the measurement of the water permeability after 10 minutes.

(Example 1)

Polyethersulfone (Sumika-Excel® 4800P, manufactured by Sumika Chemtex Co., Ltd.) (1,000 mass parts), polyvinyl pyrrolidone (Colidone® K-90, manufactured by BASF) (144 mass parts) and dimethylacetoamide (DMAc) (1,000 mass parts) were charged in a knead-melting machine of the type which efficiently kneaded the mixture by way of so-called planetary motions of two frame type blades which rotated by themselves and rotated around each other. The mixture was stirred and kneaded for 2 hours. Subsequently, a solution mixture of DMAc (3,000 mass parts) and RO water (160 mass parts) was added to the knead mixture in one hour. The mixture was further stirred for one. hour with the stirrer of which the number of revolutions was increased, to form a homogeneous solution. This kneading and dissolution was carried out under a nitrogen atmosphere. The mixture was kneaded and dissolved while being cooled so that its temperature did not exceed 40°C. The Froude number and the Reynolds number in the final dissolution were 1.0 and 100, respectively. Then, a vacuum pump was used to decompress the interior of the system to -500 mmHg, and the system was immediately sealed so as not to change the composition of the membrane-forming solution due to the evaporation of the solvent or the like, and the system was left to stand for 15 minutes. This operation was repeated three times to deaerate the membrane-forming solution. After the completion of the deaerating, the interior of the system was again displaced with a nitrogen gas and was kept to be weakly compressed. In this regard, polyvinyl pyrrolidone containing 125 ppm of hydrogen peroxide was used. The resultant membrane-forming solution was allowed to pass through two-staged sintered filters (30 μm and 15 μm) in this order. This solution was then discharged from a tube-in-orifice nozzle heated to 75°C, concurrently with an aqueous solution of 52 mass % of DMAc of 50°C as a hollow portion-forming solution? which had been previously deaerated under a reduced pressure of - 700 mmHg for 30 minutes. The strand of the discharged solutions was allowed to pass through a drying section with a length of 400 mm, sealed from an external atmosphere by a spinning tube, and was then solidified in an aqueous solution of 20 mass % of DMAc of 60°C. The resulting wet strand was directly wound onto a hank. The width of the nozzle slit of the tube-in-orifice nozzle was average 60 μm, maximum 61 μm and minimum 59 μm. The ratio of the maximum value to the minimum value of the slit width was 1.03, and the draft ratio was 1.1. The rollers which the hollow fiber membrane contacted during the spinning step were all planished at their surfaces, and the guides were all mat-finished at their surfaces. A bundle of about 10,000 hollow fiber membranes was wrapped in a polyethylene film which was embossing-finished at its surface on the side of the bundle of hollow fiber membranes. The same bundle was then cut into several portions with lengths of 27 cm. The cut portion of the bundle was washed in hot water of 80°C for 30 minutes, and this washing was repeated four times.

The obtained wet hollow fiber bundle was introduced into a microwave-exposure type drier in which a reflecting plate was provided in its oven so as to enable even heating. Then, the hollow fiber membrane bundle was dried under the following conditions: the membrane bundle was heated by exposure to microwave of an output of 1.5 kW and at a decompression degree of 7 kPa for 30 minutes, and then, the microwave exposure was stopped and simultaneously the decompression degree was increased to 1.5 kPa, which was then maintained for 3 minutes. Subsequently, the decompression degree was returned to 7 kPa, and the hollow fiber membrane was heated by exposure to microwave of an output of 0.5 kW for 10 minutes. Then, the microwave exposure was stopped, and the decompression degree was increased to 0.7 kPa, which was maintained for 3 minutes. The decompression degree was again returned to 7 kPa, and the hollow fiber membrane bundle was heated by exposure to microwave of an output of 0.2 kW for 8 minutes. Then, the microwave exposure was stopped, and the decompression degree was increased to 0.5 kPa, which was maintained for 5 minutes, to thereby make the conditioning of the hollow fiber membrane bundle. Thus, the drying step was completed. In this drying step, the highest temperature of the surface of the hollow fiber membrane bundle was 65°C. The water content of the hollow fiber membrane bundle before dried was 330 masts %; that found after the completion of the first drying step, 32 mass %; that found after the completion of the second drying step, 16 mass %; and that found after the completion of the third drying step, 1.5 mass %.

The resultant hollow fiber membrane bundle was regularly divided at 2.7 cm intervals in the lengthwise direction, to obtain 10 portions thereof, and 1 g of the hollow fiber membrane in a dried state was weighed from each of the portions and was then subjected to a test regulated in the Approval Standard for Dialysis-Type Artificial Kidney Apparatus to obtain an eluate therefrom. Then, the amount of hydrogen peroxide therein and the UV absorbance (220 to 350 nm) were measured. The amounts of hydrogen peroxide and the UV absorbances from all the portions were stable at low levels. Therefore, no partial sticking was observed in the hollow fiber membrane bundle. The results of the measurements are shown in Tables 1 and 2.

The hollow fiber membrane bundle prepared by the above-described method was inserted into a polycarbonate blood purifier container and was fixed at its both end portions with an urethane resin. The resin end portions were cut to open the hollow portions of the hollow fiber membranes, and then, caps having inlets were attached to the end portions of the hollow fiber membrane bundle. Thus, a blood purifier of which the effective length of the permselective hollow fiber membrane was 215 mm and of which the membrane area was 1.35 $m^2$ was assembled. In the meantime, deoxygenerated water of which the concentration of dissolved oxygen was 0.05 ppm was prepared by allowing RO water to

pass through a deaerated hollow fiber membrane module, and a nitrogen gas was bubbled in this deoxygenerated water to prepare water saturated with nitrogen. This water saturated with nitrogen was allowed to flow into the blood side of the blood purifier at a flow rate of 200 ml/min. for 5 minutes to fill the blood side. Then, the flowing of the water into the blood side of the blood purifier was stopped, and then, the blood side was purged of the filling water under a pressure of 0.1 MPa, using an air of 60°C. The air was allowed to further pass through the blood side to adjust the water content in the hollow fiber membrane to 10 mass %. All the inlets and the outlets of the blood side and the dialysate side of the blood purifier dried under the above conditions were tightly sealed with caps made of an ethylene-propylene-based synthesized rubber. Then, the blood purifier was sealed in a packaging bag which comprised a lamination of an outer layer consisting of a biaxially oriented polyamide film with a thickness of 25 μm and an inner layer consisting of a non-oriented polyethylene film with a thickness of 50 μm. The blood purifier in a sealed state was stored at a room temperature for 72 hours, and was then exposed to γ-ray at a dose of 25 kGy.

The characteristics of the blood purifier obtained by the above-described method and of the hollow fiber membrane in the blood purifier are shown in Table 3. The hollow fiber membrane (i.e. the polysulfone-based permselective hollow fiber membrane) obtained in this Example showed a small amount of eluting hydrogen peroxide and a low UV absorbance (at 220 to 350 nm) even after the γ-ray exposure, and thus was found to maintain its high quality. Other characteristic thereof were also sufficient. Also, the blood purifier showed a small amount of eluting polyvinyl pyrrolidone and also showed a sufficient water permeability-exhibiting rate after the priming treatment and sufficient storage stability, and thus was found to be highly reliable in practical use.

[Table 1] (Part 1)

| Measured Site | Ex.1 | Ex.2 | Ex.3 | Ex.4 |
|---|---|---|---|---|
| 1 | 1 | 2 | 2 | 2 |
| 2 | 2 | 2 | 2 | 2 |
| 3 | 2 | 1 | 1 | 1 |
| 4 | 0 | 0 | 1 | 1 |
| 5 | 0 | 0 | 0 | 0 |
| 6 | 0 | 0 | 0 | 0 |
| 7 | 0 | 0 | 0 | 0 |
| 8 | 1 | 0 | 0 | 0 |
| 9 | 2 | 1 | 2 | 2 |
| 10 | 1 | 2 | 1 | 2 |
| Maximum elution amount | 2 | 2 | 2 | 2 |
| Minimum elution amount | 0 | 0 | 0 | 0 |
| Average elution amount | 0.9 | 0.8 | 0.9 | 1.0 |
| Deviation in elution amount | 2 | 2 | 2 | 2 |
| Degree of variation in elution amount | 1.1 | 1.2 | 1.1 | 1.0 |

(Part 2)

| Measured Site | C.Ex.1 | C.Ex.2 | C.Ex.3 | C.Ex.4 | C.Ex.5 | C.Ex.6 |
|---|---|---|---|---|---|---|
| 1 | 8 | 8 | 10 | 1 | 2 | 1 |
| 2 | 1 | 4 | 8 | 1 | 2 | 0 |
| 3 | 5 | 5 | 11 | 2 | 2 | 2 |
| 4 | 4 | 7 | 7 | 2 | 0 | 0 |
| 5 | 3 | 10 | 9 | 3 | 0 | 2 |
| 6 | 4 | 9 | 8 | 1 | 0 | 0 |
| 7 | 4 | 10 | 9 | 3 | 0 | 0 |
| 8 | 6 | 4 | 5 | 2 | 1 | 1 |
| 9 | 3 | 8 | 4 | 0 | 1 | 2 |
| 10 | 7 | 7 | 6 | 1 | 1 | 1 |
| Maximum elution amount | 8 | 10 | 11 | 3 | 2 | 2 |
| Minimum elution amount | 1 | 4 | 4 | 0 | 0 | 0 |
| Average elution amount | 4.5 | 7.2 | 7.7 | 1.6 | 0.9 | 0.9 |
| Deviation in elution amount | 7 | 6 | 7 | 3 | 2 | 2 |
| Degree of variation in elution amount | 3.5 | 3.2 | 3.7 | 1.6 | 1.1 | 1.1 |

[Table 2] (Part 1)

| Measured Site | Ex.1 | Ex.2 | Ex.3 | Ex.4 |
|---|---|---|---|---|
| 1 | 0.02 | 0.02 | 0.03 | 0.02 |
| 2 | 0.02 | 0.02 | 0.02 | 0.03 |
| 3 | 0.02 | 0.02 | 0.03 | 0.03 |
| 4 | 0.03 | 0.02 | 0.03 | 0.02 |
| 5 | 0.03 | 0.03 | 0.02 | 0.02 |
| 6 | 0.02 | 0.03 | 0.02 | 0.02 |
| 7 | 0.02 | 0.03 | 0.02 | 0.02 |
| 8 | 0.02 | 0.02 | 0.04 | 0.04 |
| 9 | 0.02 | 0.02 | 0.02 | 0.02 |
| 10 | 0.03 | 0.03 | 0.03 | 0.02 |
| Max. | 0.03 | 0.03 | 0.04 | 0.04 |
| Ave. | 0.023 | 0.024 | 0.026 | 0.024 |
| Partial sticking | None | None | None | None |

(Part 2)

| Measured Site | C.Ex.1 | C.Ex.2 | C.Ex.3 | C.Ex.4 | C.Ex.5 | C.Ex.6 |
|---|---|---|---|---|---|---|
| 1 | 0.05 | 0.09 | 0.08 | 0.04 | 0.02 | 0.02 |
| 2 | 0.07 | 0.03 | 0.04 | 0.08 | 0.03 | 0.02 |
| 3 | 0.06 | 0.02 | 0.03 | 0.10 | 0.02 | 0.02 |
| 4 | 0.10 | 0.05 | 0.05 | 0.09 | 0.03 | 0.03 |
| 5 | 0.05 | 0.06 | 0.06 | 0.11 | 0.03 | 0.02 |
| 6 | 0.02 | 0.10 | 0.11 | 0.07 | 0.02 | 0.02 |
| 7 | 0.04 | 0.12 | 0.10 | 0.12 | 0.02 | 0.02 |
| 8 | 0.08 | 0.10 | 0.12 | 0.06 | 0.02 | 0.02 |
| 9 | 0.05 | 0.09 | 0.11 | 0.03 | 0.02 | 0.03 |
| 10 | 0.06 | 0.05 | 0.05 | 0.05 | 0.02 | 0.03 |
| Max. | 0.10 | 0.12 | 0.12 | 0.12 | 0.03 | 0.03 |
| Ave. | 0.058 | 0.071 | 0.075 | 0.075 | 0.023 | 0.023 |
| Partial Sticking | Some | None | None | None | None | None |

[Table 3] (Part 1)

| | Ex.1 | Ex.2 | Ex.3 | Ex.4 |
|---|---|---|---|---|
| Water content (mass %) after dried | 1.5 | 2.7 | 1.5 | 1.5 |
| Water content (mass %) during sterilization | 10 | 50 | 10 | 10 |
| Dissolved oxygen concentration (ppm) | 0.03 | 0.12 | 0.03 | 0.04 |
| Standing time until sterilization (hr.) | 72 | 216 | 120 | 50 |
| Water permeability (ml/m$^2$/hr./mmHg) | 487 | 365 | 580 | 307 |
| PVP eluting amount (ppm) | 4 | 5 | 5 | 4 |
| Thickness of hollow fiber membrane (μm) | 31 | 27 | 43 | 43 |
| PVP content in outer surface (mass %) | 32 | 29 | 33 | 36 |
| PVP content in hollow fiber membrane (mass %) | 6.3 | 7.5 | 10.1 | 7.9 |
| porosity in outer surface (%) | 22 | 20 | 15 | 24 |
| Hydrogen peroxide eluting amount (ppm) (maximum value) after sterilization | 2 | 3 | 3 | 3 |
| UV absorbance (Abs) (maximum value) | 0.04 | 0.04 | 0.04 | 0.04 |
| Crosslinking of PVP | done | done | done | done |
| Water permeability-exhibiting rate (%) after priming | 95 | 94 | 95 | 93 |
| Storage stability of blood purifier | ○ | ○ | ○ | ○ |

(Part 2)

| | C.Ex.1 | C.Ex.2 | C.Ex.3 | C.Ex.4 | C.Ex.5 | C.Ex.6 |
|---|---|---|---|---|---|---|
| Water content (mass %) after dried | 0.5 | 0.5 | 0.5 | 1.5 | 1.5 | 1.5 |
| Water content (mass %) during sterilization | 10 | 10 | 10 | 1.5 | 10 | 10 |
| Dissolved oxygen concentration (ppm) | 0.51 | 0.51 | 0.76 | 0.04 | 0.03 | 0.04 |
| Standing time until sterilization (hr.) | 50 | 120 | 216 | 168 | 24 | 40 |
| Water permeability (ml/m$^2$/hr./mmHg) | 498 | 499 | 497 | 498 | 487 | 487 |
| PVP eluting amount (ppm) | 5 | 8 | 12 | 18 | 5 | 4 |
| Thickness of hollow fiber membrane (μm) | 30 | 30 | 29 | 31 | 31 | 30 |
| PVP content in outer surface (mass %) | 35 | 36 | 35 | 34 | 32 | 31 |
| PVP content in hollow fiber membrane (mass %) | 6.4 | 6.5 | 6.6 | 6.5 | 6.4 | 6.3 |
| porosity in outer surface (%) | 22 | 21 | 20 | 21 | 22 | 22 |
| Hydrogen peroxide eluting amount (ppm) (maximm value) after sterilization | 20 | 10 | 13 | 10 | 2 | 2 |
| UV absorbance (Abs) (maximm value) | 0.21 | 0.24 | 0.29 | 0.14 | 0.04 | 0.04 |
| Crosslinking of PVP | done | done | done | not done | done | done |
| Water permeability-exhibiting rate (%) after priming | 88 | 83 | 78 | 79 | 81 | 89 |
| Storage stability of blood purifier | x | x | x | x | O | O |

(Comparative Example 1)

A hollow fiber membrane bundle was obtained in the same manner as in Example 1, except that polyvinyl pyrrolidone containing 500 ppm of hydrogen peroxide was used as a raw material, that the kneading/dissolving temperature was set at 85°C, that the interiors of the raw material-supplying system and the dissolution tank were not displaced with nitrogen gases, and that the hollow fiber membrane bundle was dried by exposure to microwave under an atmospheric pressure. The hollow fiber membrane bundle was exposed to microwave of an output of 2 kW until the water content of the hollow fiber membrane bundle reached 65 mass %. After that, the hollow fiber membrane bundle was exposed to microwave of an output of 0.8 kW until the water content of the hollow fiber membrane bundle reached 0.5 mass %. Further, a dehumidified air (of a humidity of 10% or lower) was fed from the lower side of the hollow fiber membrane bundle to the upper side thereof at an air velocity of 8 m/second during a period of time from the drying-starting time to the drying-completing time. The highest temperature of the hollow fiber membrane bundle during the drying step was 65°C. The characteristics of the resultant hollow fiber membrane bundle are shown in Tables 1 and 2. The amount of eluting hydrogen peroxide from the hollow fiber membrane of this Comparative Example was of high level, and the variation in the amount of eluting hydrogen peroxide was large in each of the sampling sites of the bundle, and thus, the quality of this hollow fiber membrane bundle was low. Further, the level of the UV absorbance (220 to 350 nm) was high, and variation in UV absorbance was large, and the partial sticking of the hollow fiber membranes was observed.

A blood purifier was assembled using the hollow fiber membrane obtained by the above-described method, in the same manner as in Example 1, except that water which was not subjected to a deoxygenerating treatment and an inert gas-substituting treatment was used. The blood purifier was stored for 50 hours and then was sterilized.

The characteristics of the blood purifier obtained by the above-described method and of the hollow fiber membrane in the blood purifier are shown in Table 3. The hollow fiber membrane (i.e. the polysulfone-based permselective hollow fiber membrane) obtained in this Comparable Example showed an increased amount of eluting hydrogen peroxide due to the γ-ray exposure and was poor in storage stability. Thus, this blood purifier was of low quality.

(Comparative Example 2)

A blood purifier was assembled and sterilized in the same manners as in Comparative Example 1, except that the inner atmosphere of the blood purifier was not displaced with an inert gas, and that the storage time until the sterilization was changed to 120 hours. The results are shown in Tables 1 to 3. In this Comparative Example, the water in the hollow fiber membrane was not saturated with nitrogen, as well as Comparative Example 1. Therefore, an oxygen gas was dissolved in the water in the hollow fiber membrane, and the effect of the water content in the hollow fiber membrane, i.e. the effect of such a water content's inhibiting the deterioration of polyvinyl pyrrolidone caused by the γ-ray exposure became lower. Accordingly, the hydrogen peroxide-eluting amount increased due to the γ-ray exposure, and the UV absorbance (at 220 to 350 nm) became higher. The storage stability of the blood purifier, therefore, became poor. In addition, the water permeability-exhibiting rate of the blood purifier after the priming treatment was inferior. Thus, the blood purifier was of low quality.

(Comparative Example 3)

A blood purifier was assembled and sterilized in the same manners as in Comparative Example 2, except that the inlets and the outlets of the blood purifier were not tightly sealed, and that the storage time until the sterilization was changed to 216 hours. The results are shown in Tables 1 to 3. In this Comparative Example, the water in the hollow fiber membrane was not saturated with nitrogen, and the inlets and the outlets of the blood side and the dialysate side were not tightly sealed. Therefore, an air infiltrated the blood purifier, and filled the ambient atmosphere around the hollow fiber membrane bundle during the γ-ray exposure. Accordingly, the deterioration of the hollow fiber membrane further proceeded, in comparison with Comparative Example 2.

(Comparative Example 4)

A permselective hollow fiber membrane bundle and a blood purifier were obtained in the same manners as in Example 1, except that the water content in the permselective hollow fiber membrane was not controlled (namely, no oxygen scavenger was used, and water was not saturated with an inert gas), and that the storage time until the sterilization was changed to 168 hours. In this Comparative Example, polyvinyl pyrrolidone in the permselective hollow fiber membrane packed in the blood purifier was not crosslinked because of the low water content in the hollow fiber membrane. Therefore, the amount of eluting polyvinyl pyrrolidone became larger, and thus, this blood purifier was of low quality. Further, the effect of the nitrogen-saturated water's inhibiting the deterioration of the hollow fiber membrane during the γ-ray 8 minutes. Then, the microwave exposure was stopped, and the decompression degree was increased to 0.5 kPa, which was maintained for 5 minutes, to thereby make the conditioning of the hollow fiber membrane bundle. Thus, the drying of the bundle was completed. In this drying step, the highest temperature of the surface of the hollow fiber membrane bundle was 65°C. The water content of the hollow fiber membrane bundle before dried was 318 mass %; that found after the completion of the first drying step, 30 mass %; that found after the completion of the second drying step, 15 mass %; and that found after the completion of the third drying step, 2.7 mass %. The rollers for changing the fiber path during the spinning step were all planished at their surfaces, and the stationary guides were all mat-finished at their surfaces. The inner diameter of the resultant hollow fiber membrane bundle was 200 μm, and the thickness of the membrane was 27 μm.

The resultant hollow fiber membrane bundle was equally divided into 10 portions in the lengthwise direction, and 1 g of the hollow fiber membrane bundle in a dried state was weighed from each of the 10 portions so as to determine the eluting amount of hydrogen peroxide therefrom. The eluting amounts of hydrogen peroxide from all the portions of the hollow fiber membrane bundle were stable at low levels. The determined values are shown in Tables 1 and 2.

A blood purifier was assembled in the same manner as in Example 1, using the hollow fiber membrane bundle thus obtained. The blood purifier was exposed to γ-ray in the same manner as in Example 1, except that the water content in the permselective hollow fiber membrane nozzle slit of the tube-in-orifice nozzle was average 45 μm, maximum 45.5 μm and minimum 44.5 μm. The ratio of the maximum value to the minimum value of the slit width was 1.02, and the draft ratio was 1.2. The hollow fiber membrane removed from the coagulation bath was allowed to pass through a

water washing bath of 85°C for 45 seconds to remove excesses of the solvent and polyvinyl pyrrolidone. After that, the hollow fiber membrane was wound up. Then, the bundle of about 10,000 hollow fiber membranes was wrapped in a polyethylene film which was the same one as that used in Example 1, and then was immersed in an aqueous solution of 40 vol % of isopropanol of 30°C for 30 minutes. This immersion was repeated twice for washing.

The obtained wet hollow fiber bundle was introduced into a microwave-exposure type drier in which a reflecting plate was provided in its oven so as to enable even heating. Then, the hollow fiber membrane bundle was dried under the following conditions: the hollow fiber membrane bundle was heated by exposure to microwave of an output of 1.5 kW and at a decompression degree of 7 kPa for 30 minutes; and then, the microwave exposure was stopped and simultaneously the decompression degree was increased to 1.5 kPa, which was maintained for 3 minutes. Subsequently, the decompression degree was returned to 7 kPa, and the hollow fiber membrane bundle was heated by exposure to microwave of an output of 0.5 kW for 10 minutes. Then, the microwave exposure was stopped, and the decompression degree was increased to 0.7 kPa, which was maintained for 3 minutes. The decompression degree was returned to 7 kPa, and the hollow fiber membrane bundle was heated by.exposure to microwave of an output of 0.2 kW for parts), polyvinyl pyrrolidone (Colidone® K-90, manufactured by BASF) (200 mass parts) and DMAc (1,500 mass parts) were kneaded with a twin-screw type kneading machine. The knead mixture was introduced into a stirring type dissolution tank charged with DMAc (2,500 mass parts) and water (280 mass parts), and the mixture was stirred and dissolved for 3 hours. The mixture was kneaded and dissolved while the tank was being cooled so that the internal temperature did not exceed 30°C. Then, a vacuum pump was used to decompress the interior of the system to -700 mmHg, and the dissolution tank was immediately sealed so as not to change the composition of the membrane-forming solution due to the evaporation of the solvent or the like, and the dissolution tank was left to stand for 10 minutes. This operation was repeated three times to deaerate the membrane-forming solution. In this regard, polyvinyl pyrrolidone containing 100 ppm of hydrogen peroxide was used. The supply tank and the dissolution tank in the raw material-supply system were displaced with nitrogen gases. The Froude number and the Reynolds number in the dissolution were 1.1 and 120, respectively. The resultant membrane-forming solution was allowed to pass through two-staged filters (15 μm and 15 μm). This solution was then discharged from a tube-in-orifice nozzle heated to 70°C, concurrently with an aqueous solution of 50 mass % of DMAc of 50°C as a hollow portion-forming material which had been previously deaerated under a reduced pressure of -700 mmHg for 2 hours. The strand of the discharged solutions was allowed to pass through an air gap with a length of 350 mm, sealed from an external atmosphere by a spinning tube, and was then solidified in water of 60°C. The width of the exposure became lower, and therefore, the deterioration reaction of polyvinyl pyrrolidone was accelerated, and the amount of eluting hydrogen peroxide became larger due to the γ-ray exposure, and further, the UV absorbance (at 220 to 350 nm) became higher. Inevitably, the storage stability of the blood purifier was poor. Furthermore, the water permeability-exhibiting rate after the priming treatment was low.

(Reference Examples 1 and 2)

Permselective hollow fiber membranes and blood purifiers were obtained in the same manners as in Example 1, except that the blood purifiers were tightly sealed in packaging bags and were then stored at room temperatures for 24 hours and for 40 hours, respectively, and then were exposed to γ-ray under the same conditions as in Example 1. The characteristics of the hollow fiber membranes and the blood purifiers are shown in Tables 1 to 3. In these Comparative Examples, because of the short periods of time from the sealing of the blood purifiers until the γ-ray exposure, the water permeability-exhibiting rates of the blood purifiers after the priming treatments were inferior to that of the blood purifier of Example 1. Accordingly, the blood purifiers of these Comparative Examples had low reliability in practical use. Also, it was known that the period of time from the sealing of the blood purifier to the γ-ray exposure gave some influence on the water permeability-exhibiting rate of the blood purifier after the priming treatment.

(Example 2)

Polyethersulfone (Sumika-Excel® 4800P, manufactured by Sumika Chemtex Co., Ltd.) (1,000 mass polyvinyl pyrrolidone, and then was wound up. A bundle of about 10,000 hollow fiber membranes thus obtained was immersed in pure water, and was washed in an autoclave at 121°C for one hour. Then, the hollow fiber membrane bundle was wrapped in a polyethylene film which was the same one as used in Example 1. After that, the hollow fiber membrane bundle was put in a container displaced with a nitrogen gas and was subjected to a crosslinking treatment by exposure to γ-ray at a dose of 25 kGy. The maximum hydrogen peroxide eluting amount from the hollow fiber membrane bundle found before the crosslinking treatment was 2 ppm. Subsequently, the hollow fiber membrane bundle was dried in the same manner as in Example 1. The rollers for changing the fiber path during the spinning step were planished at their surfaces, and the stationary guides were mat-finished at their surfaces. The resultant hollow fiber membrane bundle had an inner diameter of 201 μm, and the thickness of the membrane was 43 μm. As is apparent from Tables 1 and 2, the hydrogen peroxide eluting amounts were stable at low levels in all the sites of the hollow fiber membrane bundle.

A blood purifier was assembled, using the hollow fiber membrane bundle thus obtained, in the same manner as in Example 1, except that deaerated water was used as water for use in the control of the water content in the hollow fiber membrane, and that the water content was controlled to 4.5 mass %. The blood purifier was sealed together with one general-purpose oxygen scavenger (Tamotsu® manufactured by OJTACK) in the same packaging bag as used in Example 1, and was then left to stand at a room temperature for 120 hours, and was then exposed to γ-ray was controlled to 280 mass %, and that the storage time until the sterilization was changed to 216 hours.

The permselective hollow fiber membrane bundle and the blood purifier obtained in this Example were of high quality, as well as those obtained in Example 1. The results are shown in Table 3.

(Exampl 3)

A membrane-forming solution comprising polysulfone (P-3500, manufactured by AMOCO) (900 mass %), polyvinyl pyrrolidone (Colidone® K-60, manufactured by BASF) (450 mass %), dimethylacetoamide (DMAc) (3,500 mass %) and water (250 mass %) was prepared in the same manner as in Example 2. The polyvinyl pyrrolidone contained 100 ppm of hydrogen peroxide. The resultant membrane-forming solution was allowed to pass through two-staged filters of 15 μm and 15 μm, and was then discharged from a tube-in-orifice nozzle heated to 40°C, concurrently with an aqueous solution of 55 mass % of DMAc of 60°C as a hollow portion-forming material which had been previously deaerated. The strand of the discharged solutions was allowed to pass through an air gap section with a length of 600 mm, sealed from an external atmosphere by a spinning tube, and was then solidified in water of 50°C. The width of the nozzle slit of the tube-in-orifice nozzle was average 60 μm, maximum 61 μm and minimum 59 μm. The ratio of the maximum value to the minimum value of the slit width was 1.03, and the draft ratio was 1.1. The hollow fiber membrane was removed from the coagulation bath, and was allowed to pass through a water bath of 85°C for 45 seconds to thereby remove the solvent and an excess of the seconds to thereby remove the solvent and an excess of the polyvinyl pyrrolidone, and then was wound up. A bundle of about 10,000 hollow fiber membranes was immersed in pure water, and was washed in an autoclave at 121°C for one hour.

After the washing, the wet hollow fiber membrane bundle was wrapped in a polyethylene film. After that, such wet hollow fiber membrane bundles wrapped in the films were set on two-staged turn tables (48 hollow fiber membrane bundles on each turn table) in a drying apparatus and were exposed to microwave of an output of 12 kW and at a decompression degree of 7 kPa for 15 minutes for a heat treatment. Then, the microwave exposure was stopped, and the decompression degree was increased to 1 kPa, and this condition was maintained for 3 minutes to evaporate the water contents from the hollow fiber membrane bundles. Next, the decompression degree was returned to 7 kPa, and simultaneously, the hollow fiber membrane bundles were exposed to microwave of an output of 3.5 kW for 7 minutes for a heat treatment. After the heating, the microwave exposure was stopped, and the decompression degree was increased to 0.8 kPa, and this condition was maintained for 3 minutes. Again, the decompression degree was returned to 7 kPa, and the exposure to microwave of an output of 2.5 kW was restarted and continued for 5 minutes. After that, the microwave exposure was stopped, and the decompression degree was increased to 0.5 kPa, which was maintained for 7 minutes to dry the hollow fiber membrane bundles. Further, the hollow fiber membrane bundles were treated in an air drier at 35°C for 3 hours, so as to uniform the water contents of the hollow fiber membrane bundles. The water contents of the bundles found before the drying by exposure under the same conditions as in Example 1. In this Example, the manufacturing of the hollow fiber membrane bundle and the assembling of the blood purifier were conducted in a clean room of class 100,000. The permselective hollow fiber membrane bundle and the blood purifier obtained in this Example were of high quality, as well as those obtained in Example 1. The results are shown in Table 3.

(Example 4)

A membrane-forming solution comprising polysulfone (P-1700, manufactured.by AMOCO) (850 mass %, polyvinyl pyrrolidone (Colidone® K-60, manufactured by BASF) (250 mass %), dimethylacetoamide (DMAc) (3,700 mass %) and water (250 mass %) was prepared in the same manner as in Example 2. The polyvinyl pyrrolidone contained 120 ppm of hydrogen peroxide. The resultant membrane-forming solution was allowed to pass through a two-staged filter of 15 μm and 15 μm, and was then discharged from a tube-in-orifice nozzle heated to 40°C, concurrently with an.aqueous solution of 35 mass % of DMAc of 60°C as a hollow portion-forming material which had been previously deaerated. The strand of the discharged solutions was allowed to pass through an air gap section with a length of 600 mm, sealed from an external atmosphere by a spinning tube, and was then solidified in water of 50°C. The width of the nozzle slit of the tube-in-orifice nozzle was average 60 μm, maximum 61 μm and minimum 59 μm. The ratio of the maximum value to the minimum value of the slit width was 1.03, and the draft ratio was 1.1. The hollow fiber membrane was removed from the coagulation bath, and was allowed to pass through a water bath of 85°C for 45 to microwave were 335 mass %; those found after the completion of the treatment in the first step, 26 mass %; those found after the completion of the treatment in the second step, 13 mass %; those found after the completion of the treatment in the third

step, 5.3 mass %; and those found after the completion of the air-drying, 1.5 mass %. The highest temperature of the hollow fiber membrane bundles during the drying treatment was 56°C. The rollers for changing the fiber path during the spinning step were planished at their surfaces, and the stationary guides were mat-finished at their surfaces. The inner diameter of the resultant hollow fiber membrane bundles was 200 μm, and the thickness of the membranes was 43 μm. As is apparent from Tables 1 and 2, the hydrogen peroxide eluting amounts were stable at low levels in all the sites of the hollow fiber membrane bundles.

A blood purifier was assembled, using the hollow fiber membrane bundle thus obtained, in the same manner as in Example 3, except that the water content in the hollow fiber membrane was changed 360 mass %. The blood purifier was sterilized in the same manner as in Example 1, except that the blood purifier was exposed to an electron beam with an electron exposure apparatus of accelerating voltage of 5,000 KV, instead of γ-ray, after 50 hours had passed since the sealing and the packing of the blood purifier. The permselective hollow fiber membrane bundle and the blood purifier obtained in this Example were of high quality, as well as those obtained in Example 3. The results are shown in Table 3.

Hitherto, there has been reported no method for managing the qualities of hollow fiber membrane bundles by paying concentrated attentions to the behavior of hydrogen peroxide. The qualities of hollow fiber membrane bundles are evaluated from many view points. In the present invention, the following method is employed. A hollow fiber membrane bundle is cut along its lengthwise direction into portions with lengths of 27 cm, and each portion thereof is divided at regular 2.7 cm intervals, so as to measure the hydrogen peroxide eluting amounts from the respective sites of these intervals. The resultant values are averaged to find an average eluting amount. The difference between the maximum eluting amount and the minimum eluting amount is defined as a range in eluting amount. Further, a larger one of two values, i.e., the absolute value of the difference between the maximum eluting amount and the average eluting amount and the absolute value of the difference between the minimum eluting amount and the average eluting amount, is defined as a variation degree in eluting amount. The values of the range in eluting amount and the variation degree in eluting amount are shown in Table 1. The variations in hydrogen peroxide eluting amounts of Example 1 and Comparative Example 1 are shown in Fig. 1.

Fig. 2 shows a graph of the plotting of the maximum hydrogen peroxide eluting amounts and the variation degrees in eluting amount. When the hydrogen peroxide eluting amount increases and exceeds 5 ppm, unbalance occurs in the hydrogen peroxide eluting amounts from the respective sites of the 10 portions of the hollow fiber membrane bundle, and therefore, the range in eluting amount between each of the sites becomes large. The fact that the hydrogen peroxide eluting amount differs at each of the sites of the hollow fiber membrane bundle in spite of the use of the same materials is undesirable in view of the quality management of hollow fiber membranes, because such difference affects the performance and functions of the hollow fiber membranes. Thus, it can be understood that a hollow fiber membrane bundle showing no unbalance in the hydrogen peroxide eluting amounts from its respective sites is excellent in quality. Further, it can be understood that a range of about 5 ppm is critical in view of suppression of the variation degree.

Fig. 3 shows a graph indicating a relationship between the period of time from the sealing of the inlets and the outlets of the blood purifier until the γ-ray exposure and the water permeability-exhibiting rate of the blood purifier after the priming treatment. It can be understood that this period of time gives a critical influence on the water permeability-exhibiting rate of the blood purifier after the priming treatment.

## INDUSTRIAL APPLICABILITY

Since the blood purifier used in present invention is of dry type, the blood purifier has the following advantages: it is light in weight; it is not frozen; and bacteria are hard to proliferate.therein. Further, the blood purifier used in present invention shows an excellent water permeability-exhibiting rate after a priming treatment, and has an advantage in that the priming treatment can be done in a shorter time. There is further advantage in that the operation to previously wash off a radical-trapping agent from the blood purifier is unnecessary because no radical-trapping agent is contained. Furthermore, in the present invention, there is produced an effect which has never been achieved by any of the conventional techniques: that is, the deterioration of the permselective hollow fiber membrane due to radiation exposure can be inhibited, even when the blood purifier in a dried state is exposed to a radioactive ray in the absence of a radical-trapping agent. Therefore, the amount of hydrogen peroxide generated by the above deterioration reaction is small, and thus, the blood purifier of the present invention is excellent in long-term storage stability. For example, in the polysulfone-based permselective hollow fiber membrane packed in the blood purifier, the generation of hydrogen peroxide is inhibited even under the radiation exposure, and thus, the deterioration of polyvinyl pyrrolidone, etc. induced by the hydrogen peroxide is inhibited. Therefore, the maximum value of the UV absorbance (at 220 to 350 nm) regulated in the Approval Standard for Dialysis-Type Artificial Kidney Apparatus can be maintained at 0.10 or less, even after the long-term storage of the blood purifier. Accordingly, the blood purifier is ensured in safety relative to the long-term storage. Therefore, the present invention will make not a little contribution to this industrial field.

## Claims

1. A method for providing a sterilized blood purifier, wherein

   a blood purifier is assembled using a polyvinyl pyrrolidone-containing polysulfone-based permselective hollow fiber membrane bundle, wherein
   the amount of polyvinyl pyrrolidone which elutes from the hollow fiber membrane bundle is 10 ppm or less,
   the amounts of hydrogen peroxide which elute from extracts from all the sites of the hollow fiber membrane bundle are 5 ppm or less, when the hollow fiber membrane bundle is divided into 10 portions in the lengthwise direction to test the sites of all the 10 portions according to the method regulated in the Approval Standard for Dialysis-Type Artificial Kidney Apparatus, and
   the water permeability of the blood purifier found at a point of time when 10 minutes have passed after the priming treatment of the blood purifier is 90% or more of the water permeability of the same found at a point of time when 24 hours have passed after the priming treatment thereof, and

   the content of polyvinyl pyrrolidone in the uppermost layer of the inner surface of the permselective hollow fiber membrane is from 5 to 50 mass %,
   **characterized in that** said blood purifier, loaded with the polysulfone-based permselective hollow fiber membrane bundle which is adjusted in water content to 5 to 600 mass % by using deaerated water having an oxygen concentration of 0.5 ppm or less, with all its inlets and outlets for blood and a dialysate tightly sealed, is sealed in a packaging bag capable of shutting out an external air and water vapor,
   and then the blood purifier is exposed to a radioactive ray after at least 48 hours have passed since the tight sealing of all the inlets and outlets for the blood and the dialysate of the blood purifier.

2. The method of claim 1, wherein the content of polyvinyl pyrrolidone in the uppermost layer of the outer surface of the permselective hollow fiber membrane is from 25 to 50 mass %.

3. The method of claim 1, wherein the deaerated water present in and around the polysulfone-based permselective hollow fiber membrane is water saturated with an inert gas.

4. The method of any one of claims 1 to 3, wherein the amount of a polysulfone-based resin in the permselective hollow fiber membrane is from 99 to 80 mass %, and the amount of polyvinyl pyrrolidone in the permselective hollow fiber membrane is from 1 to 20 mass %.

## Patentansprüche

1. Ein Verfahren zur Bereitstellung eines sterilisierten Blutreinigers, wobei

   ein Blutreiniger unter Verwendung eines Polyvinylpyrrolidon-haltigen permselektiven Hohlfasermembranbündels auf Polysulfonbasis zusammengestellt wird, wobei

      die Menge an Polyvinylpyrrolidon, die von dem Hohlfasermembranbündel eluiert wird, 10 ppm oder weniger beträgt,
      die Mengen an Wasserstoffperoxid, welche aus Extrakten aus allen Stellen des Hohlfasermembranbündels eluiert werden, 5 ppm oder weniger betragen, wenn das Hohlfasermembranbündel in 10 Abschnitte in Längsrichtung unterteilt wird, um die Stellen aller 10 Abschnitte gemäß dem in der Zulassungsnorm für einen künstlichen Nierenapparat vom Dialyse-Typ festgelegten Verfahren zu testen und
      die Wasserpermeabilität des Blutreinigers zu einem Zeitpunkt, wenn 10 Minuten nach der Vorbehandlung des Blutreinigers vergangen sind, 90% oder mehr der Wasserdurchlässigkeit desselben zu einem Zeitpunkt, wenn 24 Stunden nach der Vorbehandlung desselben vergangen sind, beträgt und

   der Gehalt an Polyvinylpyrrolidon in der obersten Schicht der inneren Oberfläche der permselektiven Hohlfasermembran 5 bis 50 Massen-% beträgt,
   **dadurch gekennzeichnet, dass** der Blutreiniger, der mit dem permselektiven Hohlfasermembranbündel auf Polysulfonbasis beladen ist, dessen Wassergehalt unter Verwendung von entlüftetem Wasser mit einer Sauerstoffkonzentration von 0,5 ppm oder weniger auf 5 bis 600 Massen-% eingestellt ist, wobei all seine Einlässe und Auslässe für Blut und ein Dialysat dicht verschlossen sind, in einen Verpackungsbeutel eingeschlossen

wird, der externe Luft und Wasserdampf ausschließen kann,
und der Blutreiniger dann einer radioaktiven Strahlung ausgesetzt wird, nachdem mindestens 48 Stunden seit dem dichten Verschließen aller Einlässe und Auslässe für das Blut und das Dialysat des Blutreinigers vergangen sind.

**2.** Das Verfahren gemäß Anspruch 1, wobei der Gehalt an Polyvinylpyrrolidon in der obersten Schicht der äußeren Oberfläche der permselektiven Hohlfasermembran 25 bis 50 Massen-% beträgt.

**3.** Das Verfahren gemäß Anspruch 1, wobei das entlüftete Wasser, das in und um die permselektive Hohlfasermembran auf Polysulfonbasis vorliegt, mit einem Inertgas gesättigtes Wasser ist.

**4.** Das Verfahren gemäß einem der Ansprüche 1 bis 3, wobei die Menge eines Harzes auf Polysulfonbasis in der permselektiven Hohlfasermembran 99 bis 80 Massen-% beträgt und die Menge an Polyvinylpyrrolidon in der permselektiven Hohlfasermembran 1 bis 20 Massen-% beträgt.

## Revendications

**1.** Procédé permettant d'obtenir un dispositif de purification du sang stérilisé, comprenant
un dispositif de purification du sang assemblé à l'aide d'un faisceau de membranes à fibres creuses permsélectives à base d'une polysulfone contenant une polyvinylpyrrolidone, dans lequel
la quantité de polyvinylpyrrolidone qui s'élue à partir du faisceau de membranes à fibres creuses est de 10 ppm ou moins,
les quantités de peroxyde d'hydrogène qui s'éluent à partir d'extraits provenant de tous les sites du faisceau de membranes à fibres creuses sont de 5 ppm ou moins, quand le faisceau de membranes à fibres creuses est divisé en 10 parties dans le sens de la longueur pour tester les sites des 10 parties selon le procédé réglementé dans la norme d'approbation des reins artificiels de type à dialyse, et
la perméabilité à l'eau du dispositif de purification du sang constatée à un point temporel après que 10 minutes se sont écoulées après le traitement d'amorçage du dispositif de purification du sang est de 90 % ou plus celle du même dispositif de purification du sang constatée à un point temporel après que 24 heures se sont écoulées après ledit traitement d'amorçage, et
la teneur en polyvinylpyrrolidone dans la couche la plus à l'extérieur de la surface intérieure de la membrane à fibres creuses permsélective est de 5 à 50 % en poids,
**caractérisé en ce que** ledit dispositif de purification du sang est chargé avec le faisceau de membranes à fibres creuses permsélectives à base de polysulfone dont la teneur en eau est ajustée à de 5 à 600 % en poids à l'aide d'une eau désaérée ayant une concentration d'oxygène de 0,5 ppm ou moins, tous
ses orifices d'admission et d'évacuation du sang et d'un dialysat étant hermétiquement fermés, et est hermétiquement fermé dans un sac d'emballage capable d'exclure l'air externe et la vapeur d'eau,
puis le dispositif de purification du sang est exposé à un rayon radioactif au moins 48 heures après la fermeture hermétique de tous les orifices d'admission et d'évacuation du sang et du dialysat du dispositif de purification du sang.

**2.** Procédé selon la revendication 1, dans lequel la teneur en polyvinylpyrrolidone dans la couche la plus à l'extérieur de la surface extérieure de la membrane à fibres creuses permsélective est de 25 à 50 % en poids.

**3.** Procédé selon la revendication 1, dans lequel l'eau désaérée présente dans et autour de la membrane à fibre creuses permsélective à base de polysulfone est une eau saturée par un gaz inerte.

**4.** Procédé selon l'une quelconque des revendications 1 à 3, dans lequel la quantité de résine à base de polysulfone dans la membrane à fibres creuses permsélective est de 99 à 80 % en poids, et la quantité de polyvinylpyrrolidone dans la membrane à fibres creuses permsélective est de 1 à 20 % en poids.

Fig. 6

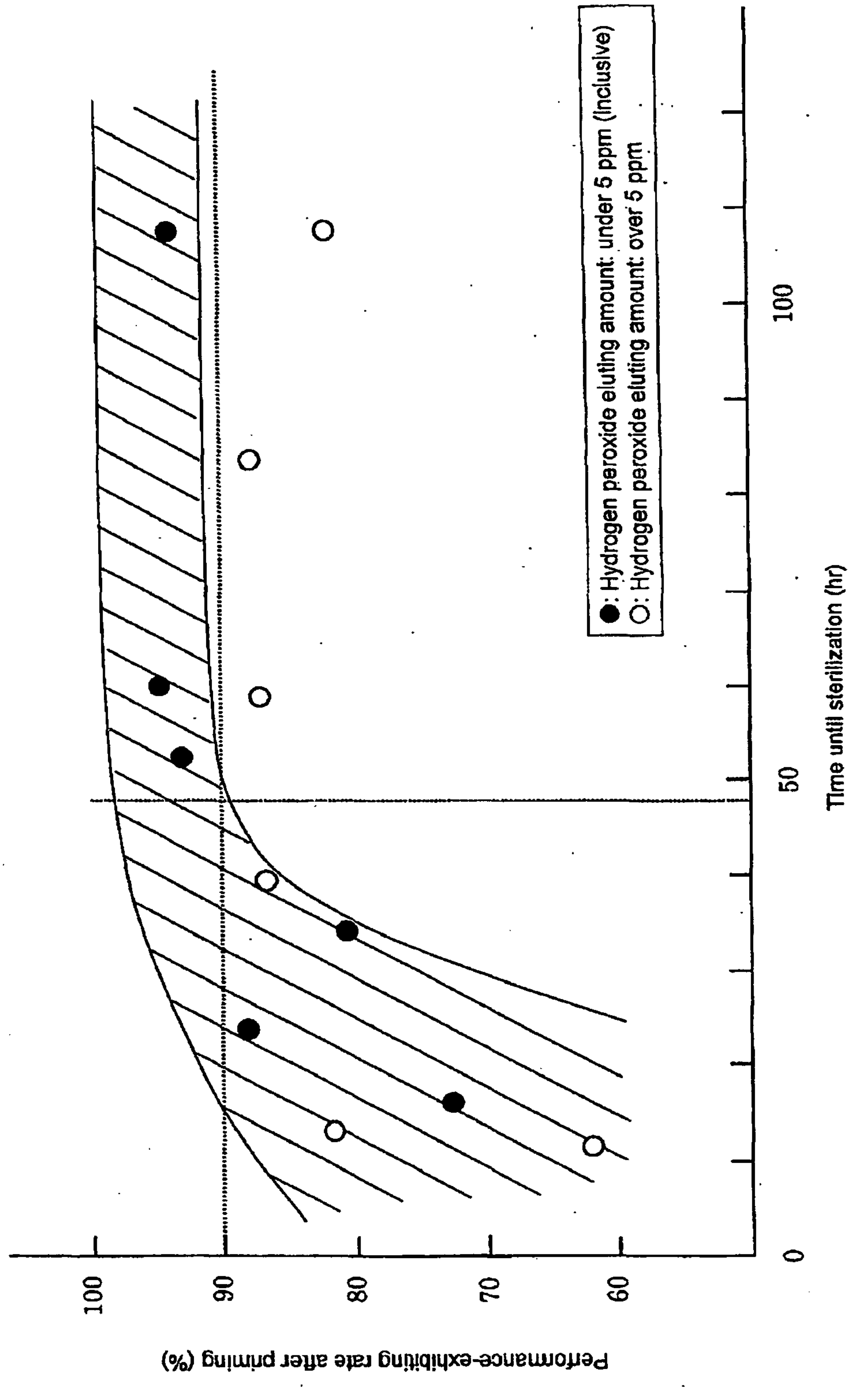
●: Hydrogen peroxide eluting amount: under 5 ppm (Inclusive)
○: Hydrogen peroxide eluting amount: over 5 ppm
Perfomance-exhibiting rate after priming (%)
100
90
80
70
60
0
50
100
Time until sterilization (hr)

Fig. 4

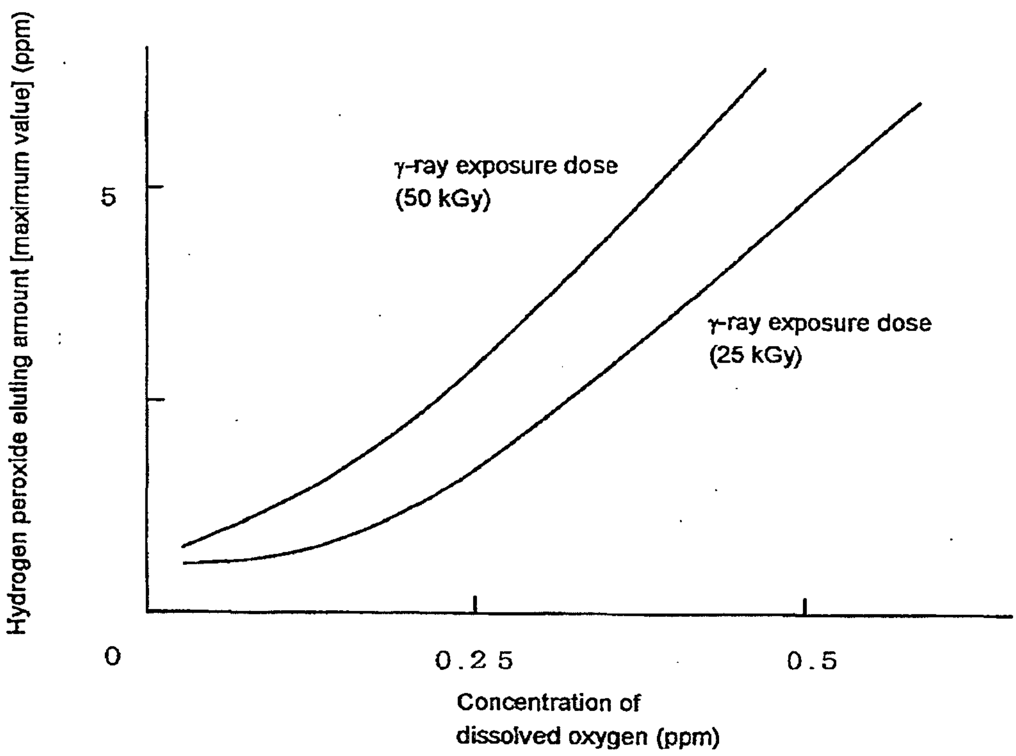
Hydrogen peroxide eluting amount [maximum value] (ppm)
5
0
0.25
0.5
Concentration of dissolved oxygen (ppm)
γ-ray exposure dose (50 kGy)
γ-ray exposure dose (25 kGy)

Fig. 5

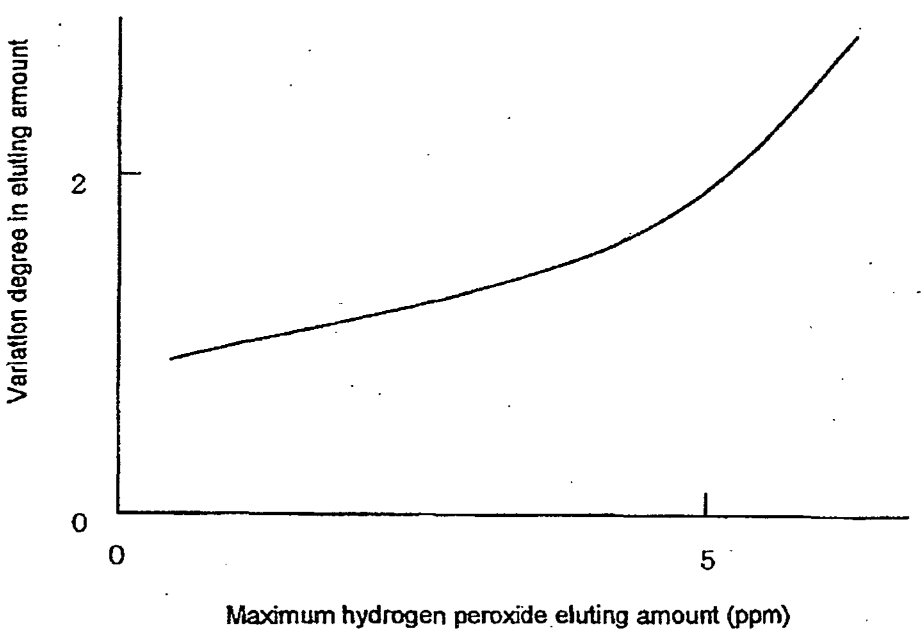
Variation degree in eluting amount
2
0
0
5
Maximum hydrogen peroxide eluting amount (ppm)

Fig. 3

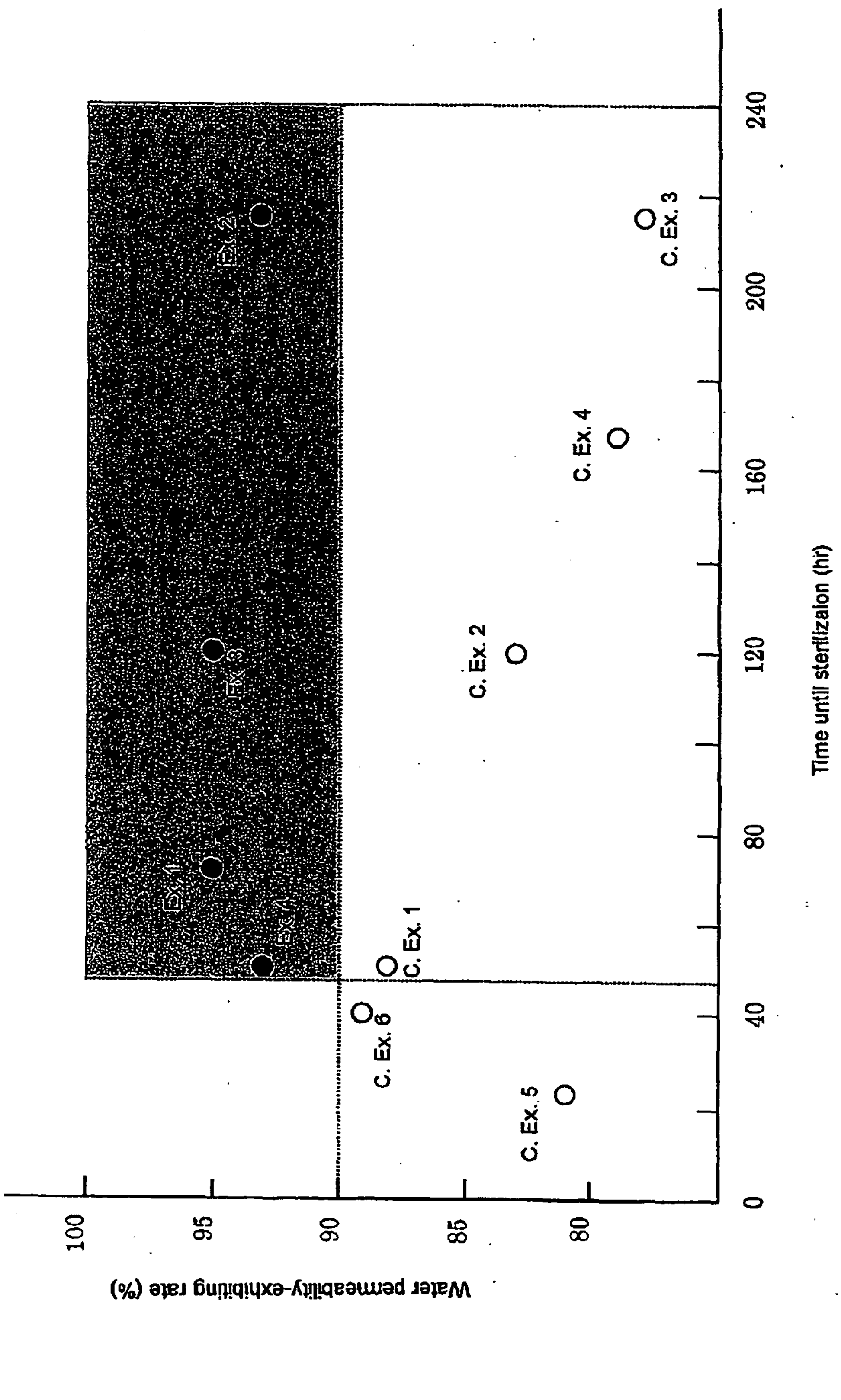
Water permeability-exhibiting rate (%)
100
95
90
85
80
Time until sterilization (hr)
0
40
80
120
160
200
240
Ex. 1
Ex. A
Ex. 3
Ex. 2
C. Ex. 1
C. Ex. 2
C. Ex. 3
C. Ex. 4
C. Ex. 5
C. Ex. 6

Fig. 2

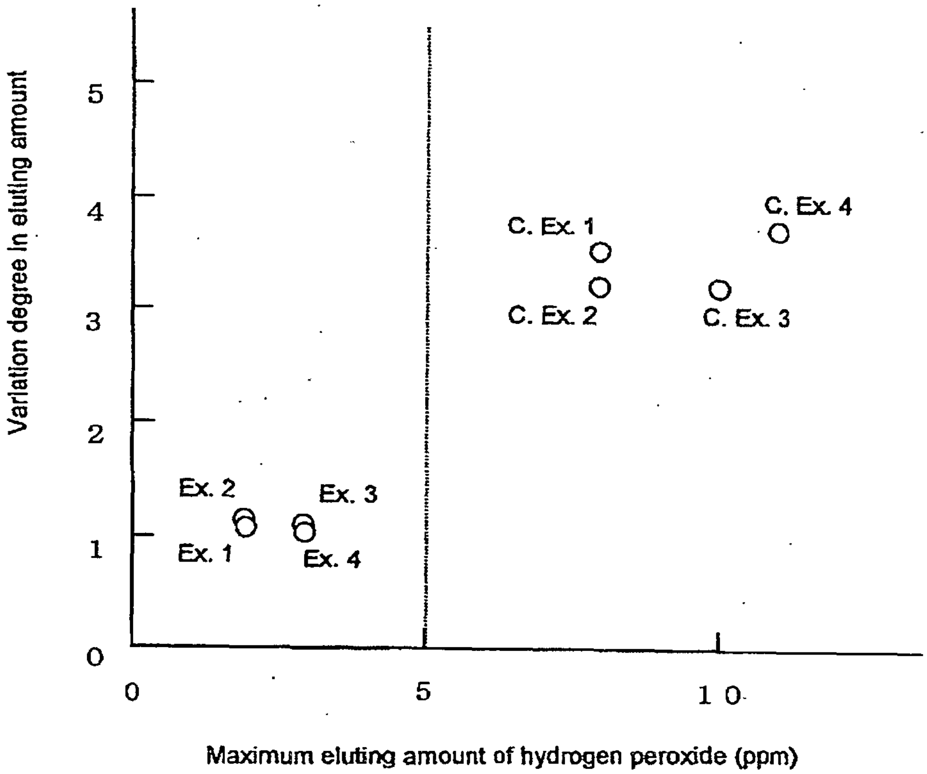
Variation degree in eluting amount
5
4
3
2
1
0
C. Ex. 1
C. Ex. 2
C. Ex. 4
C. Ex. 3
Ex. 2
Ex. 1
Ex. 3
Ex. 4
0
5
1 0
Maximum eluting amount of hydrogen peroxide (ppm)

Fig. 1

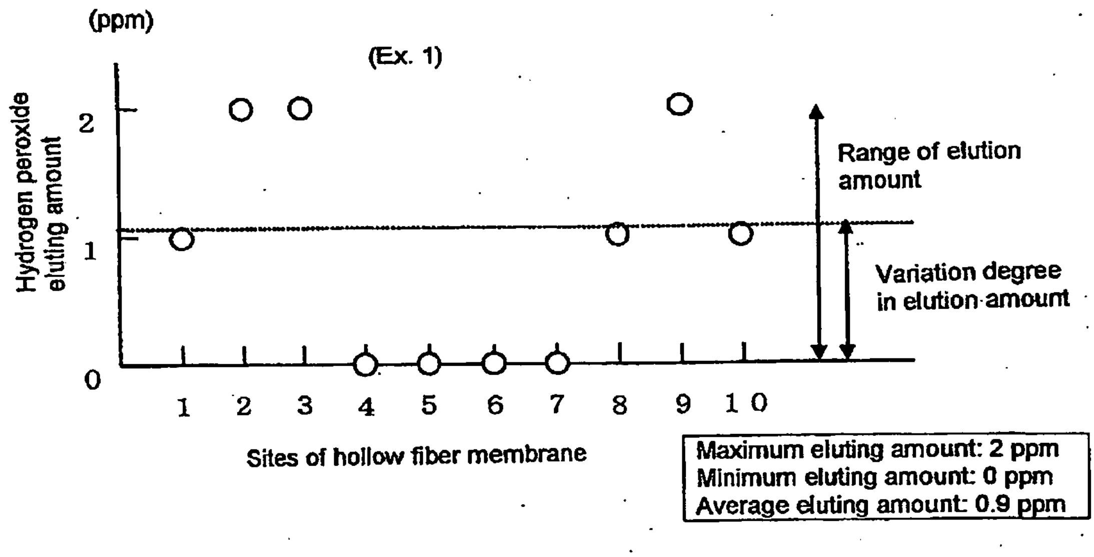
(ppm)
(Ex. 1)
Hydrogen peroxide eluting amount
2
1
0
1 2 3 4 5 6 7 8 9 10
Sites of hollow fiber membrane
Range of elution amount
Variation degree in elution amount
Maximum eluting amount: 2 ppm
Minimum eluting amount: 0 ppm
Average eluting amount: 0.9 ppm

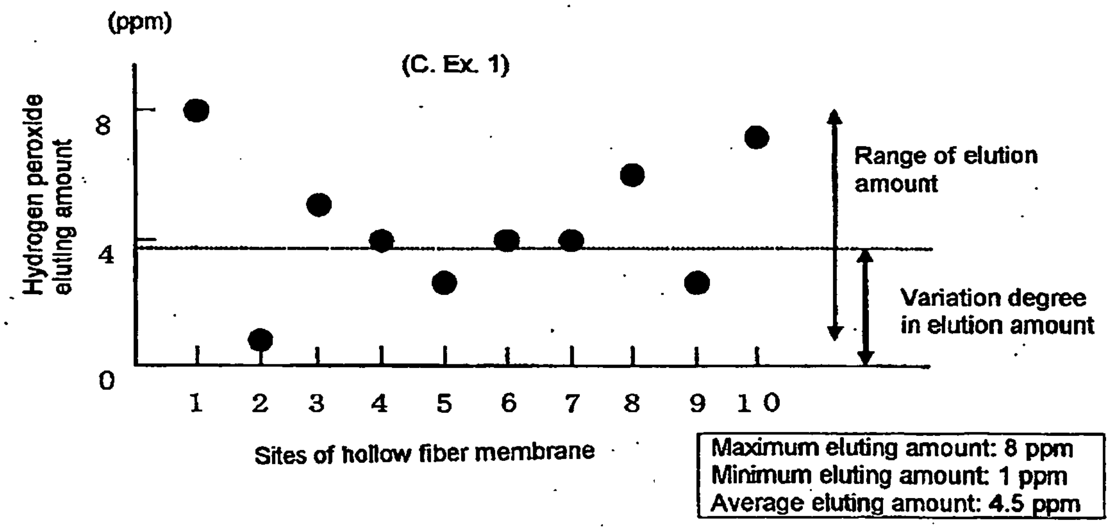
(ppm)
(C. Ex. 1)
Hydrogen peroxide eluting amount
8
4
0
1 2 3 4 5 6 7 8 9 10
Sites of hollow fiber membrane
Range of elution amount
Variation degree in elution amount
Maximum eluting amount: 8 ppm
Minimum eluting amount: 1 ppm
Average eluting amount: 4.5 ppm

Fig. 7

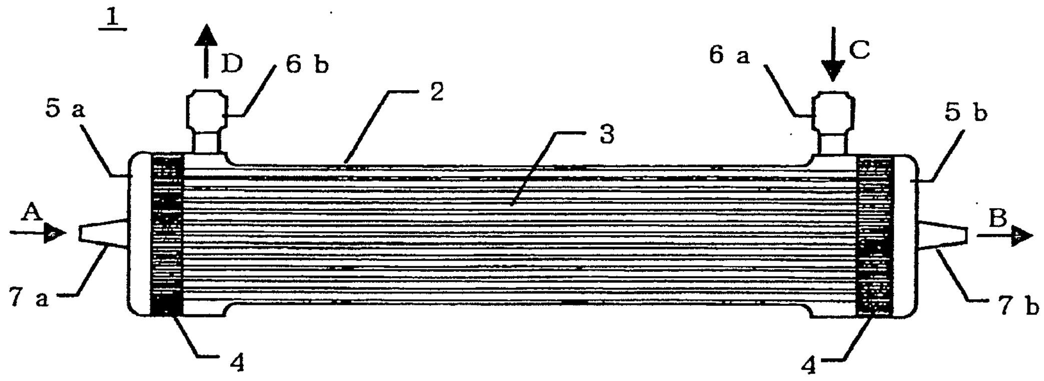


Documents for the European phase

Entry into the regional phase in Europe
based on PCT/JP2006/307033
Toyo Boseki Kabushiki Kaisha
Our Ref.: N2966 EP

VOSSIUS & PARTNER
PATENTANWÄLTE • RECHTSANWÄLTE
SIEBERTSTR. 4
81675 MÜNCHEN

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 61232860 A
- JP 58114702 A
- JP 5554373 B
- JP 6075667 B
- JP 3314861 B
- JP 6165926 A
- JP 2000350926 A
- JP 2001170171 A
- JP 2000126286 A
- JP 11309353 A
- JP 2000135421 A
- JP 55023620 B
- JP 8168524 A
- JP 2001170167 A
- JP 2000288085 A
- JP 2001205057 A
- JP 62074364 A
- JP 62204754 A
- WO 9858842 A
- JP 8280795 A
- JP 2001149471 A
- JP 2004195380 A
- JP 2003175320 A
- JP 2003175321 A
- JP 2003175322 A
- JP 2004305997 A
- JP 2004313359 A
- JP 2005058906 A
- JP 7148251 A
- EP 3636199 A
- EP O997182 A
- EP 1518564 A
- EP 1110563 A
- JP 2000300663 A
- JP 2000340356 A
- JP 2004097918 A


### Non-patent literature cited in the description


- Japan Standard Product Classification No. 877422. 2002